(19) Europäisches Patentamt
European Patent Office
Office européen des brevets

(11) EP 2 594 272 B1

(12) **EUROPEAN PATENT SPECIFICATION**

(45) Date of publication and mention of the grant of the patent:
**11.07.2018 Bulletin 2018/28**

(21) Application number: **12007354.9**

(22) Date of filing: **18.05.2006**

(51) Int Cl.:
*A61K 31/5383* (2006.01) *A61K 31/538* (2006.01)
*A61K 31/4375* (2006.01) *A61K 31/47* (2006.01)
*A61K 9/12* (2006.01) *A61P 11/00* (2006.01)
*A61K 9/08* (2006.01) *A61K 33/06* (2006.01)
*A61K 33/14* (2006.01)

# (54) Aerosolized fluoroquinolones and uses thereof

Aerosolierte Fluorchinolone und Verwendungen davon

Fluoroquinolones en aérosol et leurs utilisations

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.05.2005 US 682530 P**
**01.07.2005 US 696160 P**
**13.02.2006 US 773300 P**

(43) Date of publication of application:
**22.05.2013 Bulletin 2013/21**

(62) Document number(s) of the earlier application(s) in accordance with Art. 76 EPC:
**06760146.8 / 1 901 749**

(73) Proprietor: **Horizon Orphan LLC**
**Lake Forest, IL 60045 (US)**

(72) Inventors:
- **Bostian, Keith A.**
**Seattle, WA 98104 (US)**
- **Dudley, Michael N.**
**San Diego, CA 92127 (US)**
- **Surber, Mark**
**Coronado, CA 92118 (US)**
- **Griffith, David C.**
**San Marcos, CA 92127 (US)**
- **Lomovskaya, Olga**
**Mill Valley, CA 94941 (US)**

(74) Representative: **HGF Limited**
**1 City Walk**
**Leeds LS11 9DX (GB)**

(56) References cited:
**WO-A-02/18345**

- **WEBER A ET AL: "EFFECT OF NEBULIZER TYPE AND ANTIBIOTIC CONCENTRATION ON DEVICE PERFORMANCE", PEDIATRIC PULMONOLOGY, JOHN WILEY, NEW YORK, NY, US, vol. 23, no. 4, April 1997 (1997-04), pages 249-260, XP001118136, ISSN: 8755-6863**
- **HUNG J C. ET AL.: "Evaluation of two commercial jet nebulisers and three compressors for the nebulisation of antibiotics", ARCHIVES OF DISEASE IN CHILDHOOD, vol. 71, no. 4, October 1994 (1994-10), pages 335-338, XP009073650, ENGLAND**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 594 272 B1

## Description

## Description of the Related Art

Antibiotics have been effective tools in the treatment of infectious diseases during the last half-century. From the development of antimicrobial therapy to the late 1980s, most bacterial infections occurring in patients in developed countries could be controlled unless the infection occurred in an organ or environment where antibiotics were difficult to deliver or were ineffective, such as bacterial infections of the circulatory system in sepsis patients, or bacterial infections of the lungs in cystic fibrosis. However, even in ordinary infections, in response to the pressure of antimicrobial usage, multiple resistance mechanisms have become widespread and are threatening the clinical utility of even the most aggressive antibacterial therapy. The increase in antimicrobial resistant strains has been particularly common in major hospitals and care centers. The consequences of the increase in resistant strains include higher morbidity and mortality, longer patient hospitalization, and an increase in treatment costs. Alternative methods, compounds and compositions for inhibiting the growth of pathogen microbes *in vitro* and of treatment of pathogenic bacterial infections *in vivo* using antibacterial quinoline compounds are disclosed for example in WO 02/18345.

Bacteria have developed several different mechanisms to overcome the action of antimicrobials. These mechanisms of resistance can be specific for a molecule or a family of antimicrobials, or can be non-specific and be involved in resistance to unrelated antimicrobials. Several mechanisms of resistance can exist in a single bacterial strain, and those mechanisms may act independently or they may act synergistically to overcome the action of an antimicrobial or a combination of antimicrobials. Specific mechanisms include degradation of the drug, inactivation of the drug by enzymatic modification, and alteration of the drug target. There are, however, more general mechanisms of drug resistance, in which access of the antimicrobial to the target is prevented or reduced by decreasing the transport of the antimicrobial into the cell or by increasing the efflux of the drug from the cell to the outside medium. Both mechanisms can lower the concentration of drug at the target site and allow bacterial survival in the presence of one or more antimicrobials that would otherwise inhibit or kill the bacterial cells. Some bacteria utilize both mechanisms, combining a low permeability of the cell wall (including membranes) with an active efflux of antimicrobials.

## SUMMARY OF THE INVENTION

The present invention refers to a method of making an aerosol of a solution comprising levofloxacin or ofloxacin and a divalent or trivalent cation, said method comprising: obtaining a solution comprising levofloxacin or ofloxacin and a divalent or trivalent cation; and aerosolizing said solution to achieve an aerosol comprising a mass median aerodynamic diameter of 5$\mu$m or less, wherein said aerosol is suitable for inhalation into a lung.

Particular aspects of the method of making the aerosol according to the invention are set out in claims 2 to 11.

A further aspect of the invention provides a pharmaceutical composition, comprising an aqueous solution of levofloxacin or ofloxacin and a divalent or trivalent cation, wherein the solution is suitable for inhalation into a lung. In one embodiment the pharmaceutical composition comprises an aqueous solution of levofloxacin and a divalent or trivalent cation. In one embodiment the divalent or trivalent cation is selected from $Mg^{2+}$, $Ca^{2+}$, $Zn^{2+}$, $Fe^{2+}$ and $A1^{3+}$. In another embodiment the cation is a divalent cation selected from magnesium or calcium. In another embodiment the cation is $Mg^{2+}$.

Aerosol administration directly to the nasal, sinus, respiratory tract and pulmonary compartments through intranasal or oral inhalation enables high concentration drug delivery to a site of respiratory infection with decreased risk of extra-respiratory toxicity associated with non-respiratory routes of drug delivery. Furthermore, direct administration to the site of infection permits very high local drug levels, a property that enables "rapid administration, high concentration, local exposure" killing effect special to this class of antibiotic. Accordingly, because the microbial killing effect of a particular antibiotic compound and therapeutic composition varies depending on the formulation and delivery parameters, newer compositions and delivery methods can be developed for existing drug compounds that are re-formulated and administered through novel delivery techniques.

Members of the fluoroquinolone drug class exhibit unique pharmacologic properties, including bioavailability (F), mean absorption time (MAT) from the lung, maximal drug concentrations in the epithelial lining fluid, bronchial lavage fluid, sputum and/or lung tissue (Cmax) following aerosol administration, pulmonary retention time, area under the curve (AUC), minimal inhibitory concentrations (MIC) of the antibiotic required for antibacterial activity, AUC/MIC ratio, and local and systemic safety. Specific to the invention is the use short-term, rapid aerosol administration, delivering high concentration drug exposure directly to the affected tissue (ELF, sputum, BAL, tissue) via aerosol delivery for treatment of bacterial infection in animals and humans.

In addition to the clinical and pharmacological requirements present in any composition intended for therapeutic administration, many physicochemical factors unique to a drug compound must also be considered. These include, but are not limited to aqueous solubility, viscosity, partitioning coefficient (LogP), predicted stability in various formulations, osmolality, surface tension, pH, pKa, pKb, dissolution rate, sputum permeability, sputum binding/inactivation, taste,

throat irritability and acute tolerability.

Other factors to consider when designing the product form include fluoroquinolone physical chemistry and antibacterial activity, disease indication, clinical acceptance, and patient compliance. By non-limiting example, if desired the aerosol fluoroquinolone product may be in the form of a simple liquid (e.g. soluble fluoroquinolone with non-encapsulating soluble excipients/salts), complex liquid (e.g. fluoroquinolone encapsulated or complexed with soluble excipients such as lipids, liposomes, cyclodextrins, microencapsulations, and emulsions), complex suspension (e.g. fluoroquinolone as a low-solubility, stable nanosuspension alone, co-crystal/co-precipitate complexes, and mixtures with low solubility lipids such as solid-lipid nanoparticles), or dry powder (dry powder fluoroquinolone alone or in co-crystal/co-precipitate/spray-dried complex or mixture with low solubility excipients/salts or readily soluble blends such as lactose).

Combined with product form is a packaging consideration. By non-limiting example, considerations for packaging include intrinsic product stability, the need for stability-providing lyophilization, device selection (e.g. liquid nebulizer, dry-powder inhaler, meter-dose inhaler), and packaging form (e.g. simple liquid or complex liquid formulation in a vial as liquid or lyophilisate to be dissolved prior to or upon insertion into the device; complex suspension formulations in a vial as a liquid or lyophilisate with or without a soluble salt/excipient component to be dissolved prior to or upon insertion into the device, or separate packaging of liquid and solid components; dry powder formulations in a vial, capsule or blister pack; and other formulations packaged as readily soluble or low-solubility solid agents in separate containers alone or together with readily soluble or low-solubility solid agents. Any separately packaged agent will be manufactured to be mixed prior to or upon insertion into the delivery device).

Also disclosed are the aerosol and topical delivery of fluoroquinolone antimicrobials, such as levofloxacin. Levofloxacin has favorable solubility characteristics enabling dosing of clinically-desirable fluoroquinolone levels by aerosol (e.g. through liquid nebulization, dry powder dispersion or meter-dose administration) or topically (e.g. aqueous suspension, oily preparation or the like or as a drip, spray, suppository, salve, or an ointment or the like) and can be used in methods for acute or prophylactic treatment of an infected vertebrate, e.g. a bacterial infection, or a subject at risk of an infection. The fluoroquinolone may also be ofloxacin.

The therapeutic method disclosed but not claimed may treat a bacterial infection in a subject using concentrated aerosol levofloxacin administered to a subject infected with a pathogenic bacteria in the lungs.

The therapeutic method may also include a diagnostic step, such as identifying a patient infected with a particular pathogenic bacteria, or a resistant bacteria. In some embodiments, the method further includes identifying a patient as colonized with a bacteria that is capable of developing resistance to fluoroquinolone antimicrobials. In some embodiments, the delivered amount of aerosol levofloxacin is sufficient to overcome resistance or prevent resistance development to levofloxacin. In one embodiment, the MIC of the fluoroquinolone antibacterial compound for the microbe is greater than about 2 ug/ml.

Suitably the delivered amount of aerosol levofloxacin is sufficient to overcome resistance or prevent further resistance of an organism exhibiting an MIC of the fluoroquinolone antibacterial compound that is greater than about 4 ug/ml.

Suitably the delivered amount of aerosol fluoroquinolone is sufficient to overcome resistance or prevent further resistance of an organism exhibiting an MIC of the fluoroquinolone antibacterial compound that is greater than about 8 ug/ml.

Suitably the delivered amount of aerosol fluoroquinolone is sufficient to overcome resistance or prevent further resistance of an organism exhibiting an MIC of the fluoroquinolone antibacterial compound that is greater than about 16 ug/ml.

Suitably the delivered amount of aerosol fluoroquinolone is sufficient to overcome resistance or prevent further resistance of an organism exhibiting an MIC of the fluoroquinolone antibacterial compound that is greater than about 32 ug/ml.

Also disclosed but not claimed is a method for prophylactic treatment of a subject, including administering to a subject, susceptible to microbial infection or a chronic carrier of an asymptomatic or low symptomatic microbial infection, a fluoroquinolone antimicrobial to achieve a minimal inhibitory concentration of antimicrobial at a site of potential or current infection. In one embodiment, the method further comprising identifying a subject as a subject at risk of a bacterial infection or at risk for an exacerbation of an infection.

Also disclosed but not claimed is a method for acute or prophylactic treatment of a patient through aerosol administration of fluoroquinolone to produce and maintain threshold drug concentrations in the lung, which may be measured as drug levels in epithelial lining fluid (ELF), sputum, lung tissue or bronchial lavage fluid (BAL). One embodiment includes the use of short-term, rapid aerosol administration, delivering high concentration drug exposure directly to the affected tissue for treatment of bacterial infections in animals and humans.

Also disclosed but not claimed is a method for treating a microbial infection in a subject, including administering to a subject infected with a microbe a fluoroquinolone antimicrobial to achieve a minimal inhibitory concentration of antimicrobial at a site of infection. In one embodiment, the method further comprising identifying the subject as infected with a microbe that is resistant to an antimicrobial agent.

In some embodiments of the methods described above, fluoroquinolone antimicrobial minimal inhibitory concentration remains at the site of infection for at least about a 5 mintue period, at least about a 10 min period, at least about a 20 min period, at least about a 30 min period, at least about a 1 hour period, 2 hour period, at least about a 4 hour period or other time values on the quarter hour interval. The effective fluoroquinolone antimicrobial minimal inhibitory concentration (MIC) is sufficient to cause a therapeutic effect and the effect may be localized to the site of infection. In some embodiments, one or more levofloxacin administrations achieve an ELF, BAL, and/or sputum fluoroquinolone concentration of at least 1-fold to 5000-fold the infecting or potentially infecting organisms MIC, including all integral values therein such as 2-fold, 4-fold, 8-fold, 16-fold, 32-fold, 64-fold, 128-fold, 256-fold, 512-fold, 1028-fold, 2056-fold, and 4112-fold the microbials MIC.

In some embodiments, such as a pulmonary site, the fluoroquinolone antimicrobial is administered in one or more administrations so as to achieve a respirable delivered dose daily of at least about 5 mg to about 50 mg, including all integral values therein such as 10, 15, 20, 25, 30, 35, 40 and 45 milligrams. Similarly, the fluoroquinolone antimicrobial is administered in one or more administrations so as to achieve a respirable delivered dose daily of at least about 50 to about 100 mg including all integral values therein, such as 55, 60, 65, 70, 75, 80, 85, 90, and 95 mg. In some embodiments of the methods described above, the fluoroquinolone antimicrobial is administered in one or more administrations so as to achieve a respirable delivered daily dose of up to 150 mg including all integral values therein such as 105, 110, 115, 120, 125, 130, 135, 140 and 145 mg. The fluoroquinolone antimicrobial is administered in the described respirable delivered dose in less than 20 minutes, less than 10 minutes, less than 7 minutes, less than 5 minutes, in less than 3 minutes and in less than 2 minutes. In some embodiments of the methods described above, the antimicrobial agent is ofloxacin, although levofloxacin is preferred.

In some embodiments of the methods described above, the bacteria is a gram-negative bacteria such as Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, and Bacteroides splanchnicus. In some embodiments of the methods described above, the bacteria is a gram-negative anaerobic bacteria, by non-limiting example these include Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, and Bacteroides splanchnicus. In some embodiments of the methods described above, the bacteria is a gram-positive bacteria, by non-limiting example these include: Corynebacterium diphtheriae, Corynebacterium ulcerans, Streptococcus pneumoniae, Streptococcus agalactiae, Streptococcus pyogenes, Streptococcus milleri ; Streptococcus (Group G); Streptococcus (Group C/F); Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis, and Staphylococcus saccharolyticus. In some embodiments of the methods described above, the bacteria is a gram-positive anaerobic bacteria, by non-limiting example these include Clostridium difficile, Clostridium perfringens, Clostridium tetini, and Clostridium botulinum. In some embodiments of the methods described above, the bacteria is a acid-fast bacteria, by non-limiting example these include Mycobacterium tuberculosis, Mycobacterium avium, Mycobacterium intracellulare, and Mycobacterium leprae. In some embodiments of the methods described above, the bacteria is a atypical bacteria, by non-limiting example these include Chlamydia pneumoniae and Mycoplasma pneumoniae.

In some embodiments of the methods described above, the subject is a human. In some embodiments of the methods described above, the subject is a human with cystic fibrosis. In some embodiments of the methods described above, the subject is a human with pneumonia, a chronic obstructive pulmonary disease, or sinusitis, or a human being mechanically ventilatated.

Also disclosed is a pharmaceutical composition that includes a simple liquid fluoroquinolone antimicrobial formulation (e.g. soluble fluoroquinolone with non-encapsulating water soluble excipients) as described above having an osmolality from about 200 mOsmol/kg to about 1250 mOsmol/kg. In one such embodiment, the solution has a permeant ion concentration from about 30 mM to about 300 mM. In one embodiment, the osmolality is from about 250 mOsmol/kg to about 1050 mOsmol/kg. In one embodiment, the osmolality is from prefereably from about 350 mOsmol/kg

and about 750 mOsmol/kg and most preferably approximately 300 mOsmol/kg.

Also disclosed is a pharmaceutical composition is provided that includes a simple liquid fluoroquinolone antimicrobial formulation having a permeant ion concentration between from about 30 mM to about 300 mM and preferably between from about 50mM to 200 mM. In one such embodiment, one or more permeant ions in the composition are selected from the group consisting of chloride and bromide.

Also disclosed is a pharmaceutical composition that includes a complex liquid fluoroquinolone antimicrobial formulation (e.g. fluoroquinolone encapsulated or complexed with water soluble excipients such as lipids, liposomes, cyclodextrins, microencapsulations, and emulsions) as described above having a solution osmolality from about 200 mOsmol/kg to about 1250 mOsmol/kg. In one such embodiment, the solution has a permeant ion concentration from about 30 mM to about 300 mM. In one embodiment, the osmolality is from about 250 mOsmol/kg to about 1050 mOsmol/kg. In one embodiment, the osmolality is from prefereably from about 350 mOsmol/kg and about 750 mOsmol/kg and most preferably approximately 300 mOsmol/kg.

Also disclosed is a pharmaceutical composition that includes a complex liquid fluoroquinolone antimicrobial formulation having a permeant ion concentration from about 30 mM to about 300 mM. In one such embodiment, one or more permeant ions in the composition are selected from the group consisting of chloride and bromide.

Also disclosed is a pharmaceutical composition that includes a complex liquid fluoroquinolone antimicrobial formulation having a permeant ion concentration from about 50 mM to about 200 mM. In one such embodiment, one or more permeant ions in the composition are selected from the group consisting of chloride and bromide.

Also disclosed is a pharmaceutical composition that includes a taste-masking agent. By non-limiting example a taste-masking agent may include a sugar, a divalent or trivalent cation that complexes with a fluoroquinolone, optimized osmolality, and/or an optimized permeant ion concentration.

Also disclosed but not claimed is a system for administering a fluoroquinolone antimicrobial that includes a container comprising a solution of a fluoroquinolone antimicrobial and a nebulizer physically coupled or co-packaged with the container and adapted to produce an aerosol of the solution having a particle size from about 2 microns to about 5 microns mean mass aerodynamic diameter and a particle size geometric standard deviation of less than or equal to about 2.5 microns mean mass aerodynamic diameter. In one embodiment, the particle size geometric standard deviation is less than or equal to about 2.0 microns. In one embodiment, the particle size geometric standard deviation is less than or equal to about 1.8 microns.

Also disclosed but not claimed is a kit that includes a container comprising a pharmaceutical formulation comprising a quinolone antimicrobial agent and an aerosolizer adapted to aerosolize the pharmaceutical formulation and deliver it to the lower respiratory tract and pulmonary compartment following intraoral administration. The formulation may also be delivered as a dry powder or through a metered-dose inhaler.

Also disclosed but not claimed is a kit that includes a container comprising a pharmaceutical formulation comprising a quinolone antimicrobial agent and an aerosolizer adapted to aerosolize the pharmaceutical formulation and deliver it to nasal cavity following intranasal administration.

It is to be understood that both the foregoing general description and the following detailed description are exemplary and explanatory only and are not restrictive of the invention, as claimed.

## DESCRIPTION OF FIGURES

Figure 1 is a graph showing the dose:MIC relationship of fluoroquinolones and other antibiotics to bacterial killing.

Figure 2 is a graph showing ciprofloxacin serum concentrations following oral dosing in both CF patients vs healthy controls.

Figure 3 is a graph showing ciprofloxacin sputum and serum concentrations following oral dosing.

Figure 4A is a graph showing Levofloxacin time-kill affects on logarithmic PAM1020 cells.

Figure 4B is a graph showing Levofloxacin time-kill affects on logarithmic PAM1032 cells.

Figure 5A is a graph showing Levofloxacin time-kill affects on stationary phase PAM1020 cells.

Figure 5B is a graph showing Levofloxacin time-kill affects on stationary phase PAM1032 cells.

Figure 6A is a graph showing PAM 1020 re-growth following a 10 minute Levofloxacin exposure.

Figure 6B is a graph showing PAM 1020 re-growth following a 160 minute Levofloxacin exposure.

Figure 6C is a graph showing PAM 1032 re-growth following a 10 minute Levofloxacin exposure.

Figure 6D is a graph showing PAM 1032 re-growth following a 160 minute Levofloxacin exposure.

Figure 7A is a graph showing Levofloxacin time-kill affects on late-logarithmic PAM 1020 cells under oxygen limiting conditions.

Figure 7B is a graph showing Levofloxacin time-kill affects on late-logarithmic PAM 1032 cells under oxygen limiting conditions.

Figure 8A is a graph showing Levofloxacin killing kinetics of PAM1032 in Meuller-Hinton broth (MHB).

Figure 8B is a graph showing Levofloxacin killing kinetics of PAM1032 in cystic fibrosis sputum.

Figure 9 is a graph showing levofloxacin killing affects on *Pseudomonas* biofilms.

Figure 10 is a graph showing the bactericidal effects of levofloxacin with a Cmax of 1000 μg/ml and a 10 minute half-life in a hollow fiber model.

Figure 11 is a graph showing the bactericidal effects of levofloxacin with a Cmax of 600 μg/ml and a 10 minute half-life in a hollow fiber model.

Figure 12 is a graph showing the pH solubility profile of Levofloxacin by acid titration.

Figure 13 is a graph measuring pH while titrating Levofloxacin with HCl.

Figure 14 is a graph showing the Vt[OH] vs. Vt of Levofloxacin.

Figure 15 is a graph measuring pH while titrating Levofloxacin with NaOH.

Figure 16 is a graph measuring dpH/dV vs volume of NaOH titrant (V**t**) for titration of Levofloxacin.

Figure 17 is a graph measuring the absorbance of a Levofloxacin solution at 257 nm vs pH.

Figure 18 is a graph showing the complexation of Levofloxacin with divalent and trivalent cations.

Figure 19 is a graph showing the dual titration complexation of Levofloxacin with Mg2+.

Figure 20 is a graph showing the dual titration complexation of Levofloxacin with Fe2+.

Figure 21 is a graph showing the dual titration complexation of Levofloxacin with Ca2+.

Figure 22 is a graph showing the dual titration complexation of Levofloxacin with Zn2+.

Figure 23 is a graph showing Levofloxacin complexed with Ca2+ vs. free Levofloxacin.

Figure 24 is a graph showing Levofloxacin complexed with Mg2+ vs. free Levofloxacin.

Figure 25 is a graph showing Levofloxacin complexed with Fe2+ vs. free Levofloxacin.

Figure 26 is a graph showing Levofloxacin complexed with Zn2+ vs. free Levofloxacin.

Figure 27 is a graph showing the solubility of Levofloxacin in the presence of Mg2+.

Figure 28 is a graph showing the solubility of Levofloxacin in the presence of Mg2+ at constant ionic strength.

Figure 29 is a graph showing complexation of Levofloxacin with Fe2+ as measured by spectrofluorometry.

Figure 30 is a graph showing complexation of Levofloxacin with Zn2+ as measured by spectrofluorometry.

## DETAILED DESCRIPTION

Many of the problems associated with antimicrobial-resistant pathogens could be alleviated if the concentration of the antimicrobial could be safely increased at the site of infection. For example, pulmonary infections may be treated by administration of the antimicrobial agent directly, at high concentrations directly to the site of infection without incurring large systemic concentrations of the antimcirobial. Accordingly, some embodiments disclosed herein are improved methods for delivering drug compositions to treat pulmonary bacterial infections. More specifically, as described herein, it has been discovered that aerosol levofloxacin and other fluoroquinolones can be safely delivered by inhalation at levels sufficient to kill susceptible bacterial infections, to decrease the frequencey of antimicrobial resistance and to increase efficacy against resistant pulmonary infections.

### Definitions

The term "administration" or "administering" refers to a method of giving a dosage of an antimicrobial pharmaceutical composition to a vertebrate. The preferred method of administration can vary depending on various factors, e.g., the components of the pharmaceutical composition, the site of the potential or actual bacterial infection, the microbe involved, and the severity of an actual microbial infection.

A "carrier" or "excipient" is a compound or material used to facilitate administration of the compound, for example, to increase the solubility of the compound. Solid carriers include, e.g., starch, lactose, dicalcium phosphate, sucrose, and kaolin. Liquid carriers include, e.g., sterile water, saline, buffers, non-ionic surfactants, and edible oils such as oil, peanut and sesame oils. In addition, various adjuvants such as are commonly used in the art may be included. These and other such compounds are described in the literature, e.g., in the Merck Index, Merck & Company, Rahway, NJ. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press.

A "diagnostic" as used herein is a compound, method, system, or device that assists in the identification and characterization of a health or disease state. The diagnostic can be used in standard assays as is known in the art.

The term "mammal" is used in its usual biological sense. Thus, it specifically includes humans, cattle, horses, dogs, and cats, but also includes many other species.

The term "microbial infection" refers to the undesired proliferation or presence of invasion of pathogenic microbes in a host organism. This includes the excessive growth of microbes that are normally present in or on the body of a mammal or other organism. More generally, a microbial infection can be any situation in which the presence of a microbial population(s) is damaging to a host mammal. Thus, a microbial infection exists when excessive numbers of a microbial population are present in or on a mammal's body, or when the effects of the presence of a microbial population(s) is damaging the cells or other tissue of a mammal.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

The term "pharmaceutically acceptable salt" refers to salts that retain the biological effectiveness and properties of the compounds of this invention and, which are not biologically or otherwise undesirable. In many cases, the compounds of this invention are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, naphtoic acid, oleic acid, palmitic acid, pamoic (emboic) acid, stearic acid, glycolic acid, pyruvic acid, oxalic acid, maleic acid, malonic acid, succinic acid, fumaric acid, tartaric acid, citric acid, ascorbic acid, glucoheptonic acid, glucuronic acid, lactic acid, lactobioic acid, tartaric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, histidine, arginine, lysine, benethamine, N-methyl-glucamine, and ethanolamine. Other acids include dodecylsufuric acid, naphthalene-1,5-disulfonic acid, naphthalene-2-sulfonic acid, and saccharin.

"Solvate" refers to the compound formed by the interaction of a solvent and fluoroquinolone antimicrobial, a metabolite, or salt thereof. Suitable solvates are pharmaceutically acceptable solvates including hydrates.

In the context of the response of a microbe, such as a bacterium, to an antimicrobial agent, the term "susceptibility" refers to the sensitivity of the microbe for the presence of the antimicrobial agent. So, to increase the susceptibility means that the microbe will be inhibited by a lower concentration of the antimicrobial agent in the medium surrounding the microbial cells. This is equivalent to saying that the microbe is more sensitive to the antimicrobial agent. In most cases the minimum inhibitory concentration (MIC) of that antimicrobial agent will have been reduced.

By "therapeutically effective amount" or "pharmaceutically effective amount" is meant a fluoroquinolone antimicrobial agent, as disclosed for this invention, which has a therapeutic effect. The doses of fluoroquinolone antimicrobial agent which are useful in treatment are therapeutically effective amounts. Thus, as used herein, a therapeutically effective amount means those amounts of fluoroquinolone antimicrobial agent which produce the desired therapeutic effect as judged by clinical trial results and/or model animal infection studies. In particular embodiments, the fluoroquinolone antimicrobial agent are administered in a predetermined dose, and thus a therapeutically effective amount would be an amount of the dose administered. This amount and the amount of the fluoroquinolone antimicrobial agent can be routinely determined by one of skill in the art, and will vary, depending on several factors, such as the particular microbial strain involved. This amount can further depend upon the patient's height, weight, sex, age and medical history. For prophylactic treatments, a therapeutically effective amount is that amount which would be effective to prevent a microbial infection.

A "therapeutic effect" relieves, to some extent, one or more of the symptoms of the infection, and includes curing an infection. "Curing" means that the symptoms of active infection are eliminated, including the total or substantial elimination of excessive members of viable microbe of those involved in the infection to a point at or below the threshold of detection by traditional measurments. However, certain long-term or permanent effects of the infection may exist even after a cure is obtained (such as extensive tissue damage). As used herein, a "therapeutic effect" is defined as a statistically significant reduction in bacterial load in a host, emergence of resistance, or improvement in infection symptoms as measured by human clinical results or animal studies.

"Treat," "treatment," or "treating," as used herein refers to administering a pharmaceutical composition for prophylactic and/or therapeutic purposes. The term "prophylactic treatment" refers to treating a patient who is not yet infected, but who is susceptible to, or otherwise at risk of, a particular infection. The term "therapeutic treatment" refers to administering treatment to a patient already suffering from an infection. Thus, in preferred embodiments, treating is the administration to a mammal (either for therapeutic or prophylactic purposes) of therapeutically effective amounts of a fluoroquinolone antimicrobial agent.

Pharmacokinetics (PK) is concerned with the time course of antimicrobial concentration in the body. Pharmacodynamics (PD) is concerned with the relationship between pharmacokinetics and the antimicrobial efficacy *in vivo.* PK/PD parameters correlate antimicrobial exposure with antimicrobial activity. The rate of killing by antimicrobial is dependent on antimicrobial mode of action and is determined by either the length of time necessary to kill (time-dependent) or the effect of increasing concentrations (concentration-dependent). Accordingly, to predict the therapeutic efficacy of antimicrobials with diverse mechanisms of action different PK/PD parameters may be used.

"AUC/MIC ratio" is one example of a PK/PD parameter. AUC is defined as the area under the plasma or site-of-infection concentration-time curve of an antimicrobial agent *in vivo* (in animal or human). AUC/MIC ratio is determined by dividing the 24-hour-AUC for an individual antimicrobial by the MIC for the same antimicrobial determined *in vitro.* Activity of antimicrobials with the dose-dependent killing (such as fluoroquinolones) is well predicted by the magnitude of the AUC/MIC ratio.

"Cmax:MIC" ratio is another PK:PD parameter. It describes the maximum drug concentration in plasma or tissue relative to the MIC. Fluoroquinolones and aminoglycosides are examples where Cmax:MIC may predict in vivo bacterial killing where resitance can be suppressed.

"Time above MIC" (T>MIC) is another PK/PD parameter. It is expressed a percentage of a dosage interval in which the plasma or site-of-infection level exceeds the MIC. Activity of antimicrobials with the time-dependent killing (such as beta-lactams or oxazolidinones) is well predicted by the magnitude of the T>MIC ratio.

The term "dosing interval" refers to the time between administrations of the two sequential doses of a pharmaceutical's during multiple dosing regimens. For example, in the case of ciprofloxacin, which is administered twice daily (traditional regimen of 400 mg b.i.d) and levofloxacin, which is administered once a day (500mg or 750mg q.d.), the dosing intervals are 12 hours and 24 hours, respectively.

As used herein, the "peak period" of a pharmaceutical's *in vivo* concentration is defined as that time of the pharmaceutical dosing interval when the pharmaceutical concentration is not less than 50% of its maximum plasma or site-of-infection concentration. In some embodiments, "peak period" is used to describe an interval of antimicrobial dosing.

The "respirable delivered dose" is the amount of drug inhaled during the inspiratory phase of the breath simulator

that is equal to or less than 5 microns using a simulator programmed to the European Standard pattern of 15 breaths per minute, with an inspiration to expiration ratio of 1:1.

## Advantages of Inhaled Aerosol and Topical (Non-Oral) Fluoroquinolone Delivery

The antibiotic rate of killing is dependent upon antibiotic mode of action and is determined by either the length of time necessary for the antibiotic to kill (time-dependent) or the effect of increasing the antibiotic concentration (concentration-dependent). Fluoroquinolones are characterized by concentration-dependent, time-kill activity where a therapeutic effect requires a high local peak concentration above the MICs of the infecting pathogen.

Fluoroquinolone efficacy in humans, animals and *in vitro* models of infection is linked to AUC:MIC ratio and Cmax:MIC ratio. Given the prior uncertainty of the pharmacokinetics of fluoroquinolones in pulmonary tissue, a number of *in vitro* studies have been conducted to determine if high doses of levofloxacin with extremely short half-lives (as predicted from a rat and human PK model) result in bacterial killing superior to that seen under conditions with more prolonged residence times. In these studies, levofloxacin concentrations that were 0.018-fold - 1024-fold the MIC were evaluated in a standard kill-curve and *in vitro* hollow fiber assay. In both of these assays, high concentrations of levofloxacin were rapidly bactericidal and reached their maximum level of killing in 10-20 minutes. This level of killing was sustained whether levofloxacin was maintained at that level or given a half-life of 10 minutes. Accordingly, high doses and rapid delivery of specially formulated levofloxacin, such as a rapidly delivered 20-50 mg respirable deposited aerosol levofloxacin dose (which will produce initial ELF concentrations of 800 - 1600 ug/mL) is rapidly bactericidal for susceptible organisms and resistant organisms with MICs up to 32 ug/ml. It is expected that these unique antimicrobial properties of fluoroquinolones will also translate to topical administrations, including, but not limited to infections or prophylaxis of the skin, eye, ear, rectum, vagina, or urinary tract.

To measure the efficacy of different delivery models, AUC shape-enhancing levofloxacin formulations were prepared and measured *in vivo* in comparison to non-AUC shape-enhancing levofloxacin formulations and other antibiotics using both rat PK and mouse efficacy following intratracheal administration. As was previously shown in a rat system, there were differences among drugs in pulmonary pharmacokinetics, with some agents showing lower AUCs (e.g., levofloxacin), while others such as gemifloxacin or tobramycin show higher concentrations resulting from slower pulmonary clearance. Studies in a single dose mouse model of infection with aerosol doses have shown variable efficacy among the compounds. Referring to Figure 1, analysis of the data by dividing the aerosolized dose by the MIC indicated a strong correlation between Dose:MIC ratio and bactericidal activity (R2 = 0.89). These data suggest that the initial bactericidal activity in this model is not affected by the pulmonary clearance of the drug. Although pulmonary clearances have not been estimated in mice, transformation of dose to AUC using scaled rat values would be expected to degrade the relationship. Therefore, this data suggests that optimizing the AUC shape for levofloxacin may not be necessary for aerosol levofloxacin to be effective in treating respiratory tract and pulmonary infections.

Recent investigations with fluoroquinolones resulted in development of the concept of a "mutant selection window" (MSW) for bacterial resistance arising during therapy. This concept assists in identifying a concentration range where mutants are selected more frequently *in vitro* and *in vivo.* The lower boundary of the window is the lowest concentration that kills the majority of infecting cells (approximated by the MIC), while the upper boundary of the window is the drug concentration that blocks the growth of the least susceptible first-step mutant. Above the upper boundary concentration the growth of the infecting bacteria requires the presence of at least two resistance mutations. This upper boundary is designated the mutant prevention concentration (MPC). The values of MPC vary depending on bacteria and fluoroquinolone, and may be 10- to 20-fold higher than the MIC. Several modeling studies have demonstrated that the longer the drug concentration exceeds the MPC at the site of infection, the more effectively the treatment will prevent resistance development. Conversely, the longer the antibiotic concentration stays within the MSW, the higher the probability to select resistant mutants. Importantly, the currently approved dosing regimen for oral or intravenous levofloxacin has placed this antibiotic within the MSW for more than 20% of the dosing interval for such pathogens as *P. aeruginosa (Pa)* and *S. pneumonia.* Accordingly, a high level of levofloxacin resistance is reported for both of these pathogens.

Therefore, in one embodiment disclosed but not claimed, the concentration of levofloxacin at the site of infection is increased by delivering it directly to the lung using inhalation therapy, thereby decreasing the amount of time levofloxacin is in the MSW. Such a therapeutic approach achieves broader coverage of pathogens (including levofloxacin resistant strains), prevents further resistance development, and results in shorter courses of levofloxacin therapy.

## Pharmacokinetics of Orally Administered Fluoroquinolones in Non-CF and CF Populations

### Sputum Concentrations in CF Patients

The pharmacokinetics of ciprofloxacin has been extensively studied in CF patients after oral administration. In fact, it has been demonstrated that the serum PK profile of ciprofloxacin is very similar in CF patients to healthy volunteers

(Figure 2).

Moreover, the sputum vs time profile of ciprofloxacin is very similar to its serum profile after oral administration (Figure 3). After a 750 mg oral dose, peak concentrations of ~4.2 μg/ml and ~3.5 μg/ml were achieved for serum and sputum, respectively. Serum and sputum drug concentrations peaked at 1.5 and 4 hours, respectively. While the the total amount of ciprofloxacin into sputum is high relative to serum concentrations, the absolute concentrations are low relative to MICs of target organisms such as Pa. This data is consistant with poor clinical outcome due to resistance development to these low drug concentrations.

While data for levofloxacin intrapulmonary pharmacokinetics in cystic fibrosis are not available, data on the closely-related ofloxacin were published in the 1980's and 1990s. Ofloxacin is comprised of a racemic mixture of the dextro- (microbiologically inactive) and levo-rotatory (levofloxacin-microbiologically active). Studies have shown that the pharmacokinetic properties of the 2 components are similar. In comparative studies with ciprofloxacin, ofloxacin had a longer half-life and higher distribution into sputum (79% vs 21%) than ciprofloxacin.

**Lung Epithelial Lining Fluid**

More recent emphasis on the use and development of fluoroquinolones in community acquired gram-positive infections has focused on intrapulmonary pharmacokinetic studies in lung epithelial lining fluid (ELF). Although the relevance of drug distribution into this fluid is not clear in the setting of cystic fibrosis, insights in the drug pharmacology can be obtained from these studies. Levofloxacin penetrates well into lung tissues. Lung tissue concentrations are generally 2- to 5-fold higher than plasma concentrations. Several recent studies (summarized in Table 1) demonstrated that ELF concentrations of levofloxacin in healthy subjects following an oral dose of 750 mg reach a maximum concentration around 20 μg/mL. Similar peak concentrations are expected in the sputum of CF patients after oral or IV administration of 750 mg of levofloxacin. In contrast, ciprofloxacin penetrates lung tissues much less efficiently than levofloxacin. Based upon studies of the mutant selection window (MSW), these ELF fluoroquinolone drug levels are insufficient to achieve the required mutant prevention concentration of 10- to 20-fold the MIC for the infecting organism.

**Table 1. Concentration of Levofloxacin in Epithelial Lining Fluid in Man.**

| | | | ELF Drug Concentration (μg/ml) | | | | | | |
|---|---|---|---|---|---|---|---|---|---|
| **Drug** | **Dose** | **Route** | **0.5 hr** | **1 hr** | **2 hr** | **4 hr** | **6 hr** | **12 hr** | **24 hr** |
| levofloxacin | 500 mg | IV | | | | 11 | | 2.5 | 1.24 |
| levofloxacin | 500 mg | oral | | | | 9.9 | | 6.5 | 0.7 |
| levofloxacin | 500 mg | oral | | | | 9.94 | | 6.46 | 0.7 |
| levofloxacin | 500 mg | oral | 4.74 | 10.8 | 9 | 10.9 | 9.6 | | |
| levofloxacin | 750 mg | IV | | | | 12.94 | | 6.04 | 1.73 |
| levofloxacin | 750 mg | oral | | | | 22.1 | | 9.2 | 1.5 |
| levofloxacin | 750 mg | oral | | | | 22.13 | | 9.19 | 1.55 |
| ciprofloxacin | 500 mg | oral | | | | 1.9 | | 0.4 | |

**Methods of Treatment or Prophylaxis**

Disclosed but not claimed is a method for treating a microbial infection in an animal, specifically including in a mammal, by treating an animal suffering from such an infection with a fluoroquinolone antimicrobial. In some embodiments, fluoroquinolone antimicrobials may be administered following aerosol formation and inhalation. Thus, this method of treatment is especially appropriate for the treatment of pulmonary infections involving microbial strains that are difficult to treat using an antimicrobial agent delivered parenterally due to the need for high parenteral dose levels (which can cause undesirable side effects), or due to lack of any clinically effective antimicrobial agents. In one such embodiment, this method may be used to administer a fluoroquinolone antimicrobial directly to the site of infection. Such a method may reduce systemic exposure and maximizes the amount of antimicrobial agent to the site of microbial infection. This method is also appropriate for treating infections involving microbes that are susceptible to fluoroquinolone antimicrobials as a way of reducing the frequency of selection of resistant microbes. This method is also appropriate for treating infections involving microbes that are otherwise resistant to fluoroquinolone antimicrobials as a way of increasing the amount of antimicrobial at the site of microbial infection. A subject may be identified as infected with bacteria that are capable of developing resistance by diagnosing the subject as having symptoms that are characteristic of a bacterial

infection with a bacteria species known to have resistant strains or a with a bacteria that is a member of group that are known to have resistant strains. Alternatively, the bacteria may be cultured and identified as a species known to have resistant strains or a bacteria that is a member of group that are known to have resistant strains.

In some embodiments, the aerosol fluoroquinolone antimicrobial agent is administered at a level sufficient to overcome the emergence resistance in bacteria or increase killing efficiency such that resistance does not have the opportunity to develop.

In some embodiments, the aerosol fluoroquinolone therapy may be administered as a treatment or prophylaxis in combination or alternating therapeutic sequence with other aerosol, oral or parenteral antibiotics. By non-limiting example this may include aerosol tobramycin and/or other aminoglycoside, aerosol aztreonam and/or other beta or mono-bactam, aerosol ciprofloxacin and/or other fluoroquinolones, aerosol azithromycin and/or other macrolides or ketolides, tetracycline and/or other tetracyclines, quinupristin and/or other streptogramins, linezolid and/or other oxazolidinones, vancomycin and/or other glycopeptides, and chloramphenicol and/or other phenicols, and colisitin and/or other polymyxins.

## Pharmaceutical Compositions

For purposes of the method described herein, a fluoroquinolone antimicrobial agent may be administered using an inhaler. In some embodiments, a fluoroquinolone antimicrobial disclosed herein is produced as a pharmaceutical composition suitable for aerosol formation, good taste, storage stability, and patient safety and tolerability.

In some embodiments, the isoform content of the manufactured fluoroquinolone may be optimized for tolerability, antimicrobial activity and stability.

## Administration

The fluoroquinolone antimicrobials disclosed herein can be administered at a therapeutically effective dosage, e.g., a dosage sufficient to provide treatment for the disease states previously described. While optimum human dosage levels have yet to be determined for aerosol delivery, generally a daily aerosol dose of levofloxacin (and for most fluoroquinolone antimicrobial agents described herein) is from about 0.1 to 10 mg/kg of body weight, preferably about 0.20 to 5.0 mg/kg of body weight, and most preferably about 0.4 to 4.0 mg/kg of body weight. Thus, for administration to a 70 kg person, the dosage range would be about 7.0 to 700.0 mg per day, preferably about 14.0 to 350.0 mg per day, and most preferably about 28.0 to 280.0 mg per day. The amount of active compound administered will, of course, be dependent on the subject and disease state being treated, the severity of the affliction, the manner and schedule of administration, and the judgment of the prescribing physician; for example, a likely dose range for aerosol administration of levofloxacin would be about 20 to 400 mg per day.

Administration of the fluoroquinolone antimicrobial agents disclosed herein or the pharmaceutically acceptable salts thereof can be via any of the accepted modes of administration for agents that serve similar utilities including, but not limited to, aerosol inhalation.

Pharmaceutically acceptable compositions include liquid and aerosol dosage forms. Preferably, the compositions are provided in unit dosage forms suitable for single administration of a precise dose. The unit dosage form can also be assembled and packaged together to provide a patient with a weekly or monthly supply and can also incorporate other compounds such as saline, taste masking agents, pharmaceutical excipients, and other active ingredients or carriers.

The fluoroquinolone antimicrobial agent can be administered either alone or more typically in combination with a conventional pharmaceutical carrier, excipient or the like (e.g., mannitol, lactose, starch, magnesium stearate, sodium saccharine, talcum, cellulose, sodium crosscarmellose, glucose, gelatin, sucrose, magnesium carbonate, magnesium chloride, magnesium sulfate, calcium chloride, lactose, sucrose, glucose and the like). If desired, the pharmaceutical composition can also contain minor amounts of nontoxic auxiliary substances such as wetting agents, emulsifying agents, solubilizing agents, pH buffering agents and the like (e.g., sodium acetate, sodium citrate, cyclodextrin derivatives, sorbitan monolaurate, triethanolamine acetate, triethanolamine oleate, and the like). Generally, depending on the intended mode of administration, the pharmaceutical formulation will contain about 0.005% to 95%, preferably about 0.5% to 50% by weight of a compound of the invention. Actual methods of preparing such dosage forms are known, or will be apparent, to those skilled in this art; for example, see Remington's Pharmaceutical Sciences, Mack Publishing Company, Easton, Pennsylvania.

Liquid pharmaceutically administrable compositions can, for example, be prepared by dissolving, dispersing, etc. an active compound as defined above and optional pharmaceutical adjuvants in a carrier (e.g., water, saline, aqueous dextrose, glycerol, glycols, ethanol or the like) to form a solution or suspension. Solutions to be aerosolized can be prepared in conventional forms, either as liquid solutions or suspensions, as emulsions, or in solid forms suitable for dissolution or suspension in liquid prior to aerosol production and inhalation. The percentage of active compound contained in such aerosol compositions is highly dependent on the specific nature thereof, as well as the activity of the compound and the needs of the subject. However, percentages of active ingredient of 0.01% to 90% in solution are employable, and will be higher if the composition is a solid, which will be subsequently diluted to the above percentages. In some embodiments, the composition will comprise 1.0%-50.0% of the active agent in solution.

Fluoroquinolone formulations can be separated into two groups; those of simple formulation and complex formulations providing taste-masking properties, improved tolerability and/or an AUC shape-enhancing formulation. Simple formulations can be further separated into three groups. 1. Simple formulations may include water-based liquid formulations for nebulization. By non-limiting example water-based liquid formulations may consist of fluoroquinolone alone or with non-encapsulating water soluble excipients. 2. Simple formulations may also include organic-based liquid formulations for nebulization. By non-limiting example organic-based liquid formulations may consist of fluoroquinolone or with non-encapsulating organic soluble excipients. Complex formulations can be further separated into five groups. 1. Complex formulations may include water-based liquid formulations for nebulization. By non-limiting example water-based liquid complex formulations may consist of fluoroquinolone encapsulated or complexed with water-soluble excipients such as lipids, liposomes, cyclodextrins, microencapsulations, and emulsions. 2. Complex formulations may also include organic-based liquid formulations for nebulization. By non-limiting example organic-based liquid complex formulations may consist of fluoroquinolone encapsulated or complexed with organic-soluble excipients such as lipids, microencapsulations, and reverse-phase water-based emulsions. 3. Complex formulations may also include low-solubility, water-based liquid formulations for nebulization. By non-limiting example low-solubility, water-based liquid complex formulations may consist of fluoroquinolone as a low-water soluble, stable nanosuspension alone or in co-crystal/co-precipitate excipient complexes, or mixtures with low solubility lipids, such as lipid nanosuspensions. 4. Complex formulations may also include low-solubility, organic-based liquid formulations for nebulization. By non-limiting example low-solubility, organic-based liquid complex formulations may consist of fluoroquinolone as a low-organic soluble, stable nanosuspension alone or in co-crystal/co-precipitate excipient complexes, or mixtures with low solubility lipids, such as lipid nanosuspensions.

## Aerosol Delivery

Fluoroquinolone antimicrobial agents as described herein, are preferably directly administered as an aerosol to a site of infection in the respiratory tract. In some embodiments, aerosol delivery is used to treat an infection in the lungs, such as a Pseudomonas lung infection.

Several device technologies exist to deliver either dry powder or liquid aerosolized products. Dry powder formulations generally require less time for drug administration, yet longer and more expensive development efforts. Conversely, liquid formulations have historically suffered from longer administration times, yet have the advantage of shorter and less expensive development efforts. The fluoroquinolone antimicrobial agents disclosed herein range in solubility, are generally stable and have a range of tastes. In one such embodiment, the fluoroquinolone antimicrobial levofloxacin is water soluble at neutral pH, is stable in aqueous solution and has limited to no taste.

Accordingly, in one embodiment, a particular formulation of fluoroquinolone antimicrobial agent disclosed herein is combined with a particular aerosolizing device to provide an aerosol for inhalation that is optimized for maximum drug deposition at a site of infection and maximal tolerability. Factors that can be optimized include solution or solid particle formulation, rate of delivery, and particle size and distribution produced by the aerosolizing device.

## Particle Size and Distribution

Generally, inhaled particles are subject to deposition by one of two mechanisms: impaction, which usually predominates for larger particles, and sedimentation, which is prevalent for smaller particles. Impaction occurs when the momentum of an inhaled particle is large enough that the particle does not follow the air stream and encounters a physiological surface. In contrast, sedimentation occurs primarily in the deep lung when very small particles which have traveled with the inhaled air stream encounter physiological surfaces as a result of random diffusion within the air stream.

For pulmonary administration, the upper airways are avoided in favor of the middle and lower airways. Pulmonary drug delivery may be accomplished by inhalation of an aerosol through the mouth and throat. Particles having a mass median aerodynamic diameter (MMAD) of greater than about 5 microns generally do not reach the lung; instead, they tend to impact the back of the throat and are swallowed and possibly orally absorbed. Particles having diameters of about 2 to about 5 microns are small enough to reach the upper- to mid-pulmonary region (conducting airways), but are too large to reach the alveoli. Smaller particles, i.e., about 0.5 to about 2 microns, are capable of reaching the alveolar region. Particles having diameters smaller than about 0.5 microns can also be deposited in the alveolar region by sedimentation, although very small particles may be exhaled. Measures of particle size can be referred to as volumetric mean diameter (VMD), mass median diameter (MMD), or MMAD. These measurements may be made by impaction (MMD and MMAD) or by laser (VMD). For liquid particles, VMD, MMD and MMAD may be the same if environmental

conditions are maintained, e.g. standard humidity. However, if humidity is not maintained, MMD and MMAD determinations will be smaller than VMD due to dehydration during impator measurements. For the purposes of this description, VMD, MMD and MMAD measurements are considered to be under standard conditions such that descriptions of VMD, MMD and MMAD will be comparable. Similarly, dry powder particle size determinations in MMD, and MMAD are also considered comparable.

In some embodiments, the particle size of the aerosol is optimized to maximize fluoroquinolone antimicrobial agent deposition at the site of infection and to maximize tolerability. Aerosol particle size may be expressed in terms of the mass median aerodynamic diameter (MMAD). Large particles (e.g., MMAD >5 μm) may deposit in the upper airway because they are too large to navigate the curvature of the upper airway. Small particles (e.g., MMAD < 2 μm) may be poorly deposited in the lower airways and thus become exhaled, providing additional opportunity for upper airway deposition. Hence, intolerability (e.g., cough and bronchospasm) may occur from upper airway deposition from both inhalation impaction of large particles and settling of small particles during repeated inhalation and expiration. Thus, in one embodiment, an optimum particle size is used (e.g., MMAD = 2-5 μm) in order to maximize deposition at a mid-lung site of infection and to minimize intoleratiblity associated with upper airway deposition. Moreover, generation of a defined particle size with limited geometric standard deviation (GSD) may optimize deposition and tolerability. Narrow GSD limits the number of particles outside the desired MMAD size range. In one embodiment, an aerosol containing one or more compounds disclosed herein is provided having a MMAD from about 2 microns to about 5 microns with a GSD of less than or equal to about 2.5 microns. In another embodiment, an aerosol having an MMAD from about 2.8 microns to about 4.3 microns with a GSD less than or equal to 2 microns is provided. In another embodiment, an aerosol having an MMAD from about 2.5 microns to about 4.5 microns with a GSD less than or equal to 1.8 microns is provided.

Fluoroquinolone antimicrobial agents disclosed herein intended for respiratory delivery (for either systemic or local distribution) can be administered as aqueous formulations, as suspensions or solutions in halogenated hydrocarbon propellants. Aqueous formulations may be aerosolized by liquid nebulizers employing either hydraulic or ultrasonic atomization. A desired particle size and distribution may be obtained by choosing an appropriate device.

## Liquid Nebulizer

In one embodiment, a nebulizer is selected on the basis of allowing the formation of an aerosol of a fluoroquinolone antimicrobial agent disclosed herein having an MMAD predominantly between about 2 to about 5 microns. In one embodiment, the delivered amount of fluoroquinolone antimicrobial agent provides a therapeutic effect for respiratory infections.

Previously, two types of nebulizers, jet and ultrasonic, have been shown to be able to produce and deliver aerosol particles having sizes between 2 and 4 um. These particle sizes have been shown as being optimal for treatment of pulmonary bacterial infection cause by gram-negative bacteria such as Pseudomonas aeruginosa, Escherichia coli, Enterobacter species, Klebsiella pneumoniae, K. oxytoca, Proteus mirabilis, Pseudomonas aeruginosa, Serratia marcescens, Haemophilus influenzae, Burkholderia cepacia, Stenotrophomonas maltophilia, Alcaligenes xylosoxidans, and multidrug resistant Pseudomonas aeruginosa. However, unless a specially formulated solution is used, these nebulizers typically need larger volumes to administer sufficient amount of drug to obtain a therapeutic effect. A jet nebulizer utilizes air pressure breakage of an aqueous solution into aerosol droplets. An ultrasonic nebulizer utilizes shearing of the aqueous solution by a piezoelectric crystal. Typically, however, the jet nebulizers are only about 10% efficient under clinical conditions, while the ultrasonic nebulizer is only about 5% efficient. The amount of pharmaceutical deposited and absorbed in the lungs is thus a fraction of the 10% in spite of the large amounts of the drug placed in the nebulizer.

Accordingly, in one embodiment, a vibrating mesh nebulizer is used to deliver an aerosol of the fluoroquinolone antimicrobial agent disclosed herein. A vibrating mesh nebulizer consists of a liquid storage container in fluid contact with a diaphragm and inhalation and exhalation valves. In one embodiment, about 1 to about 5 ml of the fluoroquinolone antimicrobial agent is placed in the storage container and the aerosol generator is engaged producing atomized aerosol of particle sizes selectively between about 1 and about 5 um.

By non-limiting example, a fluoroquinolone antimicrobial agent disclosed herein is placed in a liquid nebulization inhaler and prepared in dosages to deliver from about 7 to about 700 mg from a dosing solution of about 1 to about 5 ml, preferably from about 14 to about 350 mg in about 1 to about 5 ml, and most preferably from about 28 to about 280 mg in about 1 to about 5 ml with MMAD particles sizes between about 2 to about 5 um being produced.

By non-limiting example, a nebulized fluoroquinolone antimicrobial may be administered in the described respirable delivered dose in less than about 20 min, preferably less than about 10 min, more preferably less than about 7 min, more preferably less than about 5 min, more preferably less than about 3 min, and in some cases most preferable if less than about 2 min.

By non-limiting example, in other circumstances, a nebulized fluoroquinolone antimicrobial may achieve improved tolerability and/or exhibit an AUC shape-enhancing characteristic when administered over longer periods of time. Under these conditions, the described respirable delivered dose in more than about 2 min, preferably more than about

3 min, more preferably more than about 5 min, more preferably more than about 7 min, more preferably more than about 10 min, and in some cases most preferable from about 10 to about 20 min.

For aqueous and other non-pressurized liquid systems, a variety of nebulizers (including small volume nebulizers) are available to aerosolize the formulations. Compressor-driven nebulizers incorporate jet technology and use compressed air to generate the liquid aerosol. Such devices are commercially available from, for example, Healthdyne Technologies, Inc.; Invacare, Inc.; Mountain Medical Equipment, Inc.; Pari Respiratory, Inc.; Mada Medical, Inc.; Puritan-Bennet; Schuco, Inc., DeVilbiss Health Care, Inc.; and Hospitak, Inc. Ultrasonic nebulizers rely on mechanical energy in the form of vibration of a piezoelectric crystal to generate respirable liquid droplets and are commercially available from, for example, Omron Heathcare, Inc. and DeVilbiss Health Care, Inc. Vibrating mesh nebulizers rely upon either piezoelectric or mechanical pulses to respirable liquid droplets generate. Other examples of nebulizers for use with fluoroquinolone antimicrobial agents described herein are described in U.S. Patent Nos. 4,268,460; 4,253,468; 4,046,146; 3,826,255; 4,649,911; 4,510,929; 4,624,251; 5,164,740; 5,586,550; 5,758,637; 6,644,304; 6,338,443; 5,906,202; 5,934,272; 5,960,792; 5,971,951; 6,070,575; 6,192,876; 6,230,706; 6,349,719; 6,367,470; 6,543,442; 6,584,971; 6,601,581; 4,263,907; 5,709,202; 5,823,179; 6,192,876; 6,196,219; 6,644,304; 5,549,102; 6,083,922; 6,161,536; 6,264,922; 6,557,549; and 6,612,303. Commercial examples of nebulizers that can be used with the fluoroquinolone antimicrobial agents described herein include Respirgard II®, Aeroneb®, Aeroneb® Pro, and Aeroneb® Go produced by Aerogen; AERx® and AERx Essence™ produced by Aradigm; Porta-Neb®, Freeway Freedom™, Sidestream,, Ventstream and I-neb produced by Respironics, Inc.; and PARI LC-Plus®, PARI LC-Star®, and e-Flow$^{7m}$ produced by PARI, GmbH.

In some embodiments, the drug solution is formed prior to use of the nebulizer by a patient

## Solution/Dispersion Formulations

Also disclosed is aqueous formulations containing soluble or nanoparticulate drug particles. For aqueous aerosol formulations, the drug may be present at a concentration of about 1 mg/mL up to about 700 mg/mL. Such formulations provide effective delivery to appropriate areas of the lung, with the more concentrated aerosol formulations having the additional advantage of enabling large quantities of drug substance to be delivered to the lung in a very short period of time. In one embodiment, a formulation is optimized to provide a well tolerated formulation. Accordingly, in one embodiment, fluoroquinolone antimicrobial agents disclosed herein are formulated to have good taste, pH from about 5.5 to about 7, osmolarity from about 200 to about 1250 mOsmol/kg, permeant ion concentration from about 30 to about 300 mM.

In one embodiment, the solution or diluent used for preparation of aerosol formulations has a pH range from about 4.5 to about 7.5, preferably from about 5.5 to about 7.0. This pH range improves tolerability. When the aerosol is either acidic or basic, it can cause bronchospasm and cough. Although the safe range of pH is relative and some patients may tolerate a mildly acidic aerosol, while others will experience bronchospasm. Any aerosol with a pH of less than about 4.5 typically induces bronchospasm. Aerosols with a pH from about 4.5 to about 5.5 will cause bronchospasm occasionally. Any aerosol having pH greater than about 7.5 may have low tolerability because body tissues are generally unable to buffer alkaline aerosols. Aerosols with controlled pH below about 4.5 and over about 7.5 typically result in lung irritation accompanied by severe bronchospasm cough and inflammatory reactions. For these reasons as well as for the avoidance of bronchospasm, cough or inflammation in patients, the optimum pH for the aerosol formulation was determined to be between about pH5.5 to about pH 7.0. Consequently, in one embodiment, aerosol formulations for use as described herein are adjusted to pH between about 4.5 and about 7.5 with preferred pH range from about about 5.5 to about 7.5. Most preferred pH range is from about 5.5 to about 7.5.

By non-limiting example, compositions may also include a buffer or a pH adjusting agent, typically a salt prepared from an organic acid or base. Representative buffers include organic acid salts of citric acid, ascorbic acid, gluconic acid, carbonic acid, tartaric acid, succinic acid, acetic acid, or phthalic acid, Tris, tromethamine, hydrochloride, or phosphate buffers.

Many patients have increased sensitivity to various chemical tastes, including bitter, salt, sweet, metallic sensations. To create well-tolerated drug products, by non-limiting example taste masking may be accomplished through the additition of taste-masking excipients, adjusted osmolality, and sweeteners.

Many patients have increased sensitivity to various chemical agents and have high incidence of bronchospastic, asthmatic or other coughing incidents. Their airways are particularly sensitive to hypotonic or hypertonic and acidic or alkaline conditions and to the presence of any permanent ion, such as chloride. Any imbalance in these conditions or a presence of chloride above certain value leads to bronchospastic or inflammatory events and/or cough which greatly impair treatment with inhalable formulations. Both these conditions prevent efficient delivery of aerosolized drugs into the endobronchial space.

In some embodiments, the osmolality of aqueous solutions of the fluoroquinolone antimicrobial agent disclosed herein are adjusted by providing excipients. In some cases, a certain amount of chloride or another anion is needed for successful and efficacious delivery of aerosolized antibiotic. However, it has been discovered that such amounts are

lower than amounts provided and typically used for aerosols of other compounds.

Bronchospasm or cough reflexes do not respond to the same osmolality of the diluent for aerosolization. However, they can be sufficiently controlled and/or suppressed when the osmolality of the diluent is in a certain range. A preferred solution for aerosolization of therapeutic compounds which is safe and tolerated has a total osmolality from about 200 to about 1250 mOsmol/kg with a range of chloride concentration of from about 30 mM to about 300 mM and preferably from about 50 mM to about 150 mM. This osmolality controls bronchospasm, the chloride concentration, as a permeant anion, controls cough. Because they are both permeant ions, both bromine or iodine anions may be substituted for chloride. In addition, bicarbonate may substituted for chloride ion.

By non-limiting example, the formulation for an aerosol fluoroquinolone antimicrobial agent may comprise from about 7 to about 700 mg, preferably from about 14 to about 300 mg, or most preferably from about 28 to about 280 mg fluoroquinolone antimicrobial agent per about 1 to about 5ml of dilute saline (between 1/10 to 1/1 normal saline). Accordingly, the concentration of a levofloxacin solution may be greater than about 25mg/ml, greater than about 35 mg/ml and is preferably greater than about 40 mg/ml, and is as high or greater than 50/ml.

In one embodiment, solution osmolality is from about 100 mOsmol/kg to about 600 mOsmol/kg. In various other embodiments, the solution osmolality is from about 2000 mOsmol/kg to about 1250 mOsmol/kg; from about 250 mOsmol/kg to about 1050 mOsmol/kg; and from about 350 mOsmol/kg to about 750 mOsmol/kg.

In one embodiments, permeant ion concentration is from about 25 mM to about 400 mM. In various other embodiments, permeant ion concentration is from about 30 mM to about 300 mM; from about 40 mM to about 200 mM; and from about 50 mM to about 150 mM.

## Carbohydrates

By non-limiting example, carbohydrate excipients may include monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol), pyranosyl sorbitol, myoinositol, isomalt, trehalose and the like.

## Taste Masking, Flavor, Other

By non-limiting example, compositions may further include flavoring agents, taste-masking agents, inorganic salts (e.g., sodium chloride), antimicrobial agents (e.g., benzalkonium chloride), sweeteners, antioxidants, antistatic agents, surfactants (e.g., polysorbates such as "TWEEN 20" and "TWEEN 80"), sorbitan esters, saccharin, cyclodextrins, lipids (e.g., phospholipids such as lecithin and other phosphatidylcholines, phosphatidylethanolamines), fatty acids and fatty esters, steroids (e.g., cholesterol), and chelating agents (e.g., EDTA, zinc and other such suitable cations). Other pharmaceutical excipients and/or additives suitable for use in the compositions according to the invention are listed in "Remington: The Science & Practice of Pharmacy", 19.sup.th ed., Williams & Williams, (1995), and in the "Physician's Desk Reference", 52.sup.nd ed., Medical Economics, Montvale, N.J. (1998).

By non-limiting example, classes of taste-masking agents for fluoroquinolone formulation include the addition of flavorings, sweeteners, and other various coating strategies. By non-limiting examples these may be chosen from sugars such as sucrose, dextrose, and lactose), carboxylic acids, salts such as magnesium and calcium (non-specific or chelation-based fluoroquinolone taste masking), menthol, amino acids or amino acid derivatives such as arginine, lysine, and monosodioum glutamate, and synthetic flavor oils and flavoring aeromatics and/or natural oils, extracts from plants, leaves, flowers, fruits, etc. and combinations thereof. These may include cinnamon oils, oil of wintergreen, peppermint oils, clover oil, bay oil, anise oil, eucalyptus, vanilla, citrus oil such as lemon oil, orange oil, grape and grapefruit oil, fruit essences including apple, peach, pear, strawberry, raspberry, cherry, plum, pineapple, apricot, etc. Additional sweeteners include sucrose, dextrose, aspartame (Nutrasweet®), acesulfame-K, sucrolose and saccharin, organic acids (by non-limiting example citric acid and aspartic acid). Such flavors may be present at about 0.05 to about 4 percent. Another approach to improve or mask the taste of unpleasant inhaled drugs is to decrease the drugs solubility, e.g. drugs must dissolve to interact with taste receptors. Hence, to deliver solid forms of the drug may avoid the taste response and acquire the desired improved taste affect. Non-limiting methods to decrease fluoroquinolone solubility are described in this document, e.g. salt forms of levofloxacin or gemifloxacin with xinafoic acid, oleic acid, stearic acid and pamoic acid. Additional co-precipitating agents include dihydropyridines and a polymer such as polyvinyl pyrrolidone. Moreover, taste-masking may be accomplished by creation of lipopilic vesicles. Additional coating or capping agents include dextrates (by non-limiting example cyclodextrins may include, 2-hydroxypropyl-beta-cyclodextrin, 2-hydroxypropyl-gamma-cyclodextrin, randomly methylated beta-cyclodextrin, dimethyl-alpha-cyclodextrin, dimethyl-beta-cyclodextrin, maltosyl-alpha-cyclodextrin, glucosyl-1-alpha-cyclodextrin, glucosyl-2-alpha-cyclodextrin, alpha-cyclodextrin, beta-cyclodextrin, gamma-cyclodextrin, and sulfobutylether-beta-cyclodextrin), modified celluloses such as ethyl cellulose,

methyl cellulose, hydroxypropyl cellulose, hydroxyl propyl methyl cellulose, polyalkylene glycols, polyalkylene oxides, sugars and sugar alcohols, waxes, shellacs, acrylics and mixtures thereof. By non-limiting example, other methods to deliver non-dissolved forms of fluoroquinolones are to administer the drug alone or in simple, non-solubilty affecting formulation as a crystalline micronized, dry powder, spray-dried, and nanosuspension formulation. However, an alternative method is to include taste-modifying agents. These include taste-masking substance that is mixd with, coated onto or otherwise combined with the fluoroquinolone active medicament. However, this addition may also serve to improve the taste of another chosen drug product addition, e.g. a mucolytic agent. Non-limiting examples of such substances include acid phospholipids, lysophospholipid, tocopherol polyethyleneglycol succinate, and embonic acid (pamoate). Many of these agents can be used alone or in combination with fluoroquinolones for aerosol administration.

## EXAMPLES

The following examples serve to more fully describe the manner of using the above-described invention, as well as to set forth the best modes contemplated for carrying out various aspects of the invention. It is understood that these examples in no way serve to limit the true scope of this invention, but rather are presented for illustrative purposes.

### Reference Example 1 - High Local Concentration with Short Duration Aerosol Fluoroquinolone Exposure.

Aerosol administration of fluoroquinolones such as levofloxacin produces high concentrations in the epithelial lining fluid (ELF) of rats and humans. However, this dose has been observed to rapidly decline following administration.

In order to determine if short duration, high concentrations of levofloxacin could be effective in treatment of *P. aeruginosa* (PA), studies were conducted to measure their bactericidal activity on various strains of this organism which were grown at different conditions. Those were chosen based on what is known about conditions and growth of PA in a cystic fibrosis (CF) lung. Four isogenic strains of *P. aeruginosa* were used for these experiments (Table 2).

**Table 2. Strains of PA Used in Time-Kill Experiments.**

| Strain | Genotype | Levofloxacin MIC (ug/ml) |
|---|---|---|
| PAM1020 | wt | 0.25 |
| PAM1032 | *nalB* | 1 |

PAM1020 is the parent wild-type strain, PAM1032 contains *nalB* mutation which results in increased levofloxacin resistance due to overexpression of the MexAB-OprM efflux pump which can extrude levofloxacin out of cells.

### Experiment 1. Activity of Levofloxacin Against Exponentially Grown Cells.

***Methods***

***Inoculum Preparation***

Strains were grown aerobically overnight in Mueller-Hinton Broth (MHB) at 35° C. Next, cultures were diluted 1:1000 into 100 ml of fresh MHB and grown to $OD_{600}$~0.3 to reach CFU/ml ~ $10^8$. 10 ml of this culture was moved to 50 ml flasks, each containing 10 ml of pre-warmed MHB broth with appropriate concentrations of levofloxacin (2X as compared to the exposure concentrations).

***Exposure***

All strains were treated for 10 min., 20 min., 40 min., 80 min. and 160 minutes. The following concentrations of levofloxacin (ug/ml) were used for the exposure of PAM1020 and PAM1032: 16, 32, 64, 128 and 256. All strains were treated at each concentration for 10 min., 20 min., 40 min., 80 min. and 160 minutes.

***Determination of viable cell numbers***

At appropriate times, 1 ml of each exposure culture was centrifuged for 2 minutes, the pellet was washed twice with 1 ml of drug-free MHB, and re-suspended in 1 ml of MHB. The viable cell numbers were enumerated by plating serially diluted samples (in duplicates) on MHB plates by the drop (10 ul) plating method. The limit of detection was 100 CFU/ml. Killing is reported as the log reduction calculated relative to cell count at the time of initiation of antibiotic

exposure. Relative antibiotic concentrations (relative to MIC of the corresponding strains) are used. Cell numbers at initiation of antibiotic exposure are shown in Table 3.

**Table 3. Bacterial Numbers at Time of Initial Bacterial Exposure.**

| Strain | CFU/ml |
|---|---|
| PAM1020 | 4.03E+07 |
| PAM1032 | 5.60E+07 |

***Results***

For the most susceptible strain, PAM1020, maximum killing (5.5 log decrease in viable cell counts) was achieved after incubation for 10 minutes with the concentration of levofloxacin corresponding to 256-fold MIC (64 ug/ml tested). 5-logs of killing were achieved already with the lowest concentration tested (16 ug/ml or 64-fold MIC) (Figure 4A). For the strain PAM1032, as long as the concentration above 128-fold the MIC (128 ug/ml) was reached, 10 minute of exposure was sufficient to result in maximum killing (more than 5 logs). At short duration exposures (10 or 20 minutes), less killing was observed at concentrations below 128-fold of MICs. At longer exposure times, concentration corresponding to 16-fold MICs and above resulted in similar maximum killing (Figure 4B). These results inducate that logarithmic cells of *P. aeruginosa* are efficiently killed after short duration exposures to high concentrations of levofloxacin.

**Experiment 2. Activity of Levofloxacin Against Stationary Phase Cells.**

***Methods***

***Inoculum Preparation***

Strains were grown aerobically overnight in Mueller-Hinton Broth (MHB) at 35°C (350 ml total). The spent medium was obtained after centrifugation of overnight cultures and filtering the supernatant. Cultures were diluted to OD=0.3 into spent medium. The same medium was also used to prepare antibiotic concentrations (the same as in Experiment 1).

***Exposure***

Antibiotic concentrations, time of exposure as determination of viable cell counts were the same as in Experiment 1.

***Results***

Cell numbers at initiation of antibiotic exposure are shown in Table 4.

**Table 4. Bacterial Numbers at Time of Initial Bacterial Exposure.**

| Strain | CFU/ml |
|---|---|
| PAM1020 | 8.0E+08 |
| PAM1032 | 8.50E+08 |

For stationary phase cells of PAM 1020, maximum killing was observed at the lowest concentration corresponding to 64-fold above MIC (16 ug/ml) and the shortest duration of exposure, 10 minutes (Figrue 5A). However, PAM1032 demonstrated clear dose-dependent killing with the maximum killing (4 logs) at concentrations 64 the MIC at a short exposure time. Extending the exposure times did not result in larger extent of killing. However, lower concentrations of drug were required to achieve the same killing at longer exposure times (Figure 5B).

Next, we have compared the re-growth of PAM1020 and PAM1032 after either 10 minutes or 160 minutes of treatment with various concentrations of levofloxacin. After the corresponding treatments, cells were washed twice with antibiotic-free medium. 150 µl of cells was placed into 96-well plate and the growth was continuously monitored at $A_{660}$ using SpectraMax (Molecular Devices). The results are shown in Figure 6A-6D.

The results demonstrate that the re-growth of both strains was observed at approximately the same time whether

cells were treated with high concentrations of levofloxacin for 10 minutes or 160 minutes. These results further support the efficiency of short duration treatment with high concentrations of levofloxacin.

**Experiment 3. Activity of Levofloxacin Against Cells Grown Under Oxygen-Limiting Conditions**

***Methods***

***Inoculum Preparation***

Overnight cultures were grown aerobically overnight in Mueller-Hinton Broth and next diluted 1:10000 in MHB which filled growth flasks to the very top. Cultures were grown without shaking to OD~0.3 at 37°C. Under these conditions an average of ~20 hours was required to reach an OD=0.3 as compared to ~5 hours under aerated conditions (50 ml of medium in 250 ml flasks, vigorous shaking). Upon analysis, it appeared that an OD=0.3 corresponded to a late-logarithmic phase of growth. Other than decreased aeration, antibiotic concentration, time of exposure, and determination of viable cell counts were the same as in Experiments 1 and 2.

***Results***

Cell numbers at initiation of antibiotic exposure are shown in Table 5.

**Table 5. Bacterial Numbers at Time of Initial Bacterial Exposure.**

| Strain | CFU/ml |
|---|---|
| PAM1020 | 7.5E+07 |
| PAM1032 | 8.5E+07 |

In the case of PAM1020 near maximum killing (4 logs vs 4.5 logs observed under normal aeration) was achieved after exposure with the lowest concentration of levofloxacin for the shortest duration of time (10 minutes) (Figure 7A). In the case of PAM1032 dose-dependent killing was observed for 10 minutes or 20 minutes of exposure with the highest killing observed at concentrations corresponding to 128 to 256-fold the MIC. Slightly stronger (less than 1 log difference) killing was observed for longer exposure intervals (Figure 7B). These data indicate that under conditions of oxygen limitation cells which are at the late logarithmic phase of growth are efficiently killed after short duration of exposure with high concentrations of levofloxacin.

**Experiment 4. Activity of Levofloxacin Against PAM1032 in CF Sputum.**

***Methods***

Cells of strain PAM1032 (MIC = 1 ug/ml) were grown to OD=1 (late-exponential/early stationary phase of growth) in MHB and next concentrated 10-fold in 10-fold concentrated MHB. 10 ul of cells were then added to 90 ul of sputum or water in 96-well round bottom plates, restoring MHB to its original concentration. Quantitation plates were pre-warmed for 5 minutes at 37°C and different concentrations of levofloxacin (512 ug/ml, 128 ug/ml, 32 ug/ml, 8 ug/ml, 2 ug/ml, and 0.5 ug/ml) were added. At appropriate times, 10 ul of each treatment culture was diluted 100-fold in MHB to minimize the carryover of levofloxacin. Viable cell numbers were enumerated by plating serially diluted samples on MHB plates by the drop (10 ul) plating method. The limit of detection was $10^4$ CFU/ml. Killing is reported as the percentage of the starting inoculum survived after the levofloxacin treatment. Results are shown in Figures 8A and 8B.

***Results***

The results indicate that while sputum slightly affected the degree of killing by levofloxacin, rapid and extensive (up to five orders of magnitude) killing by levofloxacin in sputum was still observed after short duration of treatment at high concentrations of antibiotic.

## Experiment 4. Activity of Levofloxacin Against Colony Biofilms of PAM1032.

### *Methods*

#### *Biofilm Preparation*

Colony biofilms were grown on polycarbonate membrane filters (diameter, 25 mm; Poretics, Livermore, CA) resting on MHB plates. Overnigh culture of PAM1032 was diluted to OD=0.3, and then diluted 1:100 in fresh MHB. 5 ul of this culture was spotted on the membrane filter. Bacteria were incubated at 37°C for 48 hours (mature biofilms).

#### *Exposure*

After growth filters were placed into tubes containing 3 ml saline or saline and levofloxacin at 128 ug/ml and 1024 ug/ml. Each tube was treated for 10 minutes and 80 minutes. At about 5 min. before incubation time elapsed, tubes were vigorously vortexed (A) or sonicated and vortexed (B) to detach cells. 1 ml of each exposure culture was centrifuged for 2 minutes, the pellet was washed twice with1 ml of drug-free MHB, and re-suspended in 1 ml of MHB. The viable cell numbers were enumerated by plating serially diluted samples (in duplicates) on MHB plates by the drop (10 ul) plating method. Results are shown in Figure 9.

### *Results*

The data demonstrate that maximum killing (~2 logs) is obtained after 10 min with the lowest concentration of levofloxacin tested (128-fold MIC). No additional killing was observed at the higher levofloxacin concentration. These data indicate that colony biofilms are more resistant to killing as compared to logarithmic or stationary phase cells. However, the maximum observed bactericidal activity against biofilms (99% under these conditions) was achieved after 10 minutes of levofloxacin exposure.

## Experiment 5. Simulated Short-Term, Rapid Aerosol Administration, Delivering High Concentration Drug Exposure in in vitro Pharmacodynamic Model.

In vitro pharmacodynamic models of infection allow for exposure of a growing bacterial inoculum to changing concentrations of drug as would occur in vivo. The strength of this approach is that the serum concentration vs. time profile of a drug in man can be simulated in the laboratory in vitro to determine the optimal exposure profile (i.e., dose and dosing interval) for a drug and target pathogen.

The following report describes experiments designed to determine the Cmax and AUC that will provide maximum bactericidal effects after an aerosol dose of a fluoroquinolone.

### *Material and Methods*

#### *In Vitro Pharmacodynamic Model of Infection*

The in vitro pharmacodynamic model consists of a central (analogous "serum" compartment) and peripheral ("extravascular") compartment. The peripheral compartments consist of artificial capillary units (Unisyn, Hopkinton, MA) arranged in series with the central compartment. Each capillary unit has a bundle of small semi-permeable fibers with a molecular size retention of ca. 10,000 MW to allow passage of nutrients but not bacteria. The entire system is set up in a dry heat incubator adjusted to 37°C.

Both the central and peripheral compartments were filled with Mueller-Hinton broth. Each peripheral compartment (capillary unit and tubing) contained ca. 23 ml of growth medium.

Bacteria were introduced into the peripheral chamber of the model and allowed to grow for 2 hours prior to the first "dose" of drug. Drug doses were administered into the central compartment and pumped to peripheral chambers by a peristaltic pump. Concentrations in the model declined according to first order elimination (half-life) by dilution of the central compartment with drug free medium introduced by an additional peristaltic pump adjusted to the desired clearance.

Samples (0.3 ml) were collected from peripheral compartments at various intervals for determination of drug and bacterial concentrations. Samples were collected from the peripheral compartments and assayed for drug concentrations by HPLC.

***Bacterial Test Strains***

Pseudomonas aeruginosa PAM1032 and PAM1582. The MICs of these strains to levofloxacin were 1.0 and 32 ug/ml, respectively.

***Inoculum Preparation***

Strains were grown aerobically overnight in Mueller-Hinton Broth (MHB) at 35°C and subcultured to fresh MHB and reincubated at 35°C for 2 hours. After 2 hours, the inoculum was further diluted 1:1000 to a final concentration of approx. 1.0 x 106 CFU/ml. Of the resulting dilution, 2.3 ml was injected into each peripheral chamber of the hollow-fiber bioreacters (Unisyn, Hopkinton, MA).

***Pharmacokinetics***

The half-life of levofloxacin was adjusted to be 10 minutes to be equivalent to that observed following aerosol delivery of levofloxacin to the pulmonary compartment of man. The targeted Cmax was 1000 and 600 ug/ml over two experiments.

***Results***

As targeted, the model exhibited a levofloxacin half-life of 10 minutes and the Cmax of 1000 ug/ml for Experiment 5. By comparison, Experiment 6 was modified to achieve the same half-life as Experiment 5, but with a targeted Cmax of 600 μg/ml.

The bactericidal effects of these two regimens correlated with the Cmax. In Experiment 5 with a Cmax of 1000 ug/ml, the maximum bactericidal effect was observed as a 5 log reduction in bacterial counts within 10 minutes with PAM1032 and a 4 log reduction in bacterial counts within 20 minutes with PAM1582 and no re-growth observed over the remaining 2 hours of the experiment (Figure 10). In contrast, while the Cmax of 600 ug/ml used in Experiment 6 maintained the 5-log reduction in bacterial counts for PAM1032, albeit taking 30 min instead of 10 min observed in Experiment 1, only a 3-log reduction in bacterial counts was observed for PAM1582 after 45 min (Figure 11). Moreover, PAM1582 exhibited initial re-growth before the end of the 2 hour experimental window.

**Conclusions**

Levofloxacin can produce up to a 99.9999% bacterial reduction with a Cmax of both 600 and 1000 ug/ml against a strain with an MIC of 1 ug/ml. However, maximal bactericidal activity requires 3X more time at a Cmax of 600 ug/ml. Levofloxacin can also produce up to 99.99% bacterial reduction with a Cmax of both 600 ug/ml against a strain with an MIC of 32ug/ml. However, the time to reach the maximum effect is 45 minutes. In contrast, levofloxacin can produce up to 99.999% bacterial reduction of this resistant strain with a Cmax of 1000 ug/ml and the time to maximum effect is reduced to 20 minutes. From these results, extremely high, but short duration exposures of levofloxacin produce rapid and sustained bacterial killing in both flask and hollow fiber models. Taken together, the above results indicate that achieving an initial 800 ug/ml levofloxacin or other fluoroquinolone human ELF or sputum concentration is sufficient to achieve the above antibiotic affects for the MIC99 population as represented by PAM1582 (MIC of 32 ug/ml).

**Example** 2 - **Determination of the Aerosol Properties of Antibacterial Fluoroquinolones.**

**Introduction**

Objective. The purpose of these studies was to evaluate the ability to formulate and deliver by nebulization a variety of fluoroquinolones for treatment of pulmonary bacterial infections by aerosol administration. The fluoroquinolones evaluated are shown in Table 6.

**Table 6. Fluoroquinolones Tested.**

| Fluoroquinolone | Sp $MIC_{90}$ (ug/mL) | MSSA $MIC_{90}$ (ug/mL) | MRSA $MIC_{90}$ (ug/mL) | Pa $MIC_{90}$ (ug/mL) | Approval Status |
|---|---|---|---|---|---|
| Ciprofloxacin* | 2 | 1 | 64 | 8 | Approved |
| Gemifloxacin* | 0.06 | 0.06 | 2 | 8 | Approved |

(continued)

| Fluoroquinolone | Sp $MIC_{90}$ (ug/mL) | MSSA $MIC_{90}$ (ug/mL) | MRSA $MIC_{90}$ (ug/mL) | Pa $MIC_{90}$ (ug/mL) | Approval Status |
|---|---|---|---|---|---|
| Levofloxacin | 2 | 0.5 | 16 | 8 | Approved |
| Marbofloxacin* | 2 | 2 | ND | 8 | Veterinary |
| Gatifloxacin* | 0.5 | 0.125 | 4 | 16 | Approved |
| Ofloxacin | 2 | 1 | >32 | 16 | Approved |
| Tosufloxacin* | 0.5 | 0.125 | >16 | 16 | Japan |
| Lomefloxacin* | 16 | 2 | >32 | 32 | Approved |
| Moxifloxacin* | 0.25 | 0.125 | 2 | 32 | Approved |
| Sparfloxacin* | 0.5 | 0.125 | 16 | 32 | Withdrawn |
| Orbifloxacin* | 2 | 2 | ND | >32 | Veterinary |
| Pefloxacin* | 32 | 2 | >32 | >32 | Europe |
| Trovafloxacin* | 0.25 | 0.06 | 8 | >32 | Withdrawn |
| *disclosed but not claimed. | | | | | |

These fluoroquinolones were chosen based on their availability, approval status and antimicrobial properties. All tested fluoroquinolones are either currently approved in the United States or have been approved but later withdrawn due to various adverse reactions. In addition, several fluoroquinolones, which are in use for veterinary applications, have also been evaluated. Among bacterial pathogens responsible for respiratory tract infections, *Pseudomonas aeruginosa* (Pa) and methicillin resistant *Staphylococcus aureus* (MRSA) are the most refractory to treatment with fluoroquinolones. *Streptocossus pneumonia* (Sp) is probably the most important pathogen responsible for respiratory tract infections and numerous reports demonstrate high rates of fluoroquinolone resistance in these bacteria. $MIC_{90}$ for Pa ranges from 4 ug/ml to 32 ug/ml and from 2 ug/ml to >32 ug/ml for Pa and MRSA, respectively. Ciprofloxacin, levofloxacin, gemifloxacin and gatifloxacin vs gemifloxacin and moxifloxacin are the most potent against Pa and MRSA, respectively.

Table 7 contains a list of additional fluoroquinolones for potential evaluation (disclosed but not claimed). The most microbiologically interesting compounds in the list are clinafloxacin and olamufloxacin, which were discontinued due to adverse reactions, and sitofloxacin, which is in Phase III clinical trials.

**Table 7. Fluoroquinolones For Potential Evaluation.**

| Fluoroquinolone | Sp MIC90 (ug/mL) | Sa $MIC_{90}$ (ug/mL) | MRSA $MIC_{90}$ (ug/mL) | Pa $MIC_{90}$ (ug/mL) | Market Status |
|---|---|---|---|---|---|
| Clinafloxacin | 0.06 | 0.06 | 2 | 4 | Discontinued |
| Sitafloxacin | 0.06 | 0.125 | 4 | 8 | Phase III |
| Olamufloxacin | 0.06 | 1 | 2 | 16 | Discontinued |
| Norfloxacin | 16 | 1 | >4 | 16 | Approved |
| Prulifloxacin | 1 | 0.25 | 32 | 16 | Phase III |
| Danofloxacin | NA | 0.125 | NA | >16 | Veterinary |
| Enrofloxacin | 1 | 0.125 | 8 | >16 | Veterinary |
| Sarafloxacin | NA | 0.25 | >16 | >16 | Veterinary |
| Balofloxacin | 0.5 | 0.25 | 8 | 32 | Korea |
| Fleroxacin | 8 | 1 | >4 | 32 | Europe |
| Difloxacin | 2 | 0.5 | NA | 32 | Veterinary |
| Rufloxacin | 32 | 2 | 64 | 32 | Europe, China |

(continued)

| Fluoroquinolone | Sp MIC90 (ug/mL) | Sa $MIC_{90}$ (ug/mL) | MRSA $MIC_{90}$ (ug/mL) | Pa $MIC_{90}$ (ug/mL) | Market Status |
|---|---|---|---|---|---|
| Enoxacin | 16 | 1 | >4 | >32 | Withdrawn |
| Garenoxacin | 0.06 | 0.06 | 8 | >32 | Phase III |
| Grepafloxacin | 0.5 | 0.125 | 32 | >32 | Withdrawn |
| Pazufloxacin | 4 | 0.5 | >16 | >32 | Japan |

The fluoroquinolones in these two tables represent one field of options for an aerosol fluroquinolone candidate. Several potent fluoroquinolones such as DX-619 and DW-286, which are at the early stage of clinical development, might also be of interest for future studies.

Specific physico-chemical considerations for nebulization include aqueous solubility, viscosity and surface tension. The aqueous solubility of the drug should advantageously be sufficient to meet or exceed the minimal dosing requirement. The loading drug concentration also affects delivery time. Longer delivery times may be commercially unacceptable or lead to poor patient compliance. Although longer delivery times may in effect modify the AUC shape, by non-limiting example, the PARI eFlow device has been discovered to administer 4 ml of aqueous levofloxacin in less than 5 min. Moreover, using such an efficient device, high concentration levofloxacin may be able to deliver the effective doses described herein in a timeframe further enabling the rapid administration, high concentration drug requirements needed for optimal fluoroquinolone therapy.

In the case of fluoroquinolones, pH directly affects solubility. In general, solubility decreases significantly with increase in pH in the range 1.5 to 6.5. Because pH also affects patient tolerability (see below), the optimal choice of fluoroquinolone for pulmonary delivery via aerosol has certain solubility and pH levels.

For the purpose of this feasibility study, the target solubility was set at 10 mg/mL or higher at a pH of 4.5 or greater, based on calculations of therapeutic dose and the delivery metrics for available nebulizers. In order to be above the mutant prevention concentration (MPC), peak concentration of fluoroquinolone after aerosol administration advantageously reaches about 100 ug/ml to about 1000 ug/ml at the site of infection, pending the MIC of the infecting organism. Based on these considerations, the minimal dose to be in this therapeutically relevant range was projected to be at least about 30-40 mg Respirable Delivered Dose (RDD). Given the relative half-life of levofloxacin in the human lung, the practical achievement of this dose by nebulization may be obtained with a loading dose of at least about 100 mg in a volume of about 2 mL (about 50 mg/mL) in a high-efficiency vibrating-mesh device operating at its maximum performance efficiency delivering this dose in less than 4 min. A standard ultrasonic or jet nebulizer may require a loading dose of at least about 400 mg in a volume of about 5 mL (about 80 mg/mL). However, the rate of administration by these less efficient devices may not be sufficient to achieve high local concentration with short duration exposure. Similar efficacious doses may also be achieved by administration of levofloxacin as a dry powder, where the rapid solubility properties of levofloxacin may permit a quick dissolution resulting in these desired soluble drug concetrations. However, alternative concentrations or alteration of the fluoroquinolone AUC shape profile may be desirable.

Alternatively, although aqueous solubility is important, it is reasonable to predict a formulation utilizing particle or complexation technology to enable nebulization of less soluble fluoroquinolones. Unfortuantely, more intricate formulations increase both the complexity and cost of drug development, and in the case of jet and ultrasonic nebulizers, a significant reduction in efficiency of delivery, and limit the ability to introduce other design elements into a final drug product.

In addition to drug solubility, for vibrating mesh devices nebulization is also sensitive to the surface tension of the drug formulation. Therefore, in one embodiment, the surface tension is adjusted during formulation by modifying drug concentration, excipient concentration and/or the addition of surfactant.

In addition to factors that affect efficient nebulization, other factors may be considered for patient tolerability and compliance. By non-limiting example, these factors may include osmolality, pH and taste. Osmolality affects acute tolerability in the respiratory tract and can be optimized for most drugs during formulation. Similarly, the pH of an aerosol also contributes to tolerability, yet only negatively when the formulation pH is less than 4.5. Therefore, because pH directly contributes to fluoroquinolone solubility, fluoroquinolones that require a pH less than 4.5 for solubility are likely to be poorly tolerated. Finally, fluoroquinolone taste can affect good patient compliance. Fluoroquinolones are known generally to be associated with an unpleasant, sometimes very intense taste. While there are technologies available that may mask poor drug taste, these technologies increase development complexity and cost, and may not be totally effective in the case of fluoroquinolones. Thus, similar to pH, taste may be considered in identifying a fluoroquinolone suitable for nebulization.

**Preparation and Characterization of the Test Solutions**

Antibiotics were purchased from one of several sources as shown in Table 8.

**Table 8. Preparation of Fluoroquinolone Test Solutions (disclosed but not claimed).**

| No. | Fluoro-quinolone | Source [a] | Purity [b] | Amount | Volume $H_2O$ | Final Conc. |
|---|---|---|---|---|---|---|
| 1 | Gatifloxacin | LKT | 99.6 | 8.7 mg | 0.87 mL | 10 mg/mL |
| 2 | Gemifloxacin | LG | 99.6 | 9.5 mg | 0.95 mL | 10 mg/mL |
| 3 | Levofloxacin | LKT | 99.2 | 10.3 mg | 1.03 mL | 10 mg/mL |
| 4 | Moxifloxacin | LKT | 99.5 | 12.5 mg | 1.25 mL | 10 mg/mL |
| 5 | Ciprofloxacin | LKT | 99.3 | 19.5 mg | 1.95 mL | 10 mg/mL |
| 6 | Ofloxacin | LKT | 99.1 | 11.7 mg | 1.17 mL | 10 mg/mL |
| 7 | Lomefloxacin | MPI | NA | 17.0 mg | 1.70 mL | 10 mg/mL |
| 8 | Marbofloxacin | Vetoquino | NA | 4.8 mg | 0.48 mL | 10 mg/mL |
| 9 | Orbifloxacin | MPI | NA | 4.2 mg | 0.42 mL | 10 mg/mL |
| 10 | Pefloxacin | MPI | NA | 15.0 mg | 1.50 mL | 10 mg/mL |
| 11 | Sparfloxacin | MPI | NA | 14.5 mg | 1.45 mL | 10 mg/mL |
| 12 | Tosufloxacin | MPI | NA | 15.2 mg | 1.52 mL | 10 mg/mL |
| 13 | Trovafloxacin | MPI | NA | 2.0 mg | 0.20 mL | 10 mg/mL |

a. LKT: LKT Laboratories. LG: LG Chem. NA. Source unavailable.
b. Purity of material tested. Described as GMP or in percent API.
c. 25 mg/ml solution.

A 2 to 20 mg sample of each antibiotic was weighed into sterile plastic tubes and brought up with a volume of sterile water to make a 10 mg/mL solution or suspension of the antibiotic. Samples were incubated for approximately 10 minutes at room temperature with occasional mixing, prior to further handling.

Following the incubation period, the antibiotic solutions were observed for their visible appearance, with results as shown in Table 9.

Five of the fluoroquinolones tested were visibly soluble, and either colorless, or a shade of yellow. Eight were visibly insoluble, appearing either cloudy (fine particulate), opaque (dense fine to medium particulate), or turbid (thick, large particulate slurry), in all cases with a visible sediment. The pH of these initial solutions were determined, and ranged from 3.5 to 7.0. The insoluble solutions were titrated with 1N HCl to the point of visible solubility, and the pH of the solubilized solution determined. In three cases, marbofloxacin, sparfloxacin and tosufloxacin, solubility was not reached by pH 1.5, and further addition of acid was stopped. With the exception of ofloxacin, the pH of these titrated solutions was in the range of 1.5 to 3.0.

**Table 9. Flouroquinolone Solution Characteristics (disclosed but not claimed).**

| | | Initial Solution | | After pH Adjustment | | |
|---|---|---|---|---|---|---|
| No. | Fluoro-quinolone | Appearance | pH | 1NHCl (uL) | Appearance [a] | pH |
| | Gatifloxacin | white, cloudy, visible sediment | 7.0 | 5 | slight yellow color, transparent, no sediment | 3.0 |
| | Gemifloxacin | colorless, transparent, no sediment | 4.7 | | NR | - |
| | Levofloxacin | slight yellow color, transparent, no sediment | 4.7 | | NR | - |
| | Moxifloxacin | bright yellow color, transparent, no sediment | 4.7 | | NR | - |

(continued)

| | | Initial Solution | | After pH Adjustment | | |
|---|---|---|---|---|---|---|
| No. | Fluoro-quinolone | Appearance | pH | 1NHCl (uL) | Appearance [a] | pH |
| | Ciprofloxacin | white, opaque (very dense), visible sediment | 5.5 | 60 | colorless, transparent, no sediment | 2.0 |
| | Ofloxacin | cloudy, visible sediment | 6.5 | 10 | slightly yellow color, transparent, no sediment | 5.2 |
| | Lomefloxacin | cloudy, visible sediment | 4.2 | - | transparent, no sediment, after 10 min. at rm. temp. | - |
| | Marbofloxacin | white, very turbid, visible sediment | 6.5 | 40 | white, turbid, visible sediment | 1.5 |
| **9** | Orbifloxacin | white, cloudy, visible sediment | | 20 | colorless, transparent, no sediment | 1.7 |
| **10** | Pefloxacin | colorless, transparent, no precipitate | 4.5 | | NR | - |
| **11** | Sparfloxacin | bright yellow, turbid, visible sediment | 5.0 | 20 | bright yellow, densely turbid, visible sediment | 1.5 |
| **12** | Tosufloxacin | white, turbid, visible sediment | 3.5 | 20 | white, cloudy, less turbid, visible sediment | 1.5 |
| **13** | Trovafloxacin | colorless, slightly cloudy, no sediment | 4.2 | | NR | - |
| a. NR: pH adjustment not required. Fluoroquinolone was soluble at a pH ≥ 4 in the initial solution. | | | | | | |

After the pH adjustment, and following a further 10 minute incubation period with occasional mixing, the final appearance of the solutions was determined, just prior to the aerosol tolerability and taste test. Results are shown in Table 10.

**Table 10. Appearance of Fluoroquinolone Final Solution (disclosed but not claimed).**

| No. | Fluoroquinolone | pH | Solubility | Color | Sediment | Opaqueness |
|---|---|---|---|---|---|---|
| **1** | Gatifloxacin | 3.0 | + | C | none | none to very slight |
| **2** | Gemifloxacin | | + | C | none | none to very slight |
| **3** | Levofloxacin | 4.7 | + | VLY | none | none |
| **4** | Moxifloxacin | 4.7 | + | Y | +/- | none |
| **5** | Ciprofloxacin | 2.0 | + | C | none | none |
| **6** | Ofloxacin | 5.2 | + | LY | +/- | none |
| **7** | Lomefloxacin | 4.2 | + | C | +/- | none to very slight |
| **8** | Marbofloxacin | 1.5 | -- | W | ++ | ++ |
| **9** | Orbifloxacin | 1.7 | + | C | none | slight |
| **10** | Pefloxacin | 4.5 | + | C | none | slight |
| **11** | Sparfloxacin | 1.5 | --- | DY | +++ | ++++ |
| **12** | Tosufloxacin | 1.5 | -- | W | ++ | +++ |
| **13** | Trovafloxacin | 4.2 | + | C | + | slight |
| Y=yellow; LY= light yellow; VLY=very light yellow; DY=dark yellow; C= colorless; W = white. | | | | | | |

The compounds exhibiting preferred solubility for solutions suitable for delivery by inhalation (10 mg/mL at a pH of 4.5 or above), were levofloxacin, gemifloxacin, moxifloxacin, ofloxacin and pefloxacin. Levofloxacin, ofloxacin and moxifloxacin exhibited the best solubility/pH characteristics.

## Taste and Tolerability Evaluation (disclosed but not claimed)

Two evaluations were done to determine the suitability of the fluoroquinolone solutions with respect to taste and tolerability.

First, in an oral taste test the taste of a 20 uL portion of test sample was determined in a single, healthy human volunteer by placing the material directly onto the center front part of the tongue. Taste was then monitored over a 1 minute period. This test was performed on the initial solutions prepared as well as the final solutions following pH adjustment. Data are shown in Table 11.

**Table 11. Oral Fluoroquinolone Taste Test.**

| No. | Fluoroquinolone | Initial Solution | Final Solution |
|---|---|---|---|
| 1 | Gatifloxacin | moderate bitter unpleasant taste, slightly aromatic | strong bitter unpleasant almond-like taste, strong aftertaste |
| 2 | Gemifloxacin | very bitter unpleasant taste with strong aftertaste, all the way into throat | not performed |
| 3 | Levofloxacin | slight chemical taste, slightly bitter, slight almond-like taste | not performed |
| 4 | Moxifloxacin | moderate bitter-sweet unpleasant taste, slightly aromatic | not performed |
| 5 | Ciprofloxacin | sweet almond-like taste | very strong bitter taste all the way into the throat |
| 6 | Ofloxacin | bitter unpleasant, almond-like taste | moderate bitter unpleasant, almond-like taste |
| 7 | Lomefloxacin | moderate to strong almond-like taste, not very unpleasant | not performed |
| 8 | Marbofloxacin | bitter unpleasant almond-like taste | moderate to strong bitter unpleasant almond-like taste |
| 9 | Orbifloxacin | strong unpleasant taste | very strong, very unpleasant bitter taste |
| 10 | Pefloxacin | strong bitter unpleasant almond-like taste | not performed |
| 11 | Sparfloxacin | slight taste | strong almond-like taste |
| 12 | Tosufloxacin | mild to moderate almond-like taste | strong almond-like taste |
| 13 | Trovafloxacin | very strong bitter unpleasant almond-like taste, strong aftertaste | not done |

Lowering of the pH generally had the effect of enhancing the taste properties of the solution. Gatifloxacin, gemifloxacin, ciprofloxacin, orbifloxacin and trovafloxacin were the least desirable in taste testing. Of the fluoroquinolones tested, Levofloxacin was the only fluoroquinolone that was tolerable with respect to taste, at the concentration tested. Lomefloxacin had a moderately strong almond-like taste, and the taste was slightly unpleasant.

In the second test, the tolerability and taste of a small aerosol sample from a 0.5 ml aliquot of the test formulation was determined in a single healthy human volunteer, following nebulization in a PARI eFlow nebulizer (Table 12).

**Table 12. Aerosol Fluoroquinolone Tolerability and Taste Test (disclosed but not claimed).**

| No. | Fluoroquinolone | Aerosol Tolerability and Taste |
|---|---|---|
| 1 | Gatifloxacin | moderate bitter unpleasant taste, mild cough sensation |

(continued)

| No. | Fluoroquinolone | Aerosol Tolerability and Taste |
|---|---|---|
| 2 | Gemifloxacin | strong unpleasant bitter taste, strong aftertaste, mild cough sensation |
| 3 | Levofloxacin | chemical taste, somewhat bitter, mild cough sensation |
| 4 | Moxifloxacin | moderate bitter unpleasant taste, some cough, strong bitter aftertaste |
| 5 | Ciprofloxacin | very strong, bitter unpleasant taste, immediate coughing |
| 6 | Ofloxacin | bitter chemical taste, mild cough sensation |
| 7 | Lomefloxacin | chemical taste, somewhat bitter, mild cough sensation |
| 8 | Marbofloxacin | too insoluble to test |
| 9 | Orbifloxacin | very acidic, strong bitter unpleasant taste, strong cough |
| 10 | Pefloxacin | chemical taste, some cough |
| 11 | Sparfloxacin | too insoluble to test |
| 12 | Tosufloxacin | too insoluble to test |
| 13 | Trovafloxacin | bitter unpleasant taste, no cough or cough sensation, no aftertaste |

In the case of orbifloxacin, marbofloxacin and trovafloxacin, smaller portions were tested, due to solubility limitations. In a calibration experiment, the inhaler produced an aerosol output of 4.1 microns VMD, with a geometric standard deviation (GSD) of 1.64 micron VMD. In addition to these measurements, the inhaler produced a fine particle dose (FPD) of 54.9% (percent of emitted dose in particles less than 5 microns). The tolerability and taste of the drug during a very brief administration period and for a period of 10 minutes post administration were monitored. Tolerability parameters were of the following types: (i) cough, cough sensation, or sneezing (ii) irritation, burning or tightness of throat, (ii) irritation or runniness in nasal passages or eyes, (iii) irritation, burning or tightness of the lungs or shortness of breadth, and (iv) dizziness, headache, nausea or other systemic effects.

Marbofloxacin, sparfloxacin and tosufloxacin were too insoluble to evaluate in this test. For the remaining fluoroquinolones tested, no tolerability effects were observed during or after aerosol exposure in categories ii, iii or iv (above). Gatifloxacin, moxifloxacin ciprofloxacin, orbifloxacin and pefloxacin were all associated with cough. In the case of ciprofloxacin and orbifloxacin this may have been associated with the low-pH of the solution. Of the fluoroquinolones tested, Levofloxacin at 10 mg/ml had the best taste characteristics. Ofloxacin, lomefloxacin and pefloxacin had a more discernible taste than levofloxacin, which were also acceptable during the short course of administration.

## Summary and Conclusions From the Fluoroquinolone Taste Test

Of the thirteen flouroquinolones tested in this study, levofloxacin had preferred physical-chemical properties for aerosol administration and a demonstration of best acute tolerability of the fluoroquinolones tested (Table 13). Levofloxacin is also recognized as having one of the best antimicrobial profiles for respiratory pathogens and has the highest *in vivo* efficacy, comparable to ciprofloxacin, for treatment of Pseudomonas aeruginosa infections.

**Table 13. Overall Suitability for Nebulization (disclosed but not claimed).**

| No. | Fluoroquinolone | Assessment | Overall Score | Limitation |
|---|---|---|---|---|
| 1 | Gatifloxacin | poor solubility and pH, moderately strong bitter aerosol taste | -- | Solubility, Taste |
| 2 | Gemifloxacin | sufficient solubility and pH, strong bitter aerosol taste, strong aftertaste | --- | Taste |
| 3 | Levofloxacin | excellent solubility and pH, chemical aerosol taste, somewhat bitter | + | Taste |

(continued)

| No. | Fluoroquinolone | Assessment | Overall Score | Limitation |
|---|---|---|---|---|
| 4 | Moxifloxacin | sufficient solubility and pH, moderately strong bitter aerosol taste, strong aftertaste | -- | Taste, Pa Activity |
| 5 | Ciprofloxacin | poor solubility and pH, very strong bitter aerosol taste, coughing | --- | Solubility, Taste |
| 6 | Ofloxacin | minimally acceptable solubility and pH, bitter chemical aerosol taste | -/+ | Taste |
| 7 | Lomefloxacin | minimally acceptable solubility and pH, chemical aerosol taste, strong liquid taste | -/+ | Pa Activity |
| 8 | Marbofloxacin | very poor solubility even at low pH, unable to test | --- | Solubility |
| 9 | Orbifloxacin | very poor solubility even at low pH, strong bitter unpleasant aerosol taste, strong cough | --- | Solubility, Taste, Pa Activity |
| 10 | Pefloxacin | sufficient solubility and pH, aerosol chemical taste, strong unpleasant liquid taste | -/+ | Pa Activity |
| 11 | Sparfloxacin | very poor solubility even at low pH, unable to test | --- | Solubility, Pa Activity |
| 12 | Tosufloxacin | very poor solubility even at low pH, unable to test | --- | Solubility |
| 13 | Trovafloxacin | moderate solubility and pH, bitter aerosol taste | -- | Taste, Pa Activity |

Ofloxacin, lomefloxacin and pefloxacin exhibited lower solubility and stronger taste at 10 mg/mL than levofloxacin. Ofloxacin is 2-fold less potent that levofloxacin, and lomefloxacin and pefloxacin are 4-fold less potent. Higher concentrations of these antibiotics have the preferred potency and administration times under 15 minutes.

In a separate study, conducted in a similar manner, norfloxacin was tested and found to have a solubility, taste and potency profile very similar to gatifloxacin, with the exception of significantly less activity against the gram-positive pathogens.

**Taste Testing of Aerosol Salt Formulations of Levofloxacin and Gemifloxacin (disclosed but not claimed)**

Based on the results of the above studies, levofloxacin, and its racemate ofloxacin, as well as gemifloxacin, and to a lesser extent gatifloxacin and norfloxacin are amenable to aerosol administration for pulmonary antibacterial treatment. To further test the taste and acute tolerability (cough sensation and cough) properties of levofloxacin and gemifloxacin, several formulations were prepared with different organic and inorganic acids and tested in the manner described above. Solutions were prepared by first adding 500 mg levofloxacin to 10 ml of water or adding 500 mg of gemifloxacin to 20 ml of saline (due to solubility limitations), titrating the pH to -6.5 with HCl or organic acid, then adjusting the osmolality of levofloxacin containing solutions to -300 mOsmol/kg with sodium chloride. The formulations tested are shown in Table 14.

These formulations were tested by a total of three healthy human volunteers in the same manner as described above, at a levofloxacin concentration of 50 mg/mL, and a gemifloxacin concentration of 25 mg/mL, in a carefully controlled, head-to-head, fully blinded test. Results are shown in Table 15 and 16.

These results demonstrate that hydrochloric acid, citric acid and ascorbic acid formulations of levofloxacin have superior taste and tolerability compared to the acetic acid, lactic acid and tartaric acid formulations of levofloxacin. Furthermore, these levofloxacin formulations have superior taste and tolerability over the equivalent gemifloxacin formulations. With respect to gemifloxacin, the citric acid formulation had superior taste and tolerability compared to the HCl and ascorbic acid formulations of gemifloxacin, and with further formulation refinement, would be amenable to aerosol administration.

**Table 14. Levofloxacin and Gemifloxacin Formulations (disclosed but not claimed).**

| Fluoroquinolone | Acid | Conc (mg/mL) | pH | Osmolality (mOsm/kg) |
|---|---|---|---|---|
| Levofloxacin | HCl | 50 | 6.5 | 181 |

(continued)

| Fluoroquinolone | Acid | Conc (mg/mL) | pH | Osmolality (mOsm/kg) |
|---|---|---|---|---|
| Levofloxacin | Acetic | 50 | 6.41 | 273 |
| Levofloxacin | Citric | 50 | 6.45 | 286 |
| Levofloxacin | Lactic | 50 | 6.42 | 286 |
| Levofloxacin | Ascorbic | 50 | 6.50 | 278 |
| Levofloxacin | Tartaric | 50 | 6.35 | 286 |
| Gemifloxacin | HCl | 25 | 5.6 | 330 |
| Gemifloxacin | Citric | 25 | 5.7 | 363 |
| Gemifloxacin | Ascorbic | 25 | 5.9 | 347 |

**Table 15. Aerosol Taste and Tolerability of Levofloxacin Formulations at 50 mg/mL (disclosed but not claimed).**

| | Taster | | |
|---|---|---|---|
| **Acid** | **1** | **2** | **3** |
| HCl | Moderate bitter taste | Bitter taste, cough sensation | Bitter taste |
| Acetic Acid | Very acidic taste | Strong acidic taste, cough sensation | Acidic taste, after taste |
| Citric Acid | Mild after taste, slightly sweet | Mild taste, sweet | Mild after taste |
| Lactic Acid | Strong bitter taste, aftertaste | Mild taste, after taste, slight cough | Bitter, mild after taste |
| Ascorbic Acid | Mild taste, slight acidity | Mild taste | Little taste or after-taste |
| Tartaric Acid | Very bitter, strong after taste | Strong bitter taste, bitter after taste | Bitter taste |

**Table 16. Taste and Tolerability of Gemifloxacin Formulations at 25 mg/mL (disclosed but not claimed).**

| | Taster | | |
|---|---|---|---|
| **Acid** | **1** | **2** | **3** |
| Hydrochloric Acid | Metallic taste, strong after taste | Slight bitter taste | Bitter taste, slightly metallic |
| Citric Acid | Slightly sweet | Mild cough, slightly bitter | Very mild taste, no after taste |
| Ascorbic Acid | Mild taste | Cough, mild bitter after taste | Slightly bitter, mild after taste |

**Taste Testing of Additional Aerosol Levofloxacin Formulations**

To further test the taste and tolerability properties of additional levofloxacin excipient combinations in a systematic manner, a series of formulations were prepared and tested. The formulations are listed in Table 17. They included sugars, salts, sweeteners and other excipients prepared by mixing levofloxacin with water, adding the excipients listed in Table 17, and titrating if necessary to the desired pH with dilute HCl, Osmolality was not optimized for these studies. However, osmolality was determined using an Advanced Instruments Model 3250 Osmometer. This measurement, made on 250μL of sample, relies on freezing point depression to determine osmolality.

These formulations were tested in a total of three healthy human volunteers in a series of tests (A-G) in the

same manner as described above, in a carefully controlled, head-to-head, fully blinded manner. All tests were performed in a fully blinded fashion. Results of the tests (Tables 19-25) are described below. The following scoring system was used (Table 18).

Test A: Taste Testing of Sweeteners, Divalent Metal Salts, and Surface Active Agents. This test included sweeteners, calcium and magnesium salts, and surface active agents (i.e., glycerin and PS-80). As shown in Table 17, formulations containing the sweeteners shown are mildly bitter and have metallic taste. The artificial sweeteners appeared to produce a bitter taste that is distinct from the bitterness otherwise observed. Most significantly, the formulation containing $CaCl_2$ had the most improved taste relative to control ($MgCl_2$ was not tested in this experiment) (Table 19).

Test B: Taste Testing of Mono- and Disaccharides in the Presence of Calcium Chloride. All of the formulations screened in this experiment were well tolerated and tasted better than the control sample. Formulations containing both the calcium salt and sugar performed better than either one alone, suggesting that these compounds improve taste through different mechanisms. Of these formulations, 5% $CaCl_2$ + 7.5 % glucose performed best. Note that the lactose is present at a lower concentration than the other sugars (Table 20).

**Table 17. Levofloxacin Formulations Containing Various Excipients.**

| Fluoroquinolone | Conc (mg/mL) | Excipients | pH | Osmolality (mOsm/kg) |
|---|---|---|---|---|
| Levofloxacin | 50 | Control A (0.225% NaCl) | 6.50 | 180 |
| Levofloxacin | 50 | Aspartame (0.1 %) | 6.49 | 175 |
| Levofloxacin | 50 | Sucrulose (0.1%) | 6.49 | 178 |
| Levofloxacin | 50 | Glucose (5%) | 6.5 | 380 |
| Levofloxacin | 50 | Sucrose (7.5%); NaCl (0.225%) | 6.51 | 329 |
| Levofloxacin | 50 | Glycerin (5%) | 6.48 | 880 |
| Levofloxacin | 50 | PS-80 (0.1%) | 6.51 | 189 |
| Levofloxacin | 50 | $CaCl_2$ (5%) | 6.10 | 784 |
| Levofloxacin | 50 | $MgSO_4$ (5%) | 6.41 | 73 |
| Levofloxacin | 50 | Control - B-E (0.225% NaCl) | 6.51 | 182 |
| Levofloxacin | 50 | $CaCl_2$ (5%) | 6.1 | 735 |
| Levofloxacin | 50 | $CaCl_2$ (5%), Sucrose (7.5%) | 6.10 | 958 |
| Levofloxacin | 50 | $CaCl_2$ (5%), Glucose (7.5%) | 6.10 | 1174 |
| Levofloxacin | 50 | $CaCl_2$ (5%), Glucose (7.5%) | 5.25 | 1246 |
| Levofloxacin | 50 | $CaCl_2$ (5%), Lactose (5%) | 6.07 | 864 |
| Levofloxacin | 50 | $MgCl_2$ (5%) | 5.90 | 600 |
| Levofloxacin | 50 | $MgCl_2$ (5%), Sucrose (7.5%) | 5.98 | 815 |
| Levofloxacin | 50 | $MgCl_2$ (5%), Glucose (7.5%) | 5.98 | 999 |
| Levofloxacin | 50 | $MgCl_2$ (5%), Glucose (7.5%) | 5.04 | 1035 |
| Levofloxacin | 50 | $MgCl_2$(5%), Lactose (5%) | 5.96 | 697 |
| Levofloxacin | 50 | $MgSO_4$(5%), Sucrose (7.5%) | 6.20 | 433 |
| Levofloxacin | 50 | $MgSO_4$(5%), Glucose (7.5%) | 6.21 | 625 |
| Levofloxacin | 50 | $MgSO_4$(5%), Glucose (7.5%) | 5.40 | 660 |
| Levofloxacin | 50 | $MgSO_4$(5%), Lactose (5%) | 6.18 | 387 |
| Levofloxacin | 50 | Control F-G (0.45% NaCl) | 6.5 | 221 |
| Levofloxacin | 50 | Glucose (5%) | 6.5 | 376 |
| Levofloxacin | 50 | Sucrose (5%) | 6.5 | 240 |
| Levofloxacin | 50 | Lactose (5%) | 6.62 | 241 |

(continued)

| Fluoroquinolone | Conc (mg/mL) | Excipients | pH | Osmolality (mOsm/kg) |
|---|---|---|---|---|
| Levofloxacin | 50 | Lactose (2.5%) | 6.55 | 170 |
| Levofloxacin | 50 | $CaCl_2$ (5%) | 6.10 | 735 |
| Levofloxacin | 50 | $CaCl_2$ (5%), Lactose (5%) | 6.21 | 1037 |
| Levofloxacin | 50 | $CaCl_2$ (2.5%), Lactose (5%) | 6.36 | 565 |
| Levofloxacin | 50 | $CaCl_2$ (2.5%), Lactose (2.5%) | 6.41 | 370 |
| Levofloxacin | 50 | $CaCl_2$ (1.25%), Lactose (2.5%) | 6.64 | 227 |
| Levofloxacin | 50 | $CaCl_2$ (0.625%), Lactose (2.5%) | 6.06 | 163 |

**Table 18. Taste Test Scoring System.**

| Score | Taste | Tolerability |
|---|---|---|
| **1** | Comparable to saline | No cough sensation, no cough |
| **1.25** | Slightly more taste than saline | Slight cough sensation, no cough |
| **1.5** | Mild bitter/metallic taste | Cough sensation, slight cough |
| **1.75** | Between 1.5 and 2 | - |
| **2** | Moderate bitter/metallic taste | Cough sensation, moderate cough |
| **2.25** | Between 2 and 2.5 | - |
| **2.5** | Strong bitter/metallic taste | - |
| **2.75** | Between 2.5 and 3 | - |
| **3** | Very strong bitter/metallic taste | Cough sensation and strong cough |
| **4** | Very strong bitter/metallic taste and other unacceptable taste | Cough sensation, strong cough and other irritation |

**Table 19. Taste and Tolerability of Levofloxacin Formulations Containing Sweeteners, Divalent Metal Salts, and Surface Active Agent.**

| Excipients | Taster | | | | | | | |
|---|---|---|---|---|---|---|---|---|
| | 1 | | 2 | | 3 | | Median | |
| | Taste | Tol. | Taste | Tol. | Taste | Tol. | Taste | Tol. |
| Aspartame (0.1 %) | 2 | 1.25 | 2 | 1 | 2 | 1 | 2 | 1 |
| Sucrulose (0.1%) | 2 | 1 | 1.75 | 1 | 2 | 1 | 2 | 1 |
| Sucrose (7.5%); NaCl (0.225%) | 2 | 1 | 2.25 | 1 | 2 | 1 | 2 | 1 |
| Glucose (5%) | 1.5 | 2 | 2.5 | 1 | 2 | 1 | 2 | 1 |
| Glycerin (5%) | 2.25 | 1 | 2.25 | 1 | 2.5 | 1 | 2.3 | 1 |
| PS-80 (0.1%) | 1.75 | 1 | 2.25 | 1 | 2.5 | 1 | 2.3 | 1 |
| $CaCl_2$ (5%) | 1.25 | 1 | 1.5 | 1.5 | 2 | 1 | 1.5 | 1 |
| $MgSO_4$(5%) | 1.5 | 1.5 | 2.5 | 2.5 | 2.5 | 1 | 2.5 | 1.5 |
| Control-A (0.225% NaCl) | 3 | 1 | 3 | 1 | 2.5 | 1 | 3 | 1 |

**Table 20. Taste and Tolerability of Levofloxacin $CaCl_2$ Formulations.**

| Excipients | Taster | | | | | | | |
|---|---|---|---|---|---|---|---|---|
| | 1 | | 2 | | 3 | | Median | |
| | Taste | Tol. | Taste | Tol. | Taste | Tol. | Taste | Tol. |
| $CaCl_2$ (5%) | 1.75 | 1 | 2 | 1 | 2.75 | 1 | 2 | 1 |
| Sucrose (5%) | 2 | 1 | 2 | 1 | 2 | 1 | 2 | 1 |
| CaCl2 $(5\%)_2$, Sucrose (7.5%) | 1.75 | 1 | 1.75 | 1 | 1.5 | 1 | 1.8 | 1 |
| $CaCl_2$(5%), Glucose (7.5%) | 1.5 | 1 | 1.5 | 1 | 2 | 1 | 1.5 | 1 |
| $CaCl_2$ (5%), Lactose (5%) | 1 | 1 | 1.75 | 1 | 2 | 1 | 1.8 | 1 |
| Control B-E (0.225% NaCl) | 3 | 1 | 2.5 | 1 | 3 | 1 | 3 | 1 |

Test C: Taste Testing of Mono- and Disaccharides in the Presence of Magnesium Chloride. As above, all of the formulations screened in this experiment were well tolerated and tasted better than the control sample. Formulations containing both the magnesium salt and lactose appeared to perform slightly better than either one alone. This experiment confirms that combining divalent metal salts and simple sugars are effective at improving taste (Table 21).

Table 21. Taste and Tolerability of Levofloxacin $MgCl_2$ Formulations.

| Excipients | Taster | | | | | | | |
|---|---|---|---|---|---|---|---|---|
| | 1 | | 2 | | 3 | | Median | |
| | Taste | Tol. | Taste | Tol. | Taste | Tol. | Taste | Tol. |
| $MgCl_2$ (5%) | 1.5 | 1 | -- | - | 1.75 | 1 | 1.6 | 1 |
| $MgCl_2$ (5%), Sucrose (7.5%) | 1.5 | 1 | 1.75 | 1 | 2 | 1 | 1.8 | 1 |
| $MgCl_2$(5%), Glucose (7.5%) | 1.25 | 1 | 2.25 | 1 | 2 | 1 | 2 | 1 |
| $MgCl_2$ (5%), Lactose (5%) | 1 | 1 | 1.5 | 1 | 1.5 | 1 | 1.5 | 1 |
| Control B-E (0.225% NaCl) | 2.25 | 1 | - | - | 2.75 | 1 | 2.5 | 1 |

Test D: Taste Testing of Mono- and Disaccharides in the Presence of Magnesium Sulfate. As with calcium and magnesium chloride, formulations containing magnesium sulfate and glucose, sucrose or lactose tasted better than the control sample. This experiment reconfirms that combining divalent metal salts and simple sugars improve taste (Table 22).

**Table 22. Taste and Tolerability of Levofloxacin $MgSO_4$Formulations.**

| Excipients | Taster | | | | | | | |
|---|---|---|---|---|---|---|---|---|
| | 1 | | 2 | | 3 | | Median | |
| | Taste | Tol. | Taste | Tol. | Taste | Tol. | Taste | Tol. |
| $MgSO_4$, Sucrose | 1.5 | 2 | 1.5 | 1.25 | 1.5 | 1 | 1.5 | 1.3 |
| $MgSO_4$, Glucose | 1.5 | 2.75 | 2 | 2.5 | 1.5 | 1.5 | 1.5 | 2.5 |
| $MgSO_4$, Lactose | 1.25 | 2.25 | 1.75 | 1.25 | 1.75 | 1 | 1.8 | 1.3 |
| Control B-E (0.225% NaCl) | 2.25 | 1 | - | - | 3 | 1 | 2.6 | 1 |

Test E: Taste Testing of Divalent Metal Salts in the Presence of Glucose at Low and High pH. In this experiment, the effect of glucose in combination with each of the three divalent cation salts on taste and tolerability was tested at low (≤ 5.5) and high (≥ 6.0) pH. Small but consistent improvements in taste were observed at the higher pH (Table 23).

**Table 23. Taste and Tolerability of Levofloxacin $CaCl_2$ Formulations at Low Versus High pH.**

| Excipients | Taster | | | | | | | |
|---|---|---|---|---|---|---|---|---|
| | 1 | | 2 | | 3 | | Median | |
| | Taste | Tol. | Taste | Tol. | Taste | Tol. | Taste | Tol. |
| $CaCl_2$(5%), Glucose (7.5%), pH6.1 | 1 | 1 | 1.5 | 1 | 2 | 1 | 1.5 | 1 |
| $CaCl_2$(5%), Glucose (7.5%), pH 5.5 | 1.25 | 1 | 1.75 | 1 | 2.5 | 1 | 1.8 | 1 |
| $MgCl_2$(5%), Glucose (7.5%), pH6.0 | 1.25 | 1 | 2 | 1 | 2 | 1 | 2 | 1 |
| $MgCl_2$(5%), Glucose (7.5%), pH 5.0 | 1.75 | 1 | 1.75 | 1 | 1.5 | 1 | 1.8 | 1 |
| $MgSO_4$(5%), Glucose (7.5%), pH 6.2 | 1.25 | 2.25 | 2.25 | 1.75 | 1.5 | 1 | 1.5 | 1.8 |
| $MgSO_4$(5%), Glucose (7.5%), pH 5.4 | 1.5 | 1.75 | 1.75 | 1.5 | 2 | 1 | 1.8 | 1.5 |
| Control B-E (0.225% NaCl) | 2 | 1 | - | - | - | - | - | - |

Test F. Taste Testing of Mono- and Disaccharides. All of the formulations screened in this experiment were well tolerated and tasted better than the control sample. All three sugars at 5% were better than the control, lactose at 2.5% tasted better than the control, but not as good as at 5%. This experiment reconfirms that simple sugars improve taste (Table 24).

**Table 24. Taste and Tolerability of Levofloxacin Sugar Formulations (disclosed but not claimed).**

| Excipients | Taster | | | | | |
|---|---|---|---|---|---|---|
| | 1 | | 3 | | Median | |
| | Taste | Tol. | Taste | Tol. | Taste | Tol. |
| Glucose (5%) | 1.5 | 1.5 | 2 | 1 | 1.8 | 1.3 |
| Sucrose (5%) | 1.5 | 1.5 | 1.5 | 1 | 1.5 | 1.3 |
| Lactose (5%) | 1.75 | 1.25 | 2 | 1 | 1.9 | 1.1 |
| Lactose (2.5%) | 2.25 | 1.5 | 2 | 1 | 2.1 | 1.3 |
| Control F-G (0.45%NaCl) | 2.5 | 1 | 2.5 | 1 | 2.5 | 1 |

Test G. Taste and Tolerability of Levofloxacin $CaCl_2$ Formulations In the Presence of Lactose. In this experiment, levofloxacin was formulated with varying concentrations of calcium chloride and lactose (Table 25). As noted through this series of experiments, all formulations containing divalent metal salts and sugar were improved with respect to taste and tolerability relative to the control formulation. Most importantly, 5% calcium chloride or 2.5% calcium chloride in the presence of 5% lactose were most effective at decreasing levofloxacin bitterness. Further decreases in the concentration of these excipients were less effective.

**Table 25. Taste and Tolerability of Levofloxacin $CaCl_2$ Formulations in the Presence of Lactose.**

| Excipients | Taster | | | | | |
|---|---|---|---|---|---|---|
| | 1 | | 3 | | Median | |
| | Taste | Tol. | Taste | Tol. | Taste | Tol. |
| $CaCl_2$ (5%) | 1.25 | 1 | 1.5 | 1 | 1.4 | 1 |
| $CaCl_2$ (5%), Lactose (5%) | 1.25 | 1 | 2 | 1 | 1.6 | 1 |
| $CaCl_2$ (2.5%), Lactose (5%) | 1.25 | 1 | 2 | 1 | 1.6 | 1 |
| $CaCl_2$ (2.5%), Lactose (2.5%) | 1.5 | 1 | 2.5 | 1 | 2 | 1 |

(continued)

| Excipients | Taster | | | | | |
|---|---|---|---|---|---|---|
| | 1 | | 3 | | Median | |
| | Taste | Tol. | Taste | Tol. | Taste | Tol. |
| $CaCl_2$ (1.25%), Lactose (2.5%) | 1.75 | 1 | 2 | 1 | 1.9 | 1 |
| $CaCl_2$ (0.625%), Lactose (2.5%) | 1.75 | 1.25 | 2 | 1 | 1.9 | 1.1 |
| Control F-G (0.45%NaCl) | 3 | 1 | 2.5 | 1 | 2.8 | 1 |

**Reference Example 3 - Aerosol Levofloxacin Characterization in PARI LC Plus Jet Nebulizer.**

The following studies describe the potential for aerosolized delivery of levofloxacin to be administered to a patient via a jet nebulizer. To accomplish this task, a simple levofloxacin formulation was prepared and the aerosol was characterized in a jet nebulizer. The results of these studies are shown in the summary below.

Levofloxacin inhalation solution (55 mg/ml) was evaluated using a PARI LC Plus Air Jet Nebulizer with ProNeb Compressor. The emitted dose, particle size distribution and fine particle fraction were measured by cascade impaction using a Marple Miller Impactor. The above-mentioned parameters were used for evaluating the *in vitro* performance of aerosolized medications.

**Marple Miller Study**

Objective. To determine the particle size distribution and estimate the amount of drug that a patient is likely to inhale (respirable fraction). A secondary objective was to estimate emitted dose, which is the amount of levofloxacin that exited the nebulizer.

Methods. Formulation: 55 mg/ml levofloxacin, 120 mM chloride, 70 mM sodium, pH 6.7. Formulation established from maximum solubility permitting a 300 mg dosage in 6 ml and neutral pH. 5.5 ml of levofloxacin formulation was added to a PARI LC Plus Air-Jet Nebulizer with ProNeb Compressor. The nebulizer cup contained a total of 302 mg of levofloxacin. The nebulizer was connected inline with a Marple Miller Impactor (MMI), which was run with an airflow rate of 60 l/min. Each nebulizer (n=2) was run to dryness (no aerosol produced as judged by visual inspection for 15 minutes. Following aerosolization, the MMI was disassembled and levofloxacin was quantitatively extracted with mobile phase (90/10 ACN:water) from the USP entry port, each of the impactor collection cups (stages) and the glass fiber filter. Any remaining formulation in the nebulizer after aerosolization (cup and mouthpiece) was also quantified.

**Results**

As shown in Table 26, the total average amount recovered from the MMI experiments was 170.2 mg. The expected recovery was 302 mg. This represents a total recovery of -57%, which does not meet the generally accepted specifications for impaction-based studies (85%-115% total recovery). This difference was found to be due to non-specific adherence of levofloxacin to the LC Plus nebulizer device. The average percent of drug exiting the nebulizer in fine particles was -72%. Thus, the respirable emitted dose was 89.7 mg. Assuming that -50% is not inhaled during normal tidal breathing, a total of -40 mg may be deposited in the lung with this 300 mg dose. However, given the slow administration time with this device, competition with pulmonary clearance would likely prevent the accumulation of sufficient levofloxacin to meet the required minimal concentration for "rapid administration, high concentration" dosing needed for maximum fluoroquinolone antimicrobial activity and resistance prevention.

**Table 26. Marple Miller Impactor Data Set.**

| Sample ID | A Emitted Dose (mg) | B Amount of Drug Remaining in Nebulizer Cup (mg) | A+B Amount of Drug Recovered (mg) | Percent of Total Drug Exiting Nebulizer in Fine Particle Fraction (% < 5um) |
|---|---|---|---|---|
| Levo Run 1 | 134.70 | 45.00 | 179.70 | 73.5 |
| Levo Run 2 | 114.40 | 46.30 | 160.70 | 70.4 |

(continued)

| Sample ID | A Emitted Dose (mg) | B Amount of Drug Remaining in Nebulizer Cup (mg) | A+B Amount of Drug Recovered (mg) | Percent of Total Drug Exiting Nebulizer in Fine Particle Fraction (% < 5um) |
|---|---|---|---|---|
| Average | 124.6 | 45.7 | 170.2 | 72.0 |

**Reference Example 4 - Animal Models and Evaluation of Fluoroquinolones and Fuoroquinolone Formulations.**

**Pharmacokinetic Model**

Six rats per study are given a single slow bolus intravenous dose of 10 mg/kg via the lateral tail vein or are given a single microspray aerosol dose of 10 mg/kg using a microspray aerosol generation device (PennCentury, Philadelphia, PA). Blood samples are taken at various times over 3 hours to determine the plasma pharmacokinetic parameters. Two rats are sacrificed at 0.5, 1.5 and 3 hours after dosing to determine lung, broncheoalveolar lavage (BAL), and epithelial lining fluid (ELF) levels. The plasma and tissue concentrations are determined by an HPLC method and the data are then fit using WinNonlin. Data are shown in Table 27.

**Efficacy Model**

*P. aeruginosa* strain PAM 1723 is grown in Mueller-Hinton Broth (MHB) at 35°C under constant aeration, after 16 hours, the inoculum is sub-cultured into fresh MHB and allowed to regrow at 35°C, under constant aeration, for 4 hours. The inoculum is adjusted to ca. 5 x $10^6$ CFU/ml by correlation of absorbance at 600 nm with predetermined plate counts. Male CFW mice (4 - 6 weeks old, N= 4/group) are made neutropenic by the intraperitoneal injection of 150 mg/kg cyclophosphamide (Cytoxan, Mead Johnson, Princeton, NJ) on days 1 and 4. On day 5, mice are infected by an intratracheal instillation of 0.05 ml of inoculum while under isoflurane anesthesia (5% isoflurane in oxygen running at 4 L/min). Two hours after infection, mice are given either intraperitoneal or intratracheal doses of each fluoroquinolone at a dose of 25 mg/kg. Mice are sacrificed 1 and 4 hours after treatment, their lungs removed, homogenized and plated to determine colony counts. Data are shown in Table 28.

**Table 27. Pharmacokinetic Modeling.**

| Drug | Route | Dose (mg/kg) | Serum AUC (0-inf) | Serum t1/2 | ELF AUC (0.5-3h) | *F*, % from Lung vs. IV |
|---|---|---|---|---|---|---|
| Levofloxacin | IV | 10 | 3.8 | 0.5 | 10.5 | NA |
| Levofloxacin | IT | 10 | 3.28 | 0.4 | 12.07 | 86% |
| | | | | | | |
| Ciprofloxacin | IV | 10 | 2.56 | 0.53 | ND | NA |
| Ciprofloxacin | IT | 3.3 | 0.8 | 0.93 | 194 | 82% |
| | | | | | | |
| Clinafloxacin | IT | 10 | 3.2 | 0.74 | 30.8 | |
| | | | | | | |
| Gatifloxacin | IV | 10 | 5.31 | 1.06 | 5.32 | |
| Gatifloxacin | IT | 10 | 5.83 | 1.13 | 54.7 | 100% |
| | | | | | | |
| Norfloxacin | IV | 10 | 4.65 | 1.21 | 3.27 | |
| Norfloxacin | IT | 10 | 4.46 | 1.13 | 41.7 | 100% |
| | | | | | | |
| Gemifloxacin | IV | 8 | 4.54 | 1.04 | 3.72 | |
| Gemifloxacin | IT | 10 | 5.86 | 1.68 | 536.5 | 86% |
| | | | | | | |

(continued)

| Drug | Route | Dose (mg/kg) | Serum AUC (0-inf) | Serum t1/2 | ELF AUC (0.5-3h) | *F*, % from Lung vs. IV |
|---|---|---|---|---|---|---|
| Tobramycin | IV | 10 | 15.7 | 0.5 | 27.6 | NA |
| Tobramycin | IT | 10 | 13.82 | 1.0 | 5152.0 | 81% |

In rat pharmacokinetic studies, aerosol administration of fluoroquinolones results in increased ELF AUCs from 0.5 - 3 hours for all fluoroquinolones tested, as well as tobramycin, suggesting that the aerosol route of administration will produce increased efficacy against lung infections.

In a mouse lung infection model, the increased efficacy, suggested by the pharmacokinetic studies rats, was confirmed. For all fluoroquinolones tested, the aerosol route of administration (intratracheal, or IT), produced larger reductions in bacterial counts than the intraperitoneal (IP) route of administration, suggesting that observed increased efficacy was due to high local concentrations produced by direct aerosol administration.

**Table 28. Efficacy Modeling.**

| Drug | Route[a] | Dose (mg/kg) | DeltaLOG CFU 1 hr[b] | DeltaLOG CFU 4 hr[b] |
|---|---|---|---|---|
| Levofloxacin | IP | 25 | -1.00 | -0.52 |
| Levofloxacin | IT | 25 | -1.97 | -1.28 |
| Gemifloxacin | IP | 25 | -0.28 | -0.32 |
| Gemifloxacin | IT | 25 | -2.45 | -1.81 |
| Levofloxacin | IP | 25 | -1.40 | -1.14 |
| Levofloxacin | IT | 25 | -2.48 | -1.45 |
| Gemifloxacin | IP | 25 | -0.74 | -0.71 |
| Gemifloxacin | IT | 25 | -3.20 | -2.28 |
| Clinafloxacin | IP | 25 | -1.32 | -1.33 |
| Clinafloxacin | IT | 25 | -2.86 | -2.47 |
| Tobramycin | IP | 5 | -0.70 | 0.29 |
| Tobramycin | IT | 5 | -1.59 | -0.94 |
| Ciprofloxacin | IP | 25 | -1.59 | -0.41 |
| Ciprofloxacin | IT | 25 | -2.32 | -1.45 |
| Gatifloxacin | IP | 25 | -0.34 | -0.02 |
| Gatifloxacin | IT | 25 | -1.48 | -2.11 |
| Clinafloxacin | IP | 10 | -0.96 | -1.39 |
| Clinafloxacin | IT | 10 | -2.71 | -2.40 |
| Sparfloxacin | IP | 25 | -0.85 | 0.09 |
| Sparfloxacin | IT | 25 | -1.56 | -0.81 |
| Tosufloxacin | IP | 25 | 0.00 | 1.33 |
| Tosufloxacin | IT | 25 | -0.48 | -0.24 |

a. Route of drug administration.
b. Time post-drug administration.

**Reference Example 5-Aerosol Levofloxacin Characterization in the PARI eFlow Nebulizer.**

**Laser Particle Sizing**

Device performance was characterized by measuring the size of the particles emitted. By non-limiting example, particle sizing of emitted aerosol of Levofloxacin solution may be conducted with a Malvern Spraytec particle sizer under the following conditions. Ambient conditions are controlled to maintain a room temperature of between 23.0°C and 24.0°C and relative humidity of 42% to 45%. Levofloxacin at 25 mg/ml was loaded into 2 PARI eFlow Nebulizers fitted with the "40" nebulizing heads. Software for the Malvern Spraytec particle sizer is programmed to calculate the following information. A) Volume Mean Diameter (VMD), the volume mean of the particles passing across the beam of the laser. B) Geometric Standard Deviation (GSD), diameter $84^{th}$ percentile/diameter $50^{th}$ percentile. C) % of particles ≤ 5 microns, the percent of the number of particles less than 5 microns or % of particle >1 micron and < 7 microns, the percent of the number of particles between 1 and 7 microns.

The device was loaded with 2 ml Levofloxacin at 25 mg/ml. The mouthpiece of the device was positioned with the tip of the mouthpiece 2 cm from the center of the beam on the x axis and as close to the optical lens of the laser as possible on the y axis. Ambient conditioned, bias flow was provided through the nebulizer in an amount to obtain a total nebulizer flow of 20 LPM. Ambient conditioned, bias flow was provided through the nebulizer in an amount to obtain a total nebulizer flow of 20 LPM. The nebulizer was turned on and allowed to run continuously for 1 minute prior to measuring. The measurement sequence was begun after 1 minute and measurements are made continuously for 1 minute in 1 second intervals. At the end of the measurement phase, these 60 records are averaged for VMD, GSD and % ≤5 micron and % >1 and <7 micron. Finally, the nebulizer was weighed for determination of output rate.

**Breath Simulation Studies**

Device performance was measured under conditions similar to natural inhalation by using a breath simulator PARI Compas breath Simulator programmed to use the European Standard pattern of 15 breaths per minute with an inspiration to expiration ratio of 1:1. Such measurements was performed under ambient conditions that can be controlled to maintain a room temperature of between 23.0°C and 24.0°C and relative humidity of 42% to 45%. For this experiment, the PARI eFlow device was loaded with 4 ml Levofloxacin solution at 25 mg/ml.

Breathing simulation was commenced, and the nebulizers begun. The devices were allowed to run continuously until nebulization ceases. The duration is timed from the beginning of nebulization. Following nebulization, the inspiratory and expiratory filters were individually washed in a known amount of solvent ($dH_2O$). The nebulizer cup is also washed individually. For quantitation, the individual washings were assayed via spectrophotometry at a wavelength of 290 nanometers and the resultant concentration converted to content. Using this quantitative data, the following analysis was made. A) Inspired dose (ID), the total amount of drug assayed from the inspiratory filter. B) Residual dose (RD), the amount of drug assayed from the nebulizer at the end of nebulization. C) Fine Particle Dose (FPD), the ID multiplied by the respirable fraction (for example, % particles ≤ 5 microns VMD depending on the method used to determine the size of the particles emitted from the selected device). D) Duration, time from the beginning to the end of nebulization. E) Respirable Delivered Dose (RDD), % ID that is, for example, ≤ 5 microns VMD.

The results in Table 29 indicate that a 100 mg dose of levofloxacin likely deposits -34 mg fluoroquinolone in the pulmonary compartment in -4 min using the PARI eFlow device (Table 29) compared to the 300 mg dose from the PAR LC Plus device delivering an equivalent dose in >15 min. From the "rapid administration, high concentration" dosing and delivery model described herein, while the 15 min delivery time from the LC Plus will likely fail, a 4 min administration time fo 35-40 mg levofloxacin may meet the criteria for maximum fluoroquinolone activity. However, increasing the drug concentration to enable more rapid administration (e.g. 50 mg/ml in a 2 ml dosing delivering 35-40 mg levofloxacin in ~2 min) will more likely meet these minimal requirements. Moreover, shorter administration times will improve patient dosing compliance. In addition, it should be noted that hypotonic solutions of levofloxacin at concentrations greatere than 10 mg/ml are poorly tolerated for inhalation.

**Table 29. Levofloxacin Aerosol Properties (100 mg Loading Dose).**

| | | | FPD (%) | | RDD (mg) | | VMD | GSD | Osmo |
|---|---|---|---|---|---|---|---|---|---|
| Duration (minutes) | Residual Dose | Inspired Dose | ≤ 5u | 1-7u | ≤ 5u | 1-7u | um | um | mOs/kg |
| 3.9 ± 0.1 | 24.8 ± 3.4 | 61.1 ± 1.6 | 54.9 | 73.8 | 33.5 | 45.1 | 4.7 | 1.6 | 67 ± 1.0 |

**Reference Example** 6 - **Tolerability of Aerosol Levofloxacin in a Healthy Human Subject. Methods**

In a single subject, healthy volunteer the feasibility of delivering levofloxacin as an aerosol was established using either an Aerogen Clinical vibrating mesh device creating a 3.4 micron volumetric mean diameter (VMD) particle, or ~2 micron MMAD (hereinafter "Aerogen Small"), or using a PARI eFlow nebulizer producing a -4.7 micron VMD particle (hereinafter "PARI Large"). Levofloxacin was tested at a concentration of 4.25 mg/mL or 18.75 mg/mL at doses of 10 mg, 35 mg and 55 mg, in isotonic solution.

**Results**

In the first test, 6 mL of the 4.25 mg/mL solution was inhaled using the Aerogen Small nebulizer. The estimated RDD based on separate in vitro device characterization studies using breath simulation was estimated to be 10 mg. Delivery time was 22 minutes. No discernable adverse effects were observed in the throat, airway or lungs, during or after administration, including cough sensation or cough, and there was only a slight chemical taste during and after administration. No adverse effects or taste were observed over a 30 minute monitoring period following drug administration. At this low concentration and dose, and slow rate of administration, levofloxacin was well tolerated.

In the second test, 4 ml of the 18.75 mg/mL solution was inhaled using the Aerogen Small nebulizer. The estimated RDD based on separate in vitro device characterization studies using a breath simulator was 35 mg. Delivery time for administration of the drug was 14 minutes. Despite the increased dose, the acute tolerability was very comparable to the first test both during and after administration. The taste, which was stronger, was the solution had a more the bitter/metallic chemical taste characteristic of levofloxacin. The taste was most discernible for a period of a few mintues after the end of administration, again a characteristic of levofloxacin.

In the third test, 4 mL of the 18.75 mg/mL solution was inhaled using the PARI Large device. The estimated RDD based on separate in vitro device characterization studies was -55 mg (using the <5 microns FPD definition). Delivery time for administration of the drug was -5 minutes. Despite the significantly increased particle size and delivery rate for drug compared to test 2, no adverse effects in the throat, airway or lungs, other than the acute effects of taste noted above, were experienced, including cough sensation or coughing, throughout the dosing period and for a 30 minute observation period following delivery of the dose. Urinary recovery of the drug, which is an accurate measure of exposure, confirms that the projected respirable dose of approximately 55 mg was successfully delivered.

These results demonstrate the feasibility of aerosol delivery of levofloxacin in a human subject at the intermediate concentrations tested, and suggest that higher concentrations and doses, properly formulated for tolerability and taste are achievable.

Reference Example 8 - Preformulation of Levofloxacin Base.

The goal of this study was to characterize levofloxacin base to understand the physico-chemical capabilities and restrictions of levofloxacin base for various formulation approaches. The purpose of this study was to characterize the physicochemical properties of levofloxacin base.

**Preformulation**

***pH-Solubility Studies***

The solubility of levofloxacin was determined as a function of pH. Buffers were first prepared in the pH range 2-10. Small aliquots of each buffer (~200-250 μL) were saturated with drug and agitated to achieve equilibrium solubility. The samples were then becentrifuged and the supernatant analyzed for dissolved drug by UV or HPLC. The buffers used in this study were shown to affect the solubility result (because different buffer counter-ions can form different levo salt forms in solution). Hence, pH-solubility will also be assessed in the absence of buffers (via titration).

***pKa Determination***

The pKa of levofloxacin was determined by titrimetry. Obtained pKa values were confirmed by UV spectrophotometry. This information was used to aid in salt selection for levofloxacin and to determine the charge on levofloxacin under the pH conditions in the lung.

***Preformulation for Liquid System***

The feasibility of a liquid formulation was investigated using (a) solubility and (b) surface tension as baseline

parameters for formulation in saline alone.

**Preformulation Studies on Levofloxacin**

***HPLC Method Transfer***

***Experimental Methodology***

A HPLC method was used to evaluate the linearity, accuracy and precision of the levofloxacin assay. The column used was a 50 X 4.6 mm, Onyx Monolithic C18 (Phenomenex) at 30°C. The mobile phase consisted of 85% of 0.1% TFA in water and 15 % 0.1% TFA acetonitrile. The flow rate was adjusted to 3 ml/min. Samples were injected into the chromatographic system and the effluent monitored at 277 nm.

***Results***

The retention time for levofloxacin was approximately 0.82 min. The assay was found to be linear over a range of 5-15 μg/ml, with a correlation coefficient of 1.000. RSD (relative standard deviation) was less than 0.5 % and accuracy was within 98-102%.

**pH- Solubility Studies**

***By Titration***

***Experimental Methodology***

A saturated solution of levofloxacin in 0.1 N HCl was titrated with NaOH. After each addition of the base, the solution was shaken by vortexing. An aliquot of the sample solution was removed, centrifuged and the supernatant analyzed by UV spectroscopy at 288 nm. The same solution was back titrated with HCl.

***Results***

The pH-solubility profile of levofloxacin is shown in Figure 12. By titrimetery levofloxacin exhibited a solubility of 25.4 mg/ml at pH 7.3. However contrary to the results of the shaking experiments, the solubility by titrimetry decreased below pH 6.5 which can be attributed to the common ion effect. Since a solution of levofloxacin was prepared in HCl, a hydrochloride salt of levofloxacin would have formed in solution. Further addition of chloride ions in the form of hydrochloric acid would suppress the solubility of the hydrochloride salt.

**pKa determination**

***By Titrimetery***

***Experimental Methodology***

A solution of levofloxacin (18 mg/g) was prepared in water (18.45 mg/g). The initial pH of the solution was 7.36. This solution was titrated with 1 N HCl. Measured aliquots of HCl were added and the pH recorded after each addition. Titration was continued till a pH of 1.

In order to determine the acidic pKa, a solution of levofloxacin (18.38 mg/g) was prepared in 0.1 N HCl. The initial pH of the solution was 1.32. The solution was titrated with I N NaOH. Titration was continued till a pH of 6.55.

***Results***

Figure 13 shows a plot of pH Vs volume of titrant added for the titration of levofloxacin with HCL. This data was fit into the following equation:

$$V_t\,[OH^-] = K_b.\ V_{ep} - K_b.V_t$$

where,

$V_t$ = Volume of titrant added
$V_{ep}$= volume of titrant added till the equivalance point
$[0H^-]$ = hydroxide ion concentration = $Kw/[H^+]$
$[H^+]$ = hydronium ion concentration = $10^{-pH}$

A plot of $V_t$ $[OH^-]$ Vs $V_t$ gave a straight line (Figure 14). Data shown is from the pre-equivalence point region. From the slope we get

$$\text{Slope: } K_b = 2.09 \times 10^{-8}$$

$$pK_b = -\log K_b = 7.7$$

$$pK_a = 14 - pK_b = 6.3$$

Figure 15 shows a plot of pH Vs volume of titrant added for the titration of levofloxacin with NaOH. Acidic pKa was difficult to calculate because it was quite low (< 2.0). However, a rough approximation of pKa can be made as the pH at half the equivalence point. From the plot dpH/dV vs volume of titrant (Vt) (Figure 16), the equivalence point is at 250 μl. The pH at half the equivalence point (i.e. when $V_t$ = 125 μl) is 1.6. So the acidic pKa ~ 1.6.

***By UV spectroscopy***

***Experimental Methodology***

Dilute solutions of levofloxacin (0.013 mg/ml) were prepared in several buffers. The buffers used were HCL (pH 1,2), acetate (pH 4,5), phosphate (pH 6,7,8) and borate (9,10). The levofloxacin solutions were analyzed by UV spectroscopy at 257 nm.

***Results***

A plot of pH Vs Absorbance of the levofloxacin solution at 257 nm is shown as Figure 17. This data was fitted into a modified Henderson Hasselbach equation:

$$Abs_{observed} = Abs_{HA} \frac{[H^+]}{Ka + [H^+]} + Abs_{A-} \frac{[H^+]}{Ka + [H^+]}$$

where,

Abs observed = Absorbance of levofloxacin solution
$Abs_{HA}$ = Absorbance of levofloxacin solution pH 1.2 ;
$Abs_{A-}$ = Absorbance of levofloxacin solution at pH 7.8;
$[H^+]$ = hydronium ion concentration = $10^{-pH}$
The fitted equation provided an estimate of pKa = 5.91.

**Example 11 - Levofloxacin Metal Ion Complexes.**

The goal of this study was to prepare levofloxacin of various chelate salt forms to obtain gain taste-masking properties, AUC shape-enhancing properties through changes in solubility, dissolution and/or bioavailability. These benefits may enhance the pharmacodynamic properties of levofloxacin following pulmonary administration using nanoparticle suspension, dry powder inhalation or simple liquid formulations. These formulations may be optimized to create AUC shape-enhancing formulations of levofloxacin from altered solubility, or slow-release or low bioavailability chelates. These properties may also be incorporated into other fluoroquinolone antibiotics including, without limitation gemifloxacin, gatifloxacin, norfloxacin, tosufloxacin, sitafloxacin sarafloxacin, prulifloxacin, and pazufloxacin. Studies are also underway to characterize various and chelate forms of gemifloxacin for taste masking, AUC shape-enhancement, nanoparticle suspension and dry powder inhalation administration.

**Preparation of Levofloxacin-Metal Ion Complexes**

***Preliminary Studies***

A mixture of levofloxacin and a salt of a given cation was solubilized in deionized water and titrated with sodium hydroxide. The titration curve was compared against one obtained for levofloxacin alone to assess formation of levofloxacin-metal complex as described in Physical Pharmacy (4th Edition) by Alfred Martin (pp 261-263). Salts of various metal cations (e.g. Ca2+, Mg2+, etc) were then evaluated to identify suitable candidate(s) for subsequent evaluations. Different molar ratios of cations and levofloxacin were also evaluated.

***Preparation of Complexes***

Levofloxacin solutions were titrated against aqueous solutions of selected metal salts. Titrations were carried out at a constant pH. Formation of complexes were monitored by different methods including titrimetry, spectrofluorometry, solubility, etc. as applicable. The end point of the complexation reaction depended on the method adopted.

**Characterization of Levofloxacin Complexes**

Levofloxacin-metal cation complexes were characterized for stoichiometry, formation constants and dissociation kinetics using appropriate methodology.

***Goals***

To formulate and characterize levofloxacin complexes with metal cations (di- and tri-valent).

***Assessment of Complexation***

Preliminary investigations suggested that levofloxacin forms soluble complexes with metal cations. As a result, evaluation of the complexation process by precipitation was not possible. Other approaches that were attempted are described below.

***Titrimetry***

This approach was based on the assumption that the carboxylic acid moiety of levofloxacin is involved in complex formation with a given metal cation and that complexation results in the release of a proton from levofloxacin. The concentration of released protons would thus be proportional to the extent of complexation (depending on the binding constant) and the stoichiometry of the complex (Physical Pharmacy: 4th Edition by Alfred Martin; pp-261-263).

***Experimental Methodology***

About 0.35 mmoles of levofloxacin (in 16 mL of deionized water) were titrated with 6N NaOH in the presence and absence of salt of a metal cation (equimolar). Levofloxacin solutions were acidified to pH values less than 2.0 with 6N HCl prior to titration with NaOH. Salts of metal cations used include calcium chloride, magnesium chloride, ferrous chloride, zinc chloride, aluminum sulfate and aluminum chloride.

***Results***

As shown in Figure 18, titrations performed in the presence of metal cations resulted in a positive shift of the titration curves as compared to the one obtained with levofloxacin alone suggesting that additional NaOH (titrant) is required to obtain a specific pH of the solution in the presence of metal cation. The magnitude of the shift in titration curve at any point would represent moles of proton released due to complexation and hence moles of complexed levofloxacin.

Extent of complexation (binding and/or stoichiometry) appears to increase in the order $Ca^{+} < Mg^{2+} < Zn^{2+} = Fe^{2+} < Al^{3+}$, which is in reasonable agreement with existing literature.

Note: It was noted from the literature that aluminum chloride and aluminum sulfate have acid-like properties and would lower the pH of aqueous solutions. Consequently, the titration curves obtained with $AlCl_3$ and $Al_2(SO_4)_3$ may not provide conclusive information on complexation with levofloxacin.

***Dual Titration***

In this approach levofloxacin solution was titrated with a solution of a given metal cation to observe a drop in pH presumably due to release of protons through complexation. This was followed by addition of NaOH to revert back to the initial pH of the levofloxacin solution (prior to addition of solution of cation). This enables determination of the fraction of levofloxacin in the complexed form at a given pH.

***Experimental Methodology***

About 1.55-1.72 mmoles of levofloxacin were solubilized in deionized water and the resulting solution was acidified with 6N HCl to the desired initial pH. This acidified levofloxacin solution was titrated with a known volume of concentrated solution of a given metal cation ($Ca^{2+}$, $Mg^{2+}$, $Fe^{2+}$ and $Zn^{2+}$). The change in pH was neutralized (to the initial pH) by the addition of 6N NaOH and volume of NaOH solution added was recorded. Addition of solution of metal cation followed by neutralization with NaOH was continued until further addition of solution of metal cation failed to result in pH change of the levofloxacin solution, which would indicate endpoint of complexation. The cumulative amounts of metal cation added were plotted against cumulative amounts of NaOH required to neutralize the change in pH (Figures 19-22).

***Results***

From Figures 19-22, the plateau regions were extrapolated to obtain total amount of NaOH required to neutralize the change in pH due to complexation. These values also represent the amounts of levofloxacin in the complexed form (assuming that complexation of levofloxacin results in an equimolar release of protons). Amounts of levofloxacin in the complexed form with $Ca^{2+}$, $Mg^{2+}$, $Fe^{2+}$ and $Zn^{2+}$ are 0.8, 1.0, 1.3 and 1.1 mmoles, respectively. These represent 46.5, 64.5, 77.8 and 64.5% complexation for $Ca^{2+}$, $Mg^{2+}$, $Fe^{2+}$ and $Zn^{2+}$, respectively. It should be noted that % complexation would depend on the total concentrations of levofloxacin.

The binding constants as well as the stoichiometry of complexation for the levofloxacin complexes with the metal cations were determined as follows:

$$M + nA \underset{K_b}{\Longleftrightarrow} MA_n$$

Where M, A and $MA_n$ represent the metal cation, levofloxacin and the complex, respectively. $K_b$ would be the equilibrium binding constant. The above reaction assumes that 'n' moles of levofloxacin react with one mole of metal to yield one mole of complex.

$$K_b = [MA_n]/\{[M][A]^n\} \text{ (units } M^{-n}) \text{ ---------------------------------- Eq.1}$$

[$MA_n$] is the concentration of complex formed

[M] and [A] are the concentrations of the unbound metal and unbound levofloxacin, respectively.

Rearranging Eq.1,

$$[MA_n]/[A]^n = K_b{*}[M] \text{ ------------------------------------------------- Eq.2}$$

$$[A] = [A]_{Total} - [A]_{bound} = [A]_{Total} - [NaOH]_{used}$$

$$[M] = [M]_{Total} - [M]_{bound} = [M]_{Total} - [NaOH]_{used}/n$$

$$[MA_n] = [A]_{bound}/n = [NaOH]_{used}/n$$

Note: $[NaOH]_{used}$ is the concentration of sodium hydroxide used at any given point to neutralize the change in

pH caused by the addition of metal cation (presumably due to complexation).

Eq.2 can be modified to obtain,

$$[A]_{bound}/[A]^n = nK_b*[M] \text{ ---------------------------------------------- Eq.3}$$

It is inferred from Eq.3 that a plot of [M] *versus* $[A]_{bound}/[A]^n$ would result in a straight line with a slope of $nK_b$ when,

n = 1, for a 1:1 complex
n = 2, for a 2:1 complex
n = 3, for a 3:1 complex etc.

Shown below in Figures 23-26 are these plots for $Ca^{2+}$, $Mg^{2+}$, $Fe^{2+}$ and $Zn^{2+}$, respectively.

As shown in Figures 23-26, for each of the cations evaluated a plot of [A]bound/[A]n *versus* $nK_b*[M]$ was linear when n=2 (for $Ca^{2+}$ n=2 resulted in a better fit than n=1). These results suggest that levofloxacin complexes with $Ca^{2+}$, $Mg^{2+}$, $Fe^{2+}$ and $Zn^{2+}$ are formed with a stoichiometry of 2 moles of drug per mole of cation (2:1).

Using n=2, the binding constants for the above complexes can be determined from the slopes of the respective linear plots.

The binding constants for 2:1 complexes represented as log ($K_b$) are as follows: $Ca^{2+}$= 2.75, $Mg^{2+}$= 3.69, $Zn^{2+}$= 4.44, $Fe^{2+}$= 4.54.

***Solubility***

This method allows for a relatively simple way of determining the stoichiometry of complexation. The approach involved evaluation of solubility of the drug (levofloxacin) in the presence of increasing concentrations of complexation agent (a given metal cation). The total solubility of the drug (complexed + uncomplexed) was expected to increase linearly owing to complexation and to reach a plateau corresponding to the saturation solubility of both the drug and the complex. Determination of the stoichiometry from such a solubility curve was explained in detail elsewhere (Physical Pharmacy: 4th Edition by Alfred Martin; pp 265).

***Experimental Methodology***

Excess quantities of levofloxacin (amounts were recorded) were agitated, in the presence of increasing concentrations of $MgCl_2$, with 25 mM MES buffer (pH 5.99) using a vortex mixer. The samples were then filtered and the filtrate was diluted appropriately and analyzed spectrophotometrically to determine levofloxacin concentrations (Figure 27).

***Results***

As shown in Figure 27, the solubility of levofloxacin did increase with increasing $MgCl_2$ concentrations. However, beyond the plateau solubility (~ 650mM levofloxacin), further increase in solubility was observed, which is not consistent with the expected profile. This was attributed to the effect of ionic strength on levofloxacin solubility. It is important to note that the final pH of all the solutions were constant, albeit greater than 5.99 (final pH -7.0).

Subsequently, the experiment was repeated at a constant ionic strength of ~1.0M (adjusted with NaCl) and with 0.5M MES buffer (pH 5.99) to enhance the buffer capacity of the solution (Figure 28).

***Spectrofluorometry***

This approach was adopted to evaluate levofloxacin complexation based on existing literature evidence that the complexation process is associated with a change in the fluoroquinolone fluorescence properties. By monitoring the change in fluorescence emission of levofloxacin in the presence of different concentrations of a given metal cation it was possible to determine the binding constant of complexation as well as the stoichiometry.

***Experimental Methodology***

The fluorescence emission of levofloxacin was evaluated at excitation and emission wavelengths of 298 nm and 498 nm, respectively. Studies were conducted at two different pH values i.e. 5.0 (acetate) and 9.0 (histidine). A series of solutions containing a constant levofloxacin concentration but increasing concentrations of a given cation were

analyzed for fluorescence emission due to levofloxacin. Metal salts studied included $CaCl_2$, $MgCl_2$, $FeCl_2$, $ZnCl_2$ and $Al_2(SO_4)_3$.

***Results***

As shown in Table 30, significant data were obtained only for $Fe^{2+}$ and $Zn^{2+}$. For the remaining cations, the relative concentrations of levofloxacin and the cation need to be further optimized to observe a specific trend in change in levofloxacin fluorescence.

The influence of increasing concentrations of Fe2+ and Zn2+ on levofloxacin fluorescence emission are shown in Figures 29 and 30, respective

As described above, both $Fe^{2+}$ and $Zn^{2+}$ appear to form 2:1 complexes with levofloxacin; however, their influence on levofloxacin fluorescence are dissimilar (Figures 29 and 30). The exact reason for this is unclear at this point.

**Table 30. Fluorescence Characeristics of Levofloxacin in the Presence of Cations.**

| Cation | Fluorescence of levofloxacin | | Results | Comments |
|---|---|---|---|---|
| | pH 5.0 | pH 9.0 | | |
| $Ca^{2+}$ | Change not significant | Change not significant | N/A | - |
| $Mg^{2+}$ | Change not significant | Change not significant | N/A | - |
| $Fe^{2+}$ | Decrease in emission with increasing $Fe^{2+}$ | N/A | Figure 3.12 (pH 5.0) | $FeCl_2$ insoluble at pH 9.0 |
| $Zn^{2+}$ | Change not significant | Increase in emission with increasing $Zn^{2+}$ | Figure 3.13 (pH 9.0) | - |
| $Al^{3+}$ | Change not significant | N/A | N/A | $Al^2(SO_4)_3$ insoluble at pH 9.0 |

**Samples of Levofloxacin Complexes**

Seven samples of levofloxacin complexes were evaluated *in vivo* for efficacy and pharmacokinetics. Details of the samples tested are shown in Table 31 below.

**Table 31. Molar Ratios of Levofloxacin Complexes.**

| Sample identifier | Cation | Molar ratio used | Total Levofloxacin (mg/mL) | Final pH of the solution |
|---|---|---|---|---|
| NB-049-001-06-066A | $Mg^{2+}$ | 1:1 | 40.2 | 6.24 |
| NB-049-001-06-066B | $Fe^{2+}$ | 1:1 | 40.1 | 6.30 |
| NB-049-001-06-066C | $Mg^{2+}$ | 1:1 | 202 | 5.98 |
| NB-049-001-06-081A | $Ca^{2+}$ | 1:1 | 40.1 | 6.53 |
| NB-049-001-06-081B | $Ca^{2+}$ | 1:1 | 201 | 6.04 |
| NB-049-001-06-081C | $Zn^{2+}$ | 1:1 | 40 | 6.33 |
| NB-049-001-06-081D | $Zn^{2+}$ | 1:1 | 200 | 5.69 |

**Conclusions and Next Steps**

Results obtained from our dual titration studies suggest that levofloxacin forms 2:1 complexes with all the divalent metal cations. The binding constants (log $K_b$) for complexation with $Ca^{2+}$, $Mg^{2+}$, $Fe^{2+}$ and $Zn^{2+}$ are 2.75, 3.69, 4.44 and 4.54, respectively.

## Reference Example 12 - Levofloxacin and Gemifloxacin Formulations with Organic Acids.

### Experimental Methodology

Levofloxacin solution was prepared by dissolving either 50 or 100 mg levofloxacin base in 15-20 ml water. The initial pH of levofloxacin solution in water was about 7.3. The pH of the solution was adjusted with about 10% solution of acid prepared in water. The following acids were used to adjust the pH of the levofloxacin solution: acetic acid, ascorbic acid, citric, lactic, tartaric and propionic acid. After making up the volume of the solution to approximately 90% of the final volume, the osmolality of the solution was measured and adjusted to 300 mOsm/ kg with about 20% solution of sodium chloride prepared in water After pH and osmolality adjustment, the volume of the solution was made up to about 25 ml with water and its surface tension measured. The pH and osmolality were measured after making up the volume and are reported in Table 32. (The exact quantities of levofloxacin weighed, acid required to adjust pH, sodium chloride to adjust osmolality and final volume of solutions are listed in Table 32). The content of levofloxacin in the solutions was determined by HPLC.

### Results

The details about the levofloxacin formulations with organic acids are shown in Table 32. The results of HPLC are shown in Table 33.

When tartaric acid was used to adjust the pH of the 100 mg/ml levofloxacin solution, a precipitate was formed.

Note: Solutions with acetic acid, citric acid and ascorbic acid were remade for HPLC analysis and hence the theoretical concentration for these solutions in Table 32 and Table 33 are different.

Table 32. Formulations of Levofloxacin With Organic Acids.

| Wt of Levo used (g) | 9.94% acetic acid used (ml) | Acetic acid used (g) | 19.7% NaCl used (ml) | NaCl used (g) | Measured Final Vol of solution (ml) | Levo conc (mg/ml) | Final osmolality (mOsm/ kg) | Final pH | Surface tension (mN/m) |
|---|---|---|---|---|---|---|---|---|---|
| 1.253 | 1.05 | 0.104 | 0.681 | 0.134 | 25.105 | 49.9 | 312 | 6.48 | 63.2 |
| 2.501 | 2.05 | 0.204 | 0.326 | 0.064 | 25.935 | 96.4 | 300 | 6.53 | 62.5 |
| | | | | | | | | | |
| Wt of Levo used (g) | 9.99 % ascorbic acid used (ml) | ascorbic acid used (g) | 19.7% NaCl used (ml) | NaCl used (g) | Measured Final Vol of solution (ml) | Levo conc (mg/ml | Final osmolality (mOsm/kg) | Final pH | Surface tension (mN/m) |
| 1.253 | 3.400 | 0.339 | 0.550 | 0.108 | 25.135 | 49.8 | 297 | 6.40 | 64.4 |
| 2.505 | 7.400 | 0.739 | 0.300 | 0.059 | 25.135 | 99.7 | 298 | 6.47 | 62.5 |
| | | | | | | | | | |
| Wt of Levo used (g) | 10.05% citric acid used (ml) | citric acid used (g) | 21.54 % NaCl used (ml) | NaCl used (g) | Measured Final Vol of solution (ml) | Levo conc (mg/ml | Final osmolality (mOsm/ kg) | Final pH | Surface tension (mN/m) |
| 1.251 | 1.25 | 0.126 | 1.005 | 0.216 | 25.12 | 49.8 | 299 | 6.54 | 61.5 |
| 2.498 | 2.6 | 0.261 | 0.918 | 0.198 | 25.82 | 96.7 | 301 | 6.53 | 61.4 |
| | | | | | | | | | |
| Wt of Levo used (g) | 10% lactic used (ml) | Lactic acid used (g) | 21.54 % NaCl used (ml) | NaCl used (g) | Measured Final Vol of solution (ml) | Levo conc (mg/ml | Final osmolality (mOsm/ kg) | Final pH | Surface tension (mN/m) |
| 1.258 | 2.1 | 0.21 | 0.745 | 0.160 | 25.135 | 50.1 | 297 | 6.54 | 59.4 |
| 2.497 | 4.2 | 0.42 | 0.392 | 0.084 | 25.605 | 97.5 | 301 | 6.63 | 57.5 |
| | | | | | | | | | |
| Wt of Levo used (g) | 10% tartaric acid used (ml) | Tartaric acid used (g) | 21.54% NaCl used (ml) | NaCl used (g) | Measured Final Vol of solution (ml) | Levo conc (mg/ml) | Final osmolality (mOsm) | Final pH | Surface tension (mN/m) |
| 1.252 | 1.55 | 0.155 | 0.948 | 0.204 | 25.180 | 49.7 | 298 | 6.51 | 61.5 |
| | | | | | | | | | |
| Wt of Levo used (g) | 9.79% propionic acid used (ml) | propionic acid used(g) | 21.53% NaCl used (ml) | NaCl used (g) | Measured Final Vol of solution (ml) | Levo conc (mg/ml) | Final osmolality (mOsm) | Final pH | Surface tension (mN/m) |
| 1.25281 | 1.310 | 0.128 | 0.737 | 0.159 | 25.045 | 50.02 | 298 | 6.50 | 58.1 |
| 2.51342 | 2.610 | 0.256 | 0.310 | 0.067 | 25.030 | 100.42 | 297 | 6.57 | 52.0 |

(continued)

| Wt of Levo used (g) | 9.79% propionic acid used (ml) | propionic acid used(g) | 21.53% NaCl used (ml) | NaCl used (g) | Measured Final Vol of solution (ml) | Levo conc (mg/ml) | Final osmolality (mOsm) | Final pH | Surface tension (mN/m) |
|---|---|---|---|---|---|---|---|---|---|
| | | | | | | | | | |

* Theoretical concentration = Theoretical quantity of gemifloxacin in filtered solution (in this case 35 mg Gemifloxacin in filtered 1.3 mL)/ Final volume of solution (in this case 1.7 mL).

Table 33. Theoretical and Measured Concentrations of Levofloxacin in the Formulations.

| Acid | Theoretical Conc. (mg/mL) | Measured Concentration (mg/ml) by HPLC |
|---|---|---|
| acetic acid | 50.05 | 51.45 |
| acetic acid | 99.9 | 102.32 |
| citric acid | 49.91 | 50.31 |
| citric acid | 99.86 | 102.99 |
| L-ascorbic acid | 49.95 | 50.01 |
| L-ascorbic acid | 100 | 102.49 |
| lactic acid | 50.05 | 50.07 |
| lactic acid | 97.54 | 95.27 |
| tartaric acid | 49.74 | 51.07 |
| Note: Solutions with acetic acid, citric acid and ascorbic acid were remade for HPLC analysis and hence the theoretical concentration for these solutions in Table 32 and Table 33 are different. | | |

**Reference Example 13 - Inhalation Toxicology in Rats.**

In a 4 day non-GLP ascending dose study of aerosolized levofloxacin in male and female Sprague-Dawley rats, a 25 mg/ml solution of levofloxacin was administered for one hour on day one and a 50 mg/ml solution of levofloxacin was administered for two hours per day on days 2 thru 4. No clinical signs of toxicity were observed during the treatment period. Necropsy 24 hours after administration of the last dose did not show any findings.

In a GLP study of aerosolized levofloxacin in male and female Sprague-Dawley rats, aerosolized levofloxacin was administered daily with an average dose of 6.92 mg/kg/day to the males and 10.04 mg/kg/day for the females over 4 days using a nose-only aerosol delivery device. Total exposures were 29 and 42 mg/kg for males and females, respectively over the study period. Each dose was delivered over 2 hours daily. The dose for this study was chosen based on the maximum solubility of levofloxacin that could be administered in the device over 2 hours. No clinical signs of toxicity were observed, and all animals survived during the 4 day treatment period. Necropsy of animals after administration of the last dose did not show any findings.

In a 28-day GLP study in Sprague-Dawley rats, animals were randomized to 3 dose levels of aerosolized levofloxacin or saline. Additional recovery groups using the vehicle control and the highest dose were also treated and observed for a 14 day recovery period following the last dose. Average aerosolized levofloxacin doses were 1.49, 3.63, and 7.29 mg/kg/day for male rats, and 2.20, 5.35, and 11.01 mg/kg/day in female rats. The total exposures over the 28-day treatment period ranged between 41.7 and 204.1 mg/kg for males and 61.6 and 308.3 mg/kg for females. Each dose was delivered over 2 hours daily. No dose related clinical signs of toxicity were observed, and all animals survived during the 28 day treatment period. Necropsy of animals after administration of the last dose showed a dose related squamous cell hyperplasia of the larynx which declined in severity during a 14 day recovery period.

## Claims

**1.** A method of making an aerosol of a solution comprising levofloxacin or ofloxacin and a divalent or trivalent cation, said method comprising:

obtaining a solution comprising levofloxacin or ofloxacin and a divalent or trivalent cation; and
aerosolizing said solution to achieve an aerosol comprising a mass median aerodynamic diameter of 5$\mu$m or less, wherein said aerosol is suitable for inhalation into a lung.

**2.** The method of claim 1, wherein said aerosolizing comprises nebulizing said solution with a nebulizer, preferably a vibrating mesh nebulizer or a compressor driven nebulizer.

**3.** The method of anyone of claims 1-2, wherein the divalent or trivalent cation is selected from the group consisting of magnesium, calcium, aluminum, zinc, and iron.

**4.** The method of any one of claims 1-3, wherein the solution has a permeant ion concentration from about 25 mM to about 400 mM, preferably from about 30 mM to about 300 mM, more preferred from about 40 mM to about 200 mM.

**5.** The method of claim 4 wherein the permeant ion is selected from chloride and bromide

**6.** The method of any one of claims 1-5, wherein the solution has a concentration of levofloxacin or ofloxacin of about 1 mg/ml to about 700 mg/ml, preferably about 7 mg/ml to 700 mg/ml, preferably greater than about 25 mg/ml, more preferred greater than about 35 mg/ml, even more preferred greater than about 40 mg/ml, highly preferred greater than about 50 mg/ml, and/or optionally wherein the solution has an osmolality from about 200 mOsmol/kg to about 1250 mOsmol/kg, preferably from about 250 mOsmol/kg to about 1050 mOsmol/kg, more preferred from about 350 mOsmol/kg to about 750 mOsmol/kg; and/or optionally wherein the solution has a pH from about 4.5 to about 7.5, preferably from about 5.5 to about 7.0, more preferred from about 5.5 to about 6.5.

**7.** The method of any one of claims 1-6, wherein the solution comprises magnesium and greater than about 50 mg/ml levofloxacin, and has an osmolality from about 350 mOsmol/kg to about 750 mOsmol/kg, and a pH from about 5.5 to about 6.5.

**8.** The method of any one of claims 1-7, wherein the aerosol has a mass median aerodynamic diameter from about 2 microns to about 5 microns with a geometric standard deviation less than or equal to about 2.5 microns, preferably from about 2.5 microns to about 4.5 microns with a geometric standard deviation less than or equal to about 1.8 microns, more preferred from about 2.8 microns to about 4.3 microns with a geometric standard deviation less than or equal to about 2 microns.

**9.** The method of any one of claims 1-8, wherein the solution further comprises a component selected from the group consisting of a sweetener, second antimicrobial, dornase alpha, a hypertonic formulation, mannitol, and sodium chloride.

**10.** The method of claim 9, wherein the second antimicrobial is selected from the group consisting of an aminoglycoside, polymyxin, monobactam, macrolide, ketolide, glycopeptide, and fluoroquinolone.

**11.** The method of claim 9, wherein the aminoglycoside is tobramycin, the polymyxin is colistin, the monobactam is aztreonam, the glycopeptide is vancomycin or the fluoroquinolone is selected from the group consisting of lomefloxacin, pefloxacin, ciprofloxacin, gatifloxacin, gemifloxacin, moxifloxacin, tosufloxacin, pazufloxacin, rufloxacin, fleroxacin, balofloxacin, sparfloxacin, trovafloxacin, enoxacin, norfloxacin, clinafloxacin, grepafloxacin, sitafloxacin, temafloxacin, marbofloxacin, orbifloxacin, sarafloxacin, danofloxacin, difloxacin, enrofloxacin, garenoxacin, prulifloxacin, olamufloxacin, DX-619, TG-873870 and DW-286.

**12.** A pharmaceutical composition, comprising an aqueous solution of levofloxacin or ofloxacin and a divalent or trivalent cation, wherein the solution is suitable for inhalation into a lung.

**13.** The pharmaceutical composition of claim 12, comprising an aqueous solution of levofloxacin and a divalent or trivalent cation.

**14.** The pharmaceutical composition of claim 13, wherein the divalent or trivalent cation is selected from $Mg^{2+}$, $Ca^{2+}$, $Zn^{2+}$, $Fe^{2+}$ and $Al^{3+}$.

**15.** The pharmaceutical composition of claim 12 or claim 13, wherein the cation is a divalent cation selected from magnesium or calcium.

**16.** The pharmaceutical composition of claim 13, wherein the cation is $Mg^{2+}$.

## Patentansprüche

**1.** Ein Verfahren zum Herstellen eines Aerosols aus einer Lösung, die Levofloxacin oder Ofloxacin und ein zweiwertiges oder dreiwertiges Kation beinhaltet, wobei das Verfahren Folgendes beinhaltet:

Erhalten einer Lösung, die die Levofloxacin oder Ofloxacin und ein zweiwertiges oder dreiwertiges Kation

beinhaltet; und
Aerosolisieren der Lösung, um ein Aerosol zu erzielen, das einen medianen aerodynamischen Massendurchmesser von 5 μm oder weniger beinhaltet, wobei das Aerosol für die Inhalation in eine Lunge geeignet ist.

**2.** Verfahren gemäß Anspruch 1, wobei das Aerosolisieren das Vernebeln der Lösung mit einem Vernebler, bevorzugt einem vibrierenden Mesh-Vernebler oder einem kompressorgetriebenen Vernebler beinhaltet.

**3.** Verfahren gemäß einem der Ansprüche 1-2, wobei das zweiwertige oder dreiwertige Kation aus der Gruppe ausgewählt ist, die aus Magnesium, Calcium, Aluminium, Zink und Eisen besteht.

**4.** Verfahren gemäß einem der Ansprüche 1-3, wobei die Lösung eine Konzentration permeierender Ionen von etwa 25 mM bis etwa 400 mM, bevorzugt von etwa 30 mM bis etwa 300 mM, besonders bevorzugt von etwa 40 mM bis etwa 200 mM aufweist.

**5.** Verfahren gemäß Anspruch 4, wobei das permeierende Ion aus Chlorid und Bromid ausgewählt ist.

**6.** Verfahren gemäß einem der Ansprüche 1-5, wobei die Lösung eine Konzentration von Levofloxacin oder Ofloxacin von etwa 1 mg/ml bis etwa 700 mg/ml, bevorzugt etwa 7 mg/ml bis 700 mg/ml, bevorzugt über 25 mg/ml, besonders bevorzugt über etwa 35 mg/ml, noch bevorzugter über 40 mg/ml, höchst bevorzugt über 50 mg/ml aufweist, und/oder optional wobei die Lösung eine Osmolalität von etwa 200 mOsmol/kg bis etwa 1250 mOsmol/kg, bevorzugt von etwa 250 mOsmol/kg bis etwa 1050 mOsmol/kg, besonders bevorzugt von etwa 350 mOsmol/kg bis etwa 750 mOsmol/kg aufweist; und/oder optional wobei die Lösung einen pH von etwa 4,5 bis etwa 7,5, bevorzugt von etwa 5,5 bis etwa 7,0, besonders bevorzugt von etwa 5,5 bis etwa 6,5 aufweist.

**7.** Verfahren gemäß einem der Ansprüche 1-6, wobei die Lösung Magnesium und über etwa 50 mg/ml Levofloxacin beinhaltet und eine Osmolalität von etwa 350 mOsmol/kg bis etwa 750 mOsmol/kg und einen pH von etwa 5,5 bis etwa 6,5 aufweist.

**8.** Verfahren gemäß einem der Ansprüche 1-7, wobei das Aerosol einen medianen aerodynamischen Massendurchmesser von etwa 2 Micron bis etwa 5 Micron mit einer geometrischen Standardabweichung von weniger als oder gleich etwa 2,5 Micron, bevorzugt von etwa 2,5 Micron bis etwa 4,5 Micron mit einer geometrischen Standardabweichung von weniger als oder gleich etwa 1,8 Micron, besonders bevorzugt von etwa 2,8 Micron bis etwa 4,3 Micron mit einer geometrischen Standardabweichung von weniger als oder gleich etwa 2 Micron aufweist.

**9.** Verfahren gemäß einem der Ansprüche 1-8, wobei die Lösung ferner eine Komponente beinhaltet, die aus der Gruppe ausgewählt ist, die aus einem Süßstoff, zweiten Antimikrobiotikum, Dornase alfa, einer hypertonischen Formulierung, Mannitol oder Natriumchlorid besteht.

**10.** Verfahren gemäß Anspruch 9, wobei das zweite Antimikrobiotikum aus der Gruppe ausgewählt ist, die aus einem Aminoglykosid, Polymyxin, Monobactam, Makrolid, Ketolid, Glykopeptid und Fluoroquinolon besteht.

**11.** Verfahren gemäß Anspruch Anspruch 9, wobei das Aminoglykosid Tobramycin ist, das Polymyxin Colistin ist, das Monobactam Aztreonam ist, das Glykopeptid Vancomycin ist oder das Fluoroquinolon aus der Gruppe ausgewählt ist, die aus Lomefloxacin, Pefloxacin, Ciprofloxacin, Gatifloxacin, Gemifloxacin, Moxifloxacin, Tosufloxacin, Pazufloxacin, Rufloxacin, Fleroxacin, Balofloxacin, Sparfloxacin, Trovafloxacin, Enoxacin, Norfloxacin, Clinafloxacin, Grepafloxacin, Sitafloxacin, Temafloxacin, Marbofloxacin, Orbifloxacin, Sarafloxacin, Danofloxacin, Difloxacin, Enrofloxacin, Garenoxacin, Prulifloxacin, Olamufloxacin, DX-619, TG-873870 und DW-286 besteht.

**12.** Pharmazeutische Zusammensetzung, die eine wässrige Lösung aus Levofloxacin oder Ofloxacin und einem zweiwertigen oder dreiwertigen Kation beinhaltet, wobei die Lösung für die Inhalation in eine Lunge geeignet ist.

**13.** Pharmazeutische Zusammensetzung gemäß Anspruch 12, die eine wässrige Lösung aus Levofloxacin und einem zweiwertigen oder dreiwertigen Kation beinhaltet.

**14.** Pharmazeutische Zusammensetzung gemäß Anspruch 13, wobei das zweiwertige oder dreiwertige Kation aus $Mg^{2+}$, $Ca^{2+}$, $Zn^{2+}$, $Fe^{2+}$ und $Al^{3+}$ ausgewählt ist.

**15.** Pharmazeutische Zusammensetzung gemäß Anspruch 12 oder Anspruch 13, wobei das Kation ein zweiwertiges

Kation ist, das aus Magnesium oder Calcium ausgewählt ist.

**16.** Pharmazeutische Zusammensetzung gemäß Anspruch 13, wobei das Kation $Mg^{2+}$ ist.

**Revendications**

**1.** Procédé pour fabriquer un aérosol d'une solution comprenant de la lévofloxacine ou de l'ofloxacine et un cation bivalent ou trivalent, ledit procédé comprenant :

l'obtention d'une solution comprenant de la lévofloxacine ou de l'ofloxacine et un cation bivalent ou trivalent ; et l'aérosolisation de ladite solution pour obtenir un aérosol comprenant un diamètre aérodynamique moyen en masse de 5 $\mu$m ou moins, ledit aérosol convenant à l'inhalation dans un poumon.

**2.** Procédé selon la revendication 1, dans lequel ladite aérosolisation comprend la nébulisation de ladite solution avec un nébuliseur, de préférence un nébuliseur à grille vibratoire ou un nébuliseur entraîné par compresseur.

**3.** Procédé selon l'une quelconque des revendications 1-2, dans lequel le cation bivalent ou trivalent est sélectionné dans le groupe constitué de : magnésium, calcium, aluminium, zinc et fer.

**4.** Procédé selon l'une quelconque des revendications 1-3, dans lequel la solution a une concentration d'ions perméants d'environ 25 mM à environ 400 mM, de préférence d'environ 30 mM à environ 300 mM, ou mieux encore d'environ 40 mM à environ 200 mM.

**5.** Procédé selon la revendication 4, dans lequel l'ion perméant est sélectionné entre chlorure et bromure.

**6.** Procédé selon l'une quelconque des revendications 1-5, dans lequel la solution a une concentration de lévofloxacine ou d'ofloxacine d'environ 1 mg/ml à environ 700 mg/ml, par ordre ascendant de préférence: 7 mg/ml à 700 mg/ml, supérieure à environ 25 mg/ml, supérieure à environ 35 mg/ml, supérieure à environ 40 mg/ml, la concentration optimale étant supérieure à environ 50 mg/ml, et/ou dans lequel la solution a une osmolalité d'environ 200 mOsmol/kg à environ 1250 mOsmol/kg, de préférence d'environ 250 mOsmol/kg à environ 1050 mOsmol/kg, ou mieux encore d'environ 350 mOsmol/kg à environ 750 mOsmol/kg ; et/ou optionnellement dans lequel la solution a un pH d'environ 4,5 à environ 7,5, de préférence d'environ 5,5 à environ 7,0, ou mieux encore d'environ 5,5 à environ 6,5.

**7.** Procédé selon l'une quelconque des revendications 1-6, dans lequel la solution comprend du magnésium et plus d'environ 50 mg/ml de lévofloxacine, et a une osmolalité d'environ 350 mOsmol/kg à environ 750 mOsmol/kg, et un pH d'environ 5,5 à environ 6,5.

**8.** Procédé selon l'une quelconque des revendications 1-7, dans lequel l'aérosol a un diamètre aérodynamique moyen en masse d'environ 2 microns à environ 5 microns avec un écart-type géométrique inférieur ou égal à environ 2,5 microns, de préférence d'environ 2,5 microns à environ 4,5 microns avec un écart-type géométrique inférieur ou égal à environ 1,8 micron, ou mieux encore d'environ 2,8 microns à environ 4,3 microns avec un écart-type géométrique inférieur ou égal à environ 2 microns.

**9.** Procédé selon l'une quelconque des revendications 1-8, dans lequel la solution comprend en outre un composant sélectionné dans le groupe constitué de : un édulcorant, un deuxième antimicrobien, domase alpha, une formulation hypertonique, mannitol, et chlorure de sodium.

**10.** Procédé selon la revendication 9, dans lequel le deuxième antimicrobien est sélectionné dans le groupe constitué de : un aminoglycoside, polymyxine, monobactam, macrolide, kétolide, glycopeptide et fluoroquinolone.

**11.** Procédé selon la revendication 9, dans lequel l'aminoglycoside est la tobramycine, la polymyxine est la colistine, le monobactam est l'aztréonam, le glycopeptide est la vancomycine, ou la fluoroquinolone est sélectionnée dans le groupe constitué de : loméfloxacine, péfloxacine, ciprofloxacine, gatifloxacine, gémifloxacine, moxifloxacine, tosufloxacine, pazufloxacine, rufloxacine, fléroxacine, balofloxacine, sparfloxacine, trovafloxacine, énoxacine, norfloxacine, clinafloxacine, grépafloxacine, sitafloxacine, témafloxacine, marbofloxacine, orbifloxacine, sarafloxacine, danofloxacine, difloxacine, enrofloxacine, garénoxacine, prulifloxacine, olamufloxacine, DX-619, TG-873870 et DW-286.

**12.** Composition pharmaceutique, comprenant une solution aqueuse de lévofloxacine ou d'ofloxacine et un cation bivalent ou trivalent, la solution convenant à l'inhalation dans un poumon.

**13.** Composition pharmaceutique selon la revendication 12, comprenant une solution aqueuse de lévofloxacine et un cation bivalent ou trivalent.

**14.** Composition selon la revendication 13, dans laquelle le cation bivalent ou trivalent est sélectionné entre $Mg^{2+}$, $Ca^{2+}$, $Zn^{2+}$, $Fe^{2+}$ et $Al^{3+}$.

**15.** Composition pharmaceutique selon la revendication 12 ou la revendication 13, dans laquelle le cation est un cation bivalent sélectionné entre le magnésium et le calcium.

**16.** Composition pharmaceutique selon la revendication 13, dans laquelle le cation est $Mg^{2+}$.

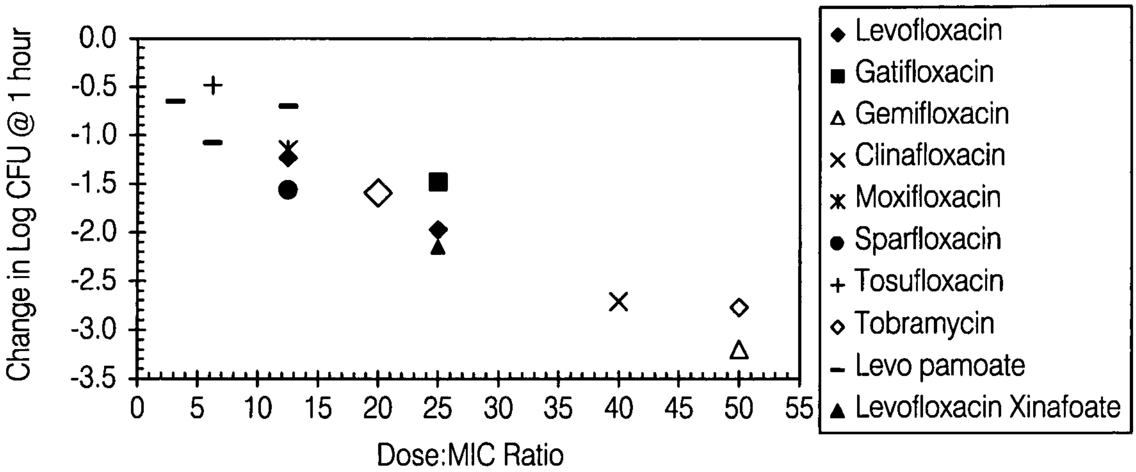
Change in Log CFU @ 1 hour
0.0
-0.5
-1.0
-1.5
-2.0
-2.5
-3.0
-3.5
0 5 10 15 20 25 30 35 40 45 50 55
Dose:MIC Ratio
Levofloxacin
Gatifloxacin
Gemifloxacin
Clinafloxacin
Moxifloxacin
Sparfloxacin
Tosufloxacin
Tobramycin
Levo pamoate
Levofloxacin Xinafoate

# FIG. 1

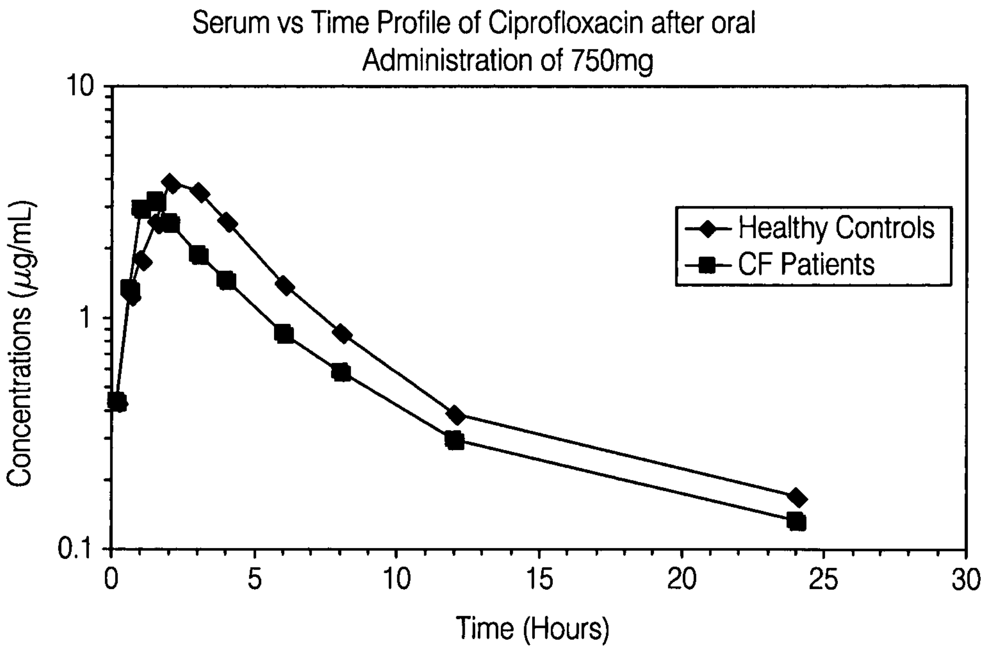
Serum vs Time Profile of Ciprofloxacin after oral Administration of 750mg
Concentrations (μg/mL)
10
1
0.1
0 5 10 15 20 25 30
Time (Hours)
Healthy Controls
CF Patients

# FIG. 2

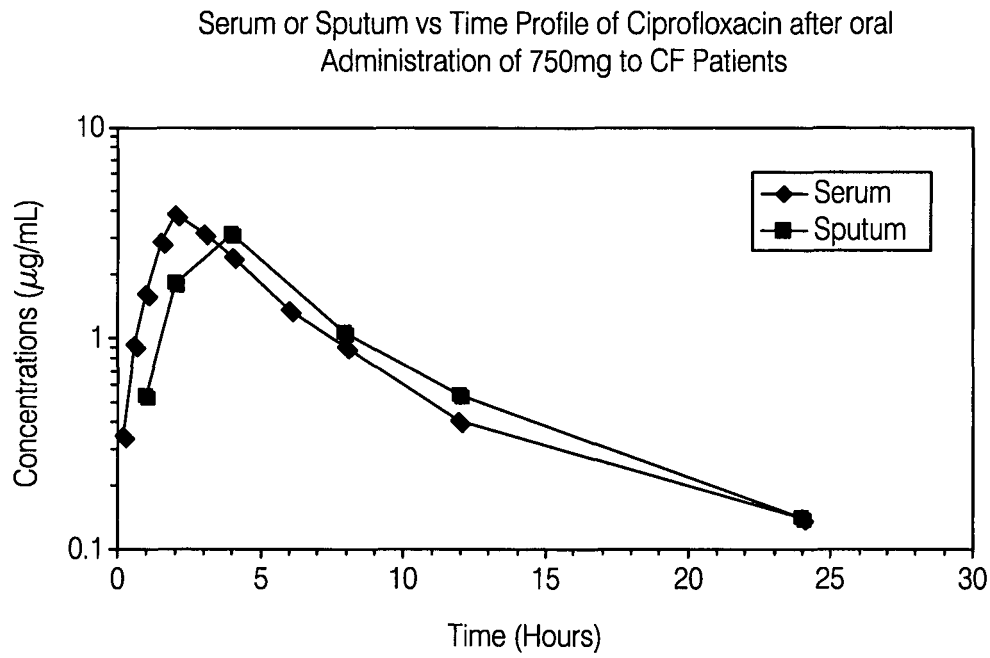
Serum or Sputum vs Time Profile of Ciprofloxacin after oral Administration of 750mg to CF Patients
Concentrations (μg/mL)
10
1
0.1
0
5
10
15
20
25
30
Time (Hours)
Serum
Sputum

FIG. 3

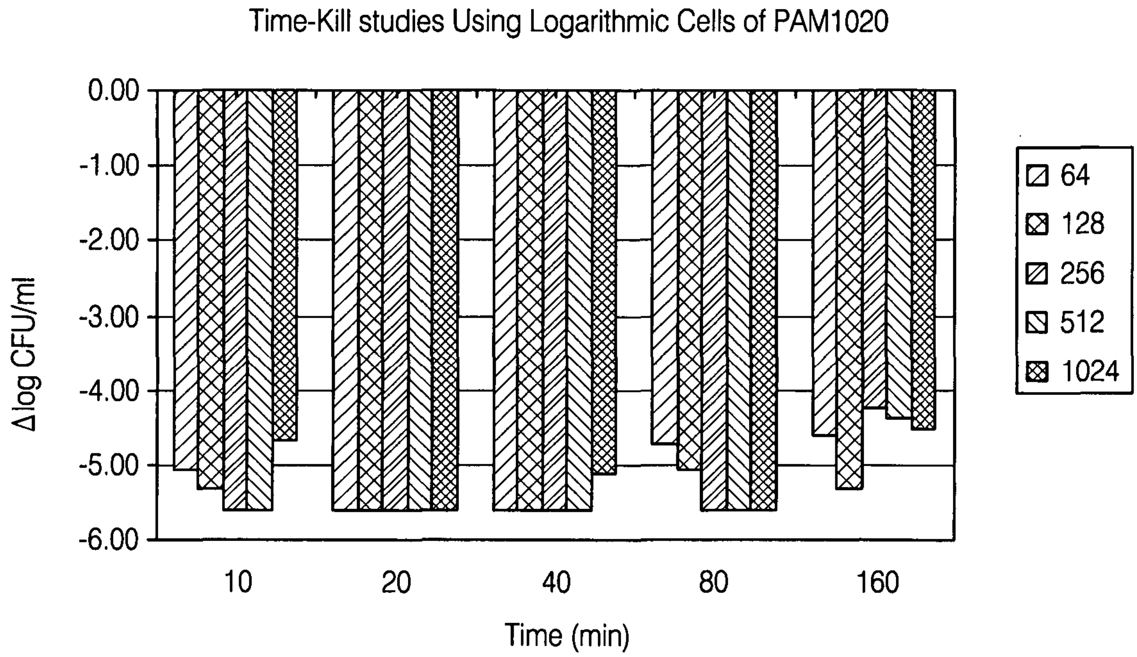
Time-Kill studies Using Logarithmic Cells of PAM1020
Δlog CFU/ml
0.00
-1.00
-2.00
-3.00
-4.00
-5.00
-6.00
10
20
40
80
160
Time (min)
64
128
256
512
1024

FIG. 4A

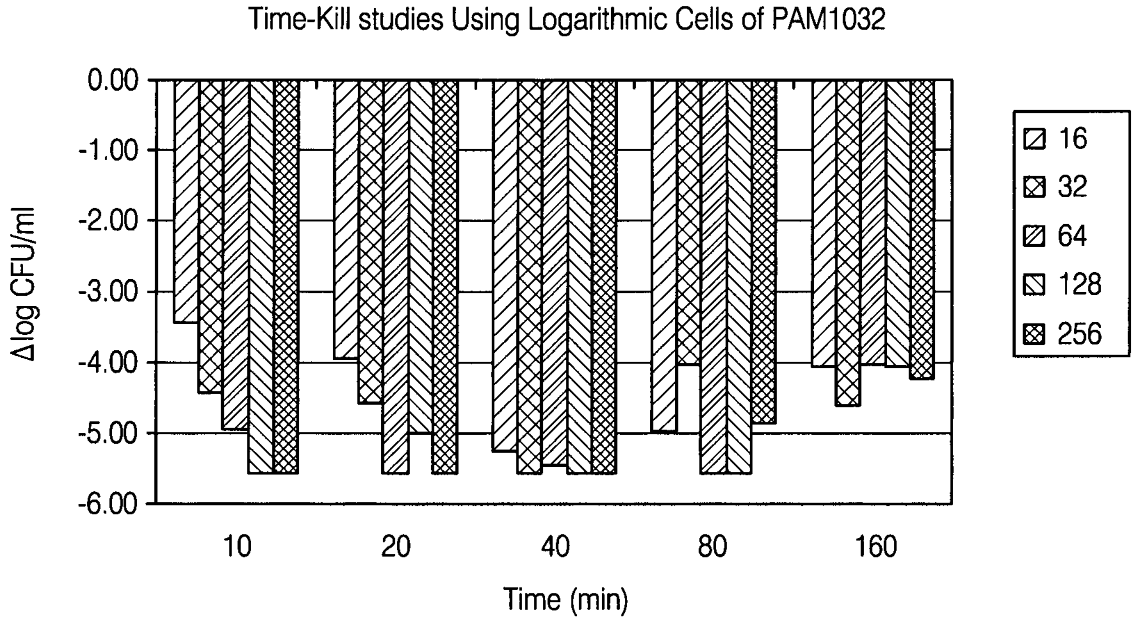
Time-Kill studies Using Logarithmic Cells of PAM1032
0.00
-1.00
-2.00
-3.00
-4.00
-5.00
-6.00
Δlog CFU/ml
10
20
40
80
160
Time (min)
16
32
64
128
256

FIG. 4B

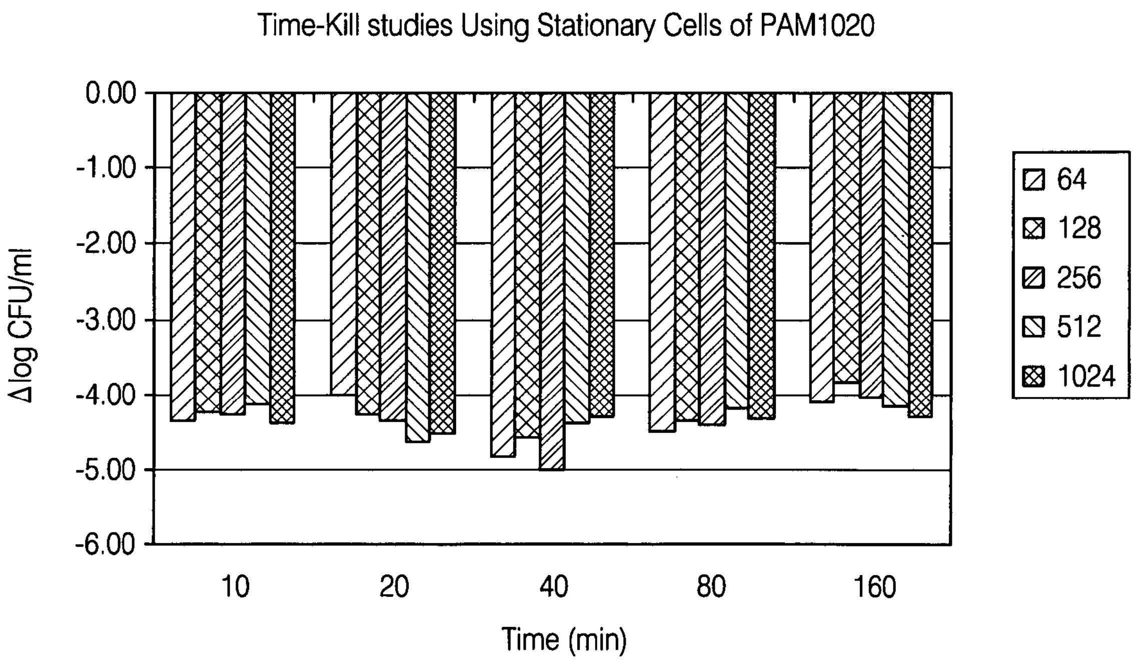
Time-Kill studies Using Stationary Cells of PAM1020
0.00
-1.00
-2.00
-3.00
-4.00
-5.00
-6.00
Δlog CFU/ml
10
20
40
80
160
Time (min)
64
128
256
512
1024

FIG. 5A

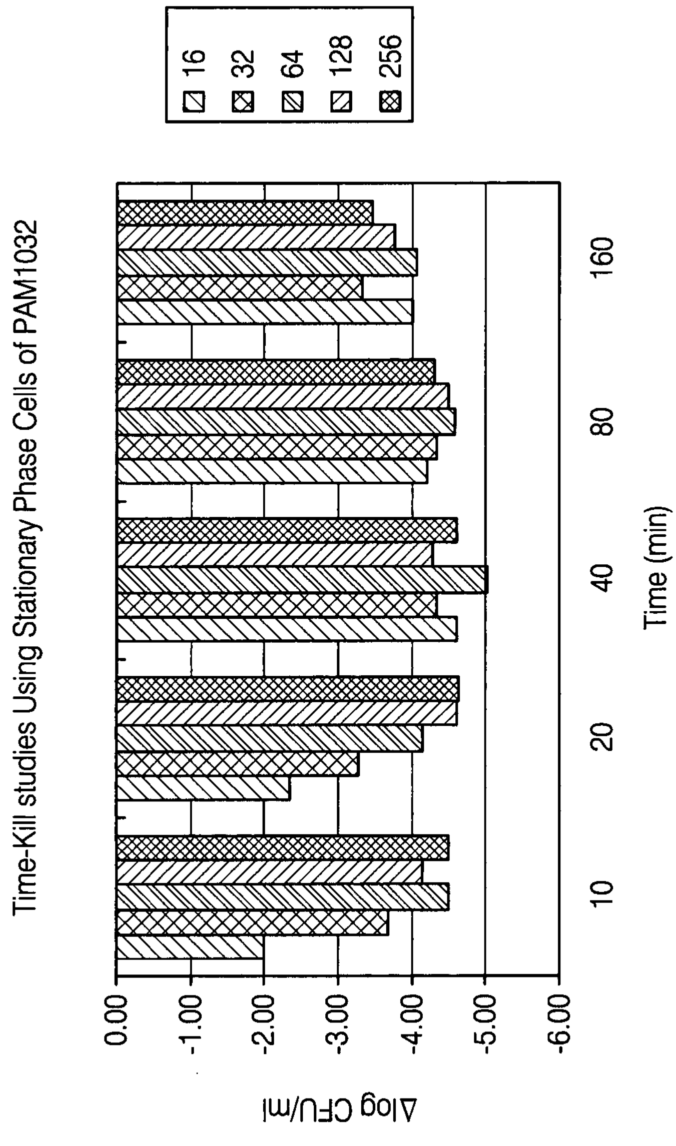
Time-Kill studies Using Stationary Phase Cells of PAM1032
0.00
-1.00
-2.00
-3.00
-4.00
-5.00
-6.00
Δlog CFU/ml
10
20
40
80
160
Time (min)
16
32
64
128
256

FIG. 5B

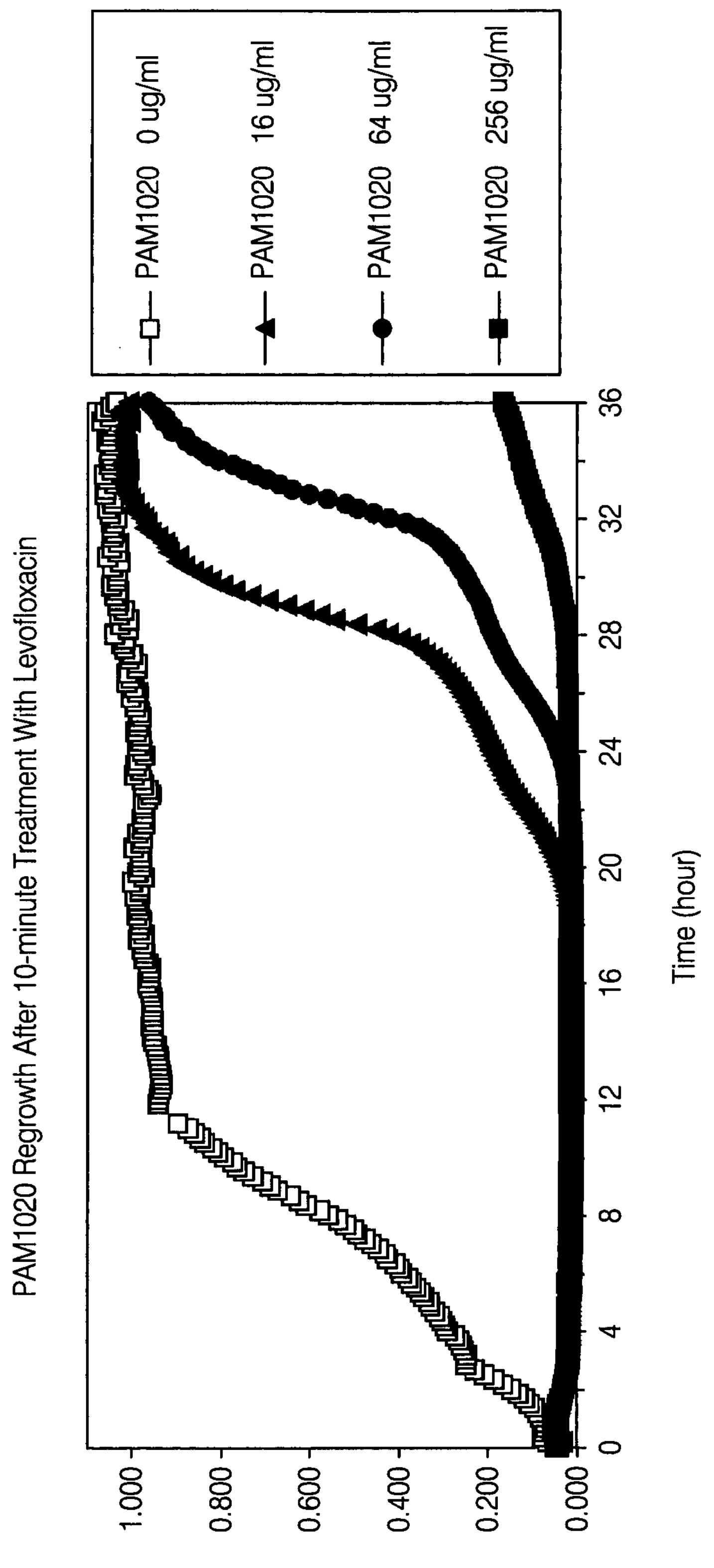
PAM1020 Regrowth After 10-minute Treatment With Levofloxacin
OD660
1.000
0.800
0.600
0.400
0.200
0.000
0
4
8
12
16
20
24
28
32
36
Time (hour)
PAM1020 0 ug/ml
PAM1020 16 ug/ml
PAM1020 64 ug/ml
PAM1020 256 ug/ml

FIG. 6A

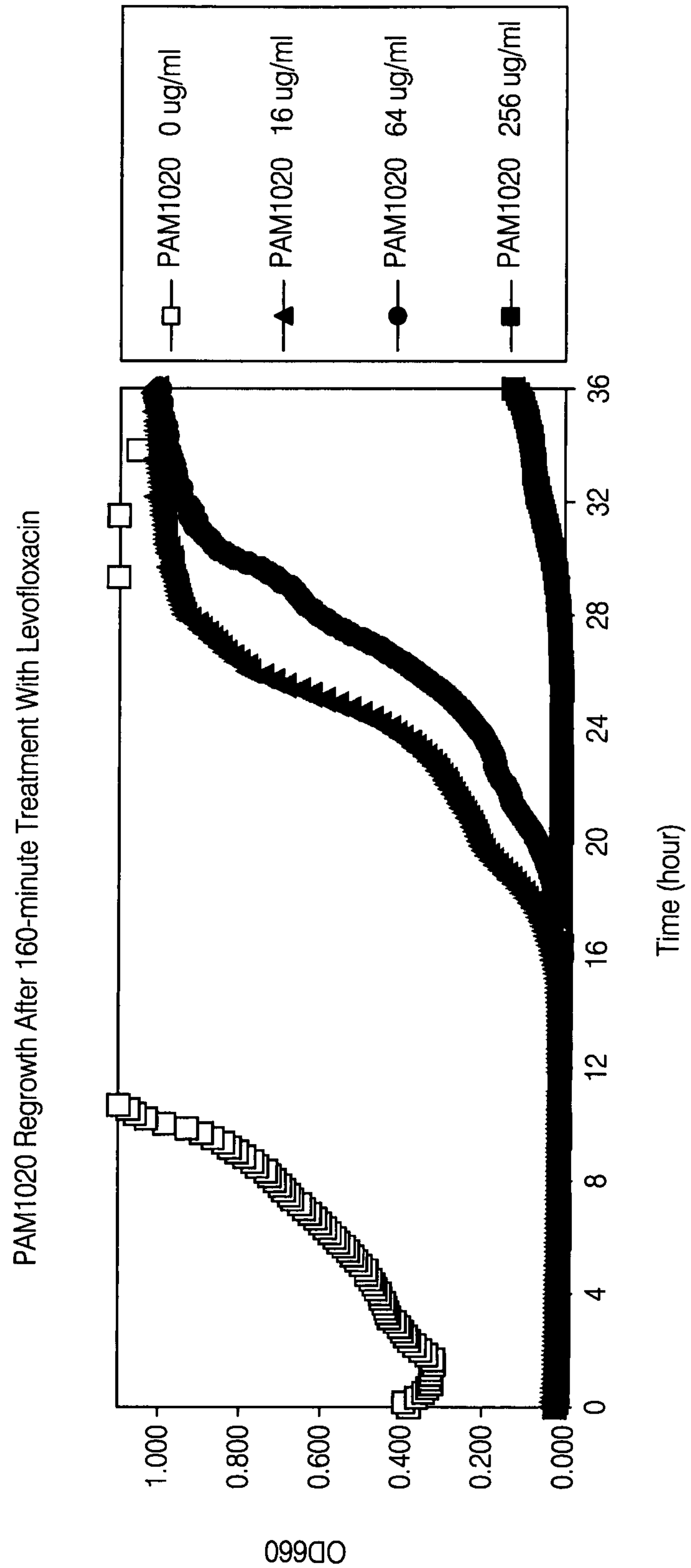
PAM1020 Regrowth After 160-minute Treatment With Levofloxacin
OD660
1.000
0.800
0.600
0.400
0.200
0.000
0
4
8
12
16
20
24
28
32
36
Time (hour)
PAM1020 0 ug/ml
PAM1020 16 ug/ml
PAM1020 64 ug/ml
PAM1020 256 ug/ml

FIG. 6B

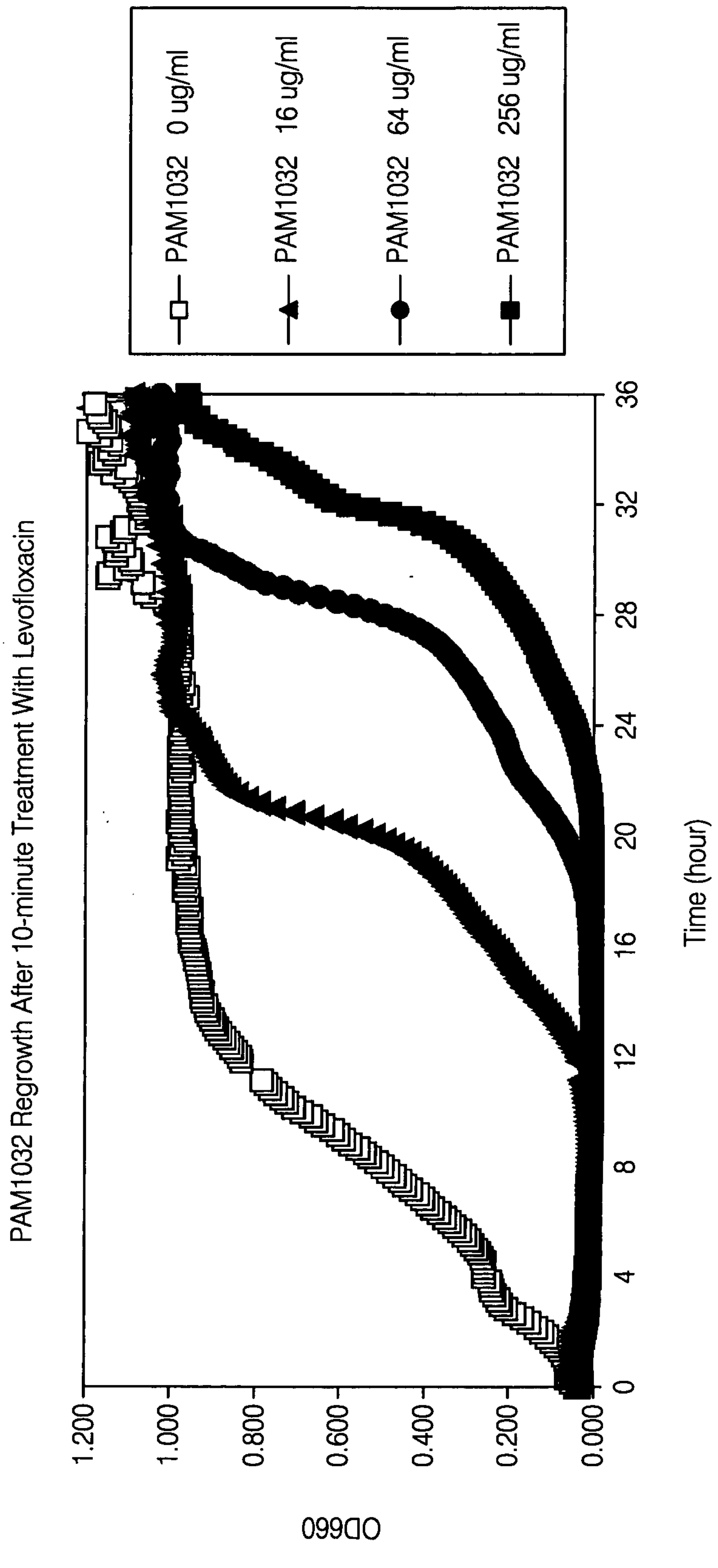
PAM1032 Regrowth After 10-minute Treatment With Levofloxacin
OD660
1.200
1.000
0.800
0.600
0.400
0.200
0.000
0
4
8
12
16
20
24
28
32
36
Time (hour)
PAM1032 0 ug/ml
PAM1032 16 ug/ml
PAM1032 64 ug/ml
PAM1032 256 ug/ml

FIG. 6C

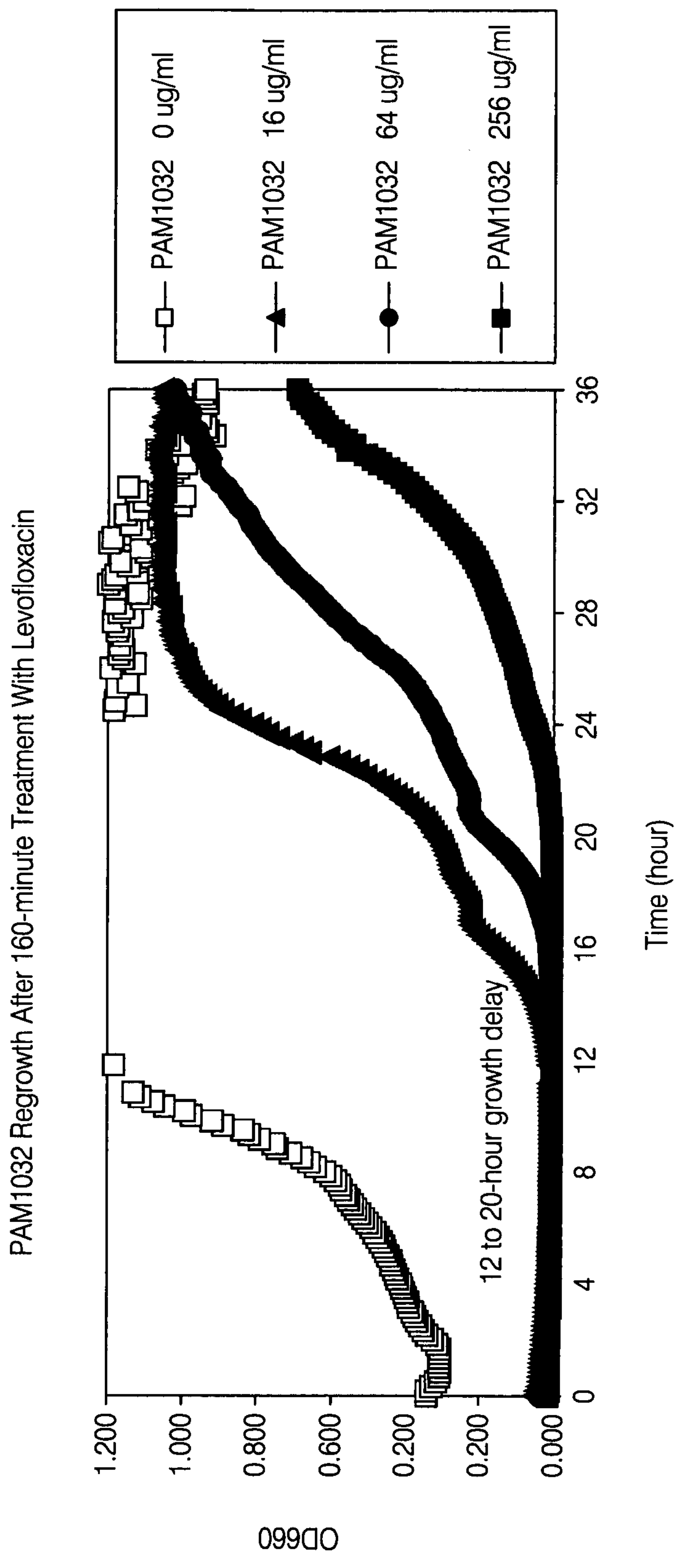
PAM1032 Regrowth After 160-minute Treatment With Levofloxacin
OD660
1.200
1.000
0.800
0.600
0.200
0.200
0.000
0
4
8
12
16
20
24
28
32
36
Time (hour)
12 to 20-hour growth delay
PAM1032 0 ug/ml
PAM1032 16 ug/ml
PAM1032 64 ug/ml
PAM1032 256 ug/ml

FIG. 6D

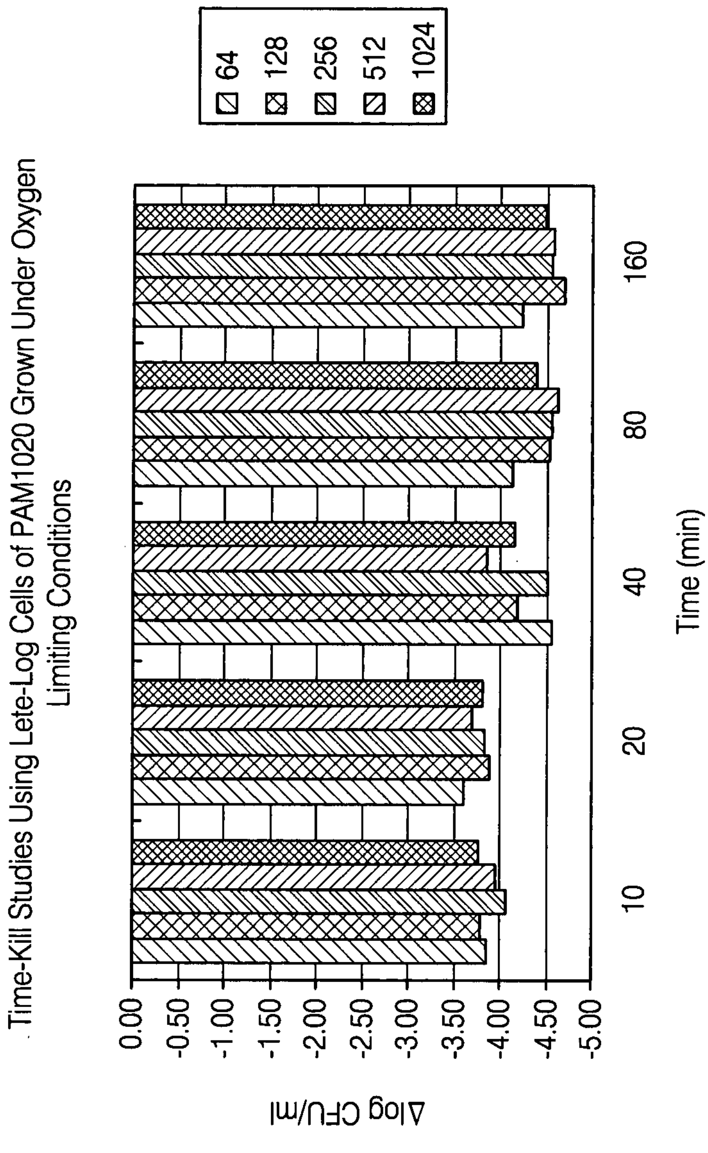
Time-Kill Studies Using Lete-Log Cells of PAM1020 Grown Under Oxygen Limiting Conditions
Δlog CFU/ml
0.00
-0.50
-1.00
-1.50
-2.00
-2.50
-3.00
-3.50
-4.00
-4.50
-5.00
10
20
40
80
160
Time (min)
64
128
256
512
1024

FIG. 7A

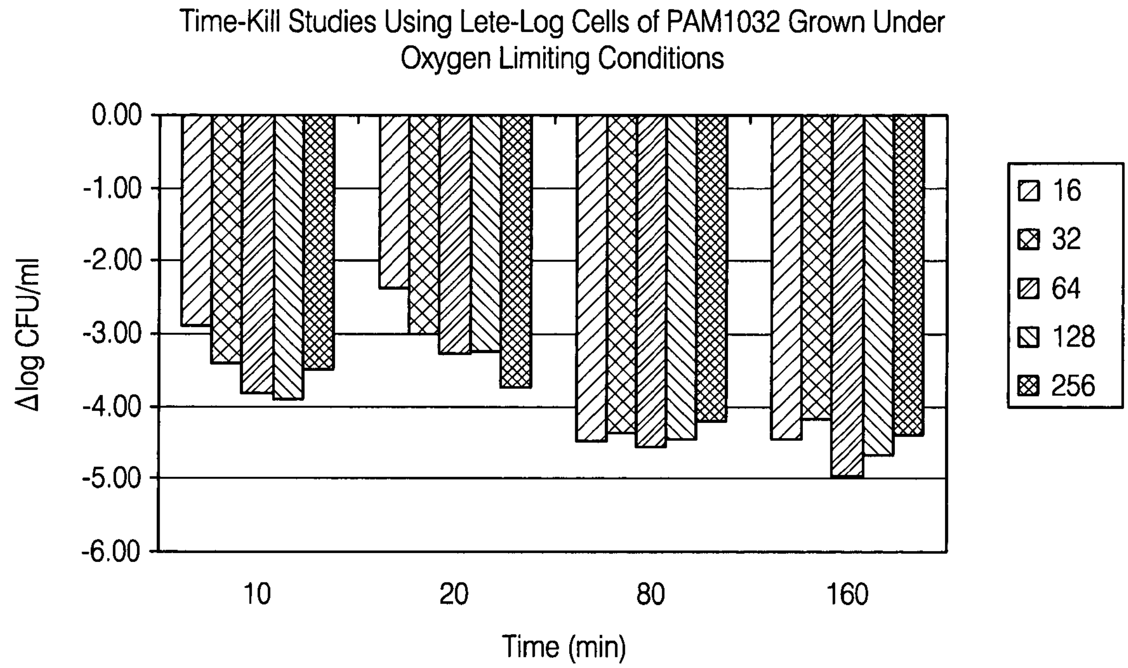
Time-Kill Studies Using Lete-Log Cells of PAM1032 Grown Under Oxygen Limiting Conditions
0.00
-1.00
-2.00
-3.00
-4.00
-5.00
-6.00
Δlog CFU/ml
10
20
80
160
Time (min)
16
32
64
128
256

FIG. 7B

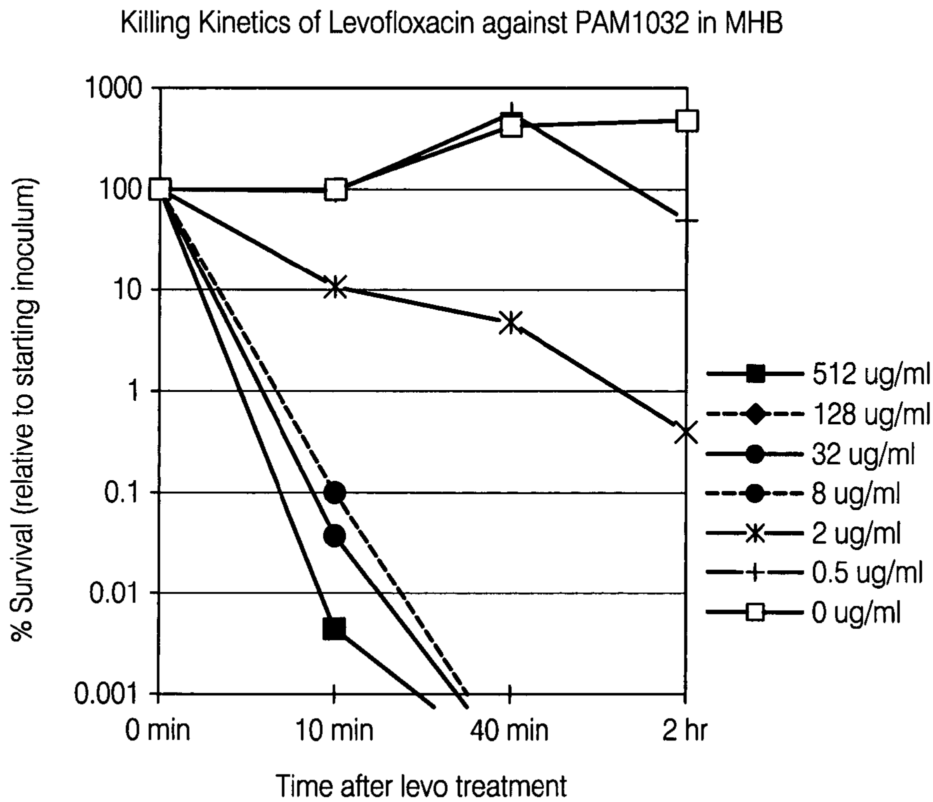
Killing Kinetics of Levofloxacin against PAM1032 in MHB
1000
100
10
1
0.1
0.01
0.001
% Survival (relative to starting inoculum)
0 min
10 min
40 min
2 hr
Time after levo treatment
512 ug/ml
128 ug/ml
32 ug/ml
8 ug/ml
2 ug/ml
0.5 ug/ml
0 ug/ml

FIG. 8A

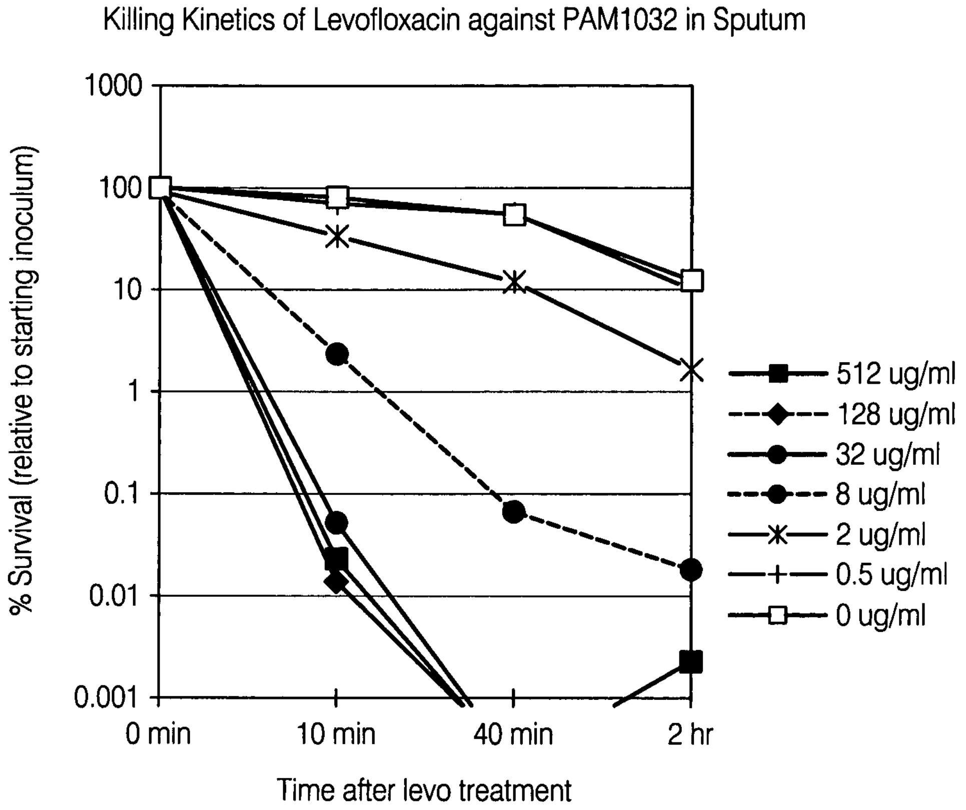
Killing Kinetics of Levofloxacin against PAM1032 in Sputum
1000
100
10
1
0.1
0.01
0.001
% Survival (relative to starting inoculum)
0 min
10 min
40 min
2 hr
Time after levo treatment
512 ug/ml
128 ug/ml
32 ug/ml
8 ug/ml
2 ug/ml
0.5 ug/ml
0 ug/ml

FIG. 8B

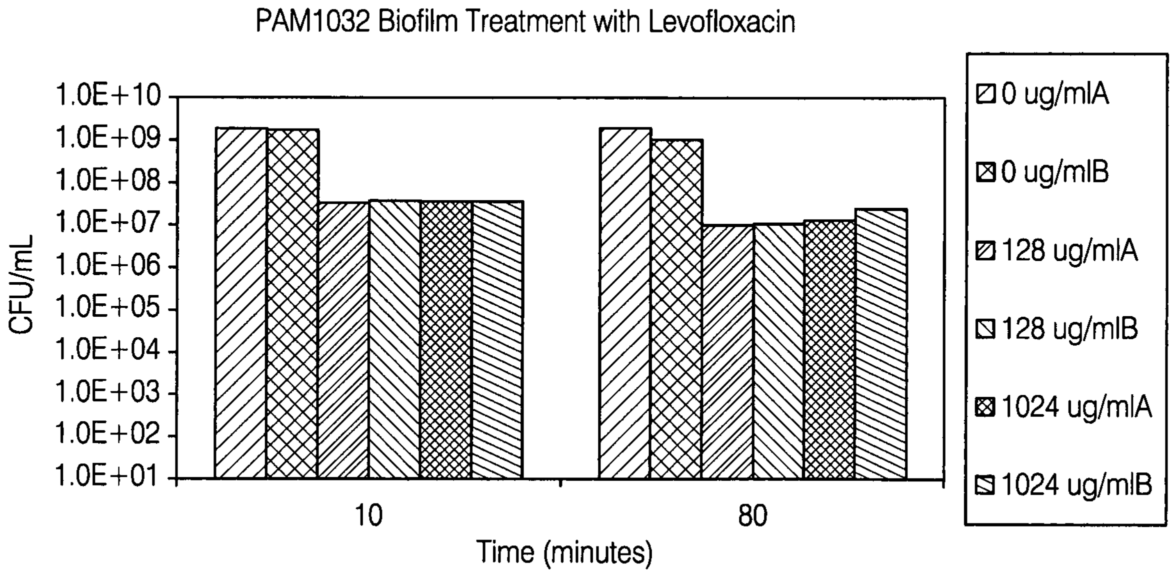
PAM1032 Biofilm Treatment with Levofloxacin
1.0E+10
1.0E+09
1.0E+08
1.0E+07
1.0E+06
1.0E+05
1.0E+04
1.0E+03
1.0E+02
1.0E+01
CFU/mL
10
80
Time (minutes)
0 ug/mlA
0 ug/mlB
128 ug/mlA
128 ug/mlB
1024 ug/mlA
1024 ug/mlB

FIG. 9

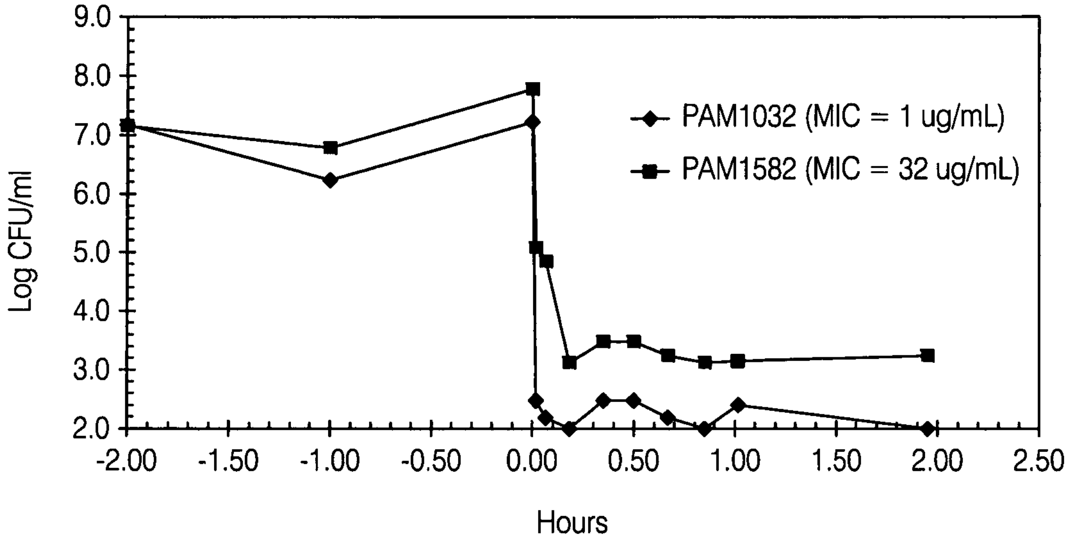
9.0
8.0
7.0
6.0
5.0
4.0
3.0
2.0
Log CFU/ml
-2.00
-1.50
-1.00
-0.50
0.00
0.50
1.00
1.50
2.00
2.50
Hours
PAM1032 (MIC = 1 ug/mL)
PAM1582 (MIC = 32 ug/mL)

FIG. 10

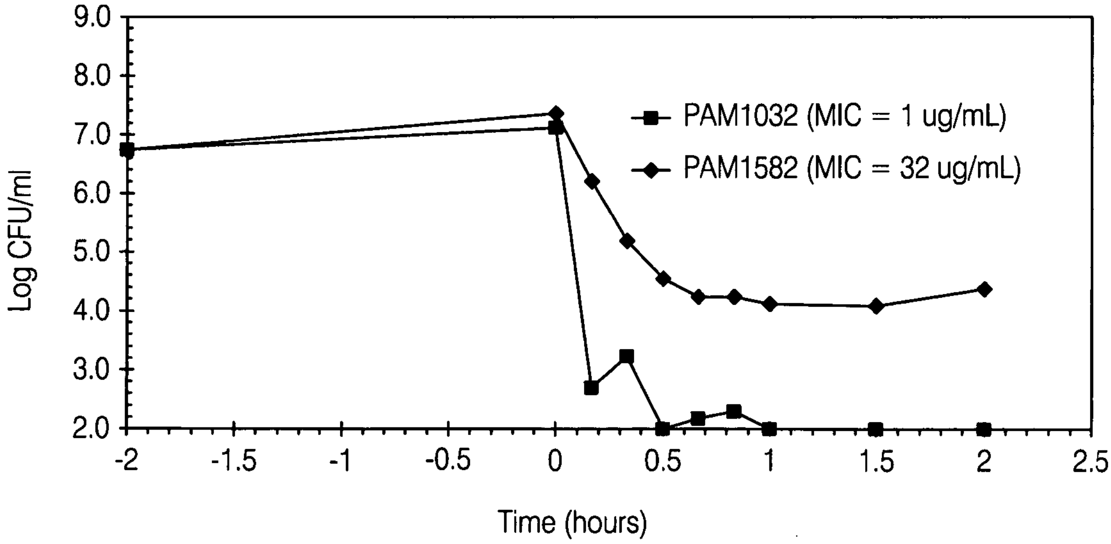
9.0
8.0
7.0
6.0
5.0
4.0
3.0
2.0
Log CFU/ml
-2
-1.5
-1
-0.5
0
0.5
1
1.5
2
2.5
Time (hours)
PAM1032 (MIC = 1 ug/mL)
PAM1582 (MIC = 32 ug/mL)

FIG. 11

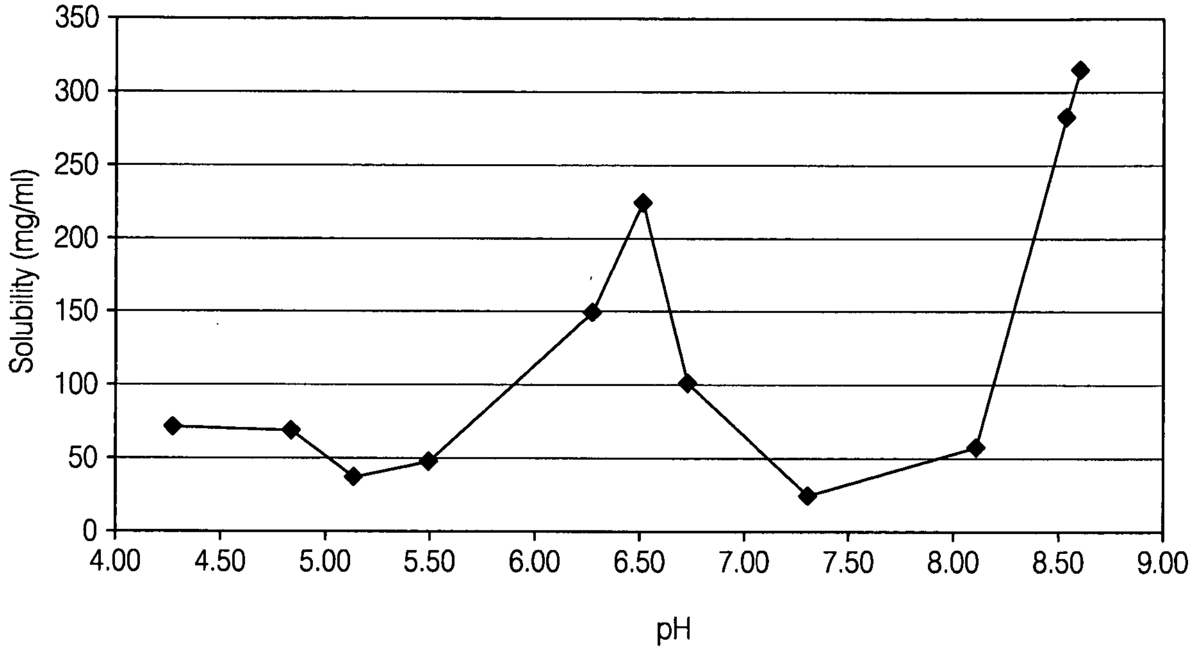
350
300
250
200
150
100
50
0
Solubility (mg/ml)
4.00
4.50
5.00
5.50
6.00
6.50
7.00
7.50
8.00
8.50
9.00
pH

FIG. 12

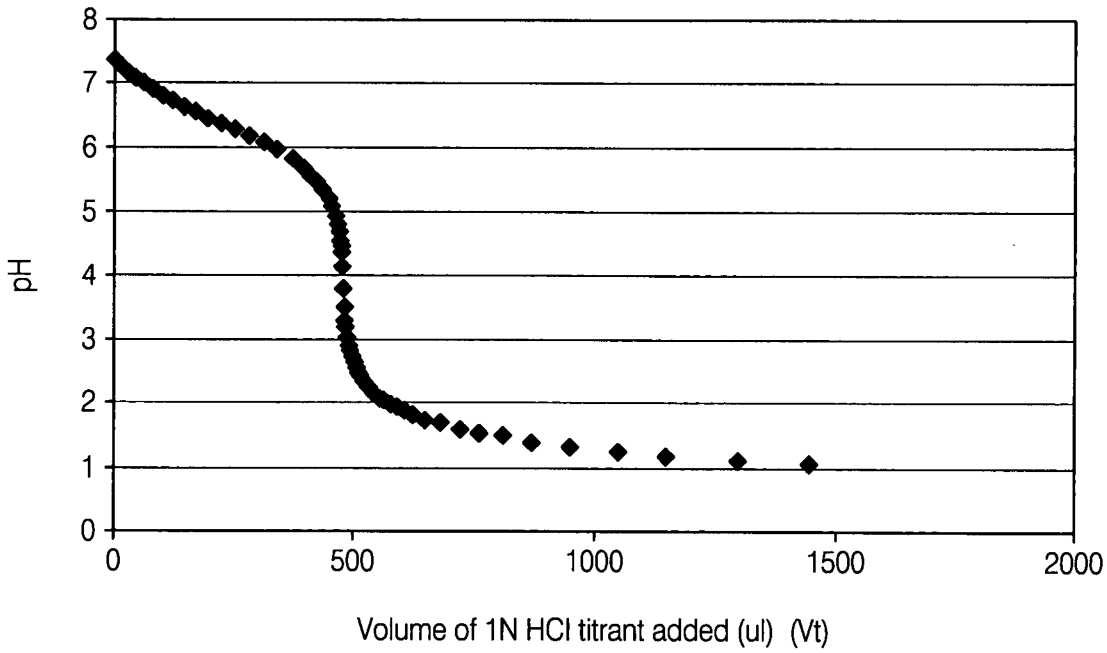
8
7
6
5
4
3
2
1
0
pH
0
500
1000
1500
2000
Volume of 1N HCl titrant added (ul) (Vt)

FIG. 13

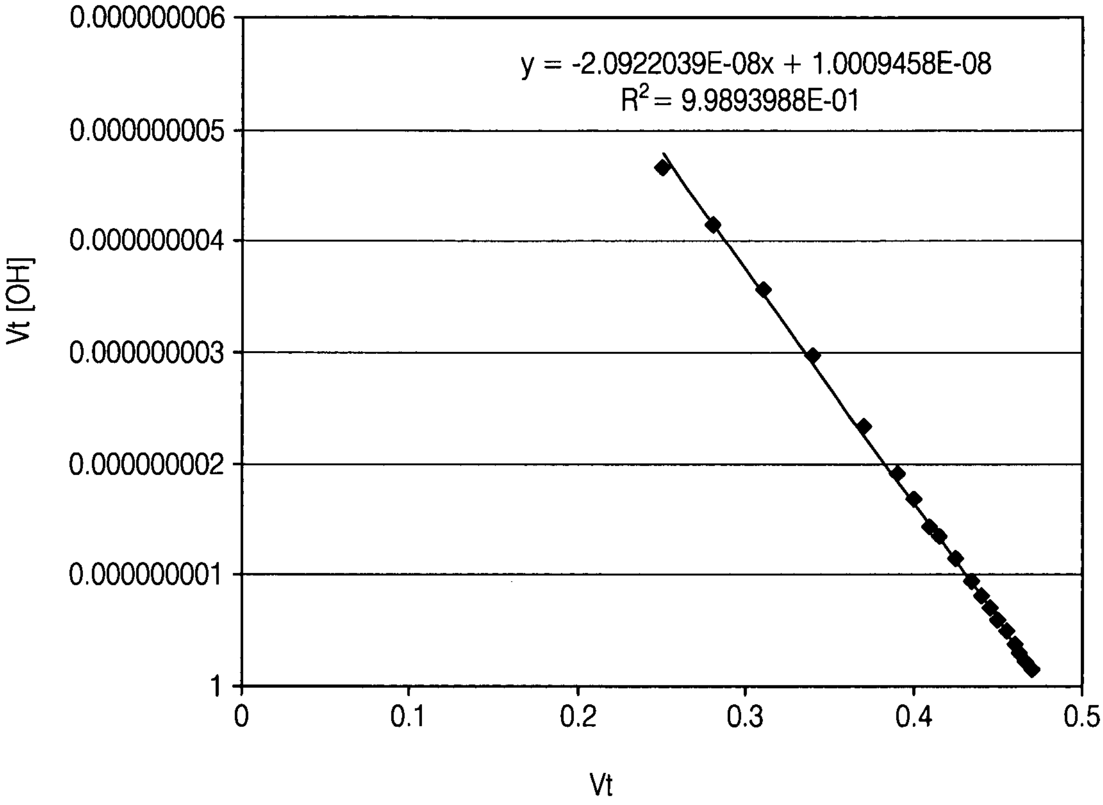
y = -2.0922039E-08x + 1.0009458E-08
R2 = 9.9893988E-01
0.000000006
0.000000005
0.000000004
0.000000003
0.000000002
0.000000001
1
0
0.1
0.2
0.3
0.4
0.5
Vt [OH]
Vt

FIG. 14

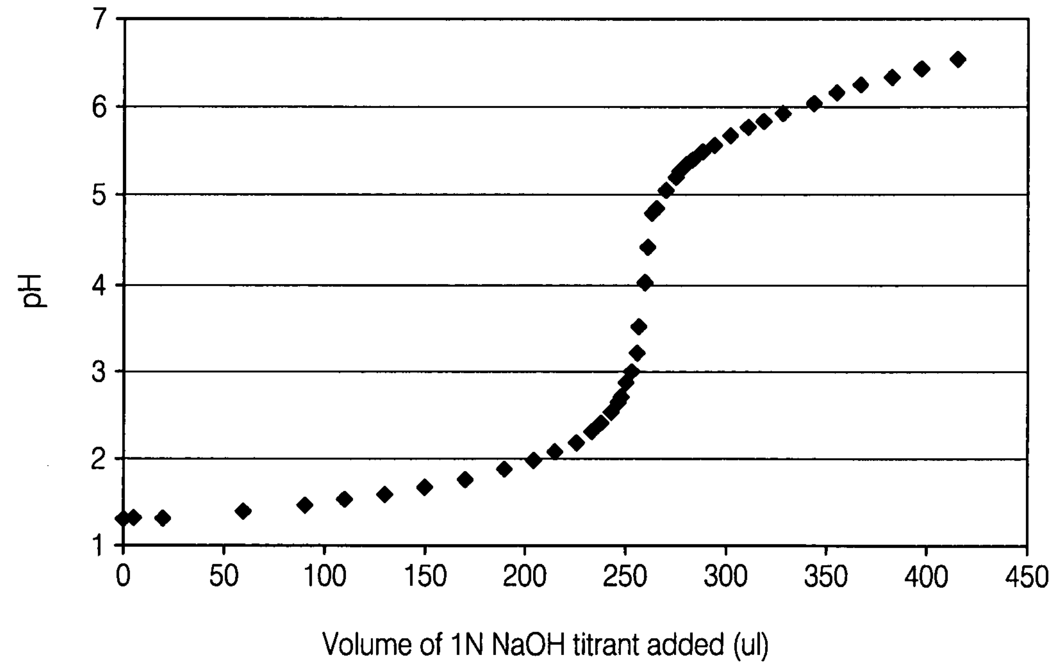
7
6
5
4
3
2
1
0
50
100
150
200
250
300
350
400
450
pH
Volume of 1N NaOH titrant added (ul)

FIG.15

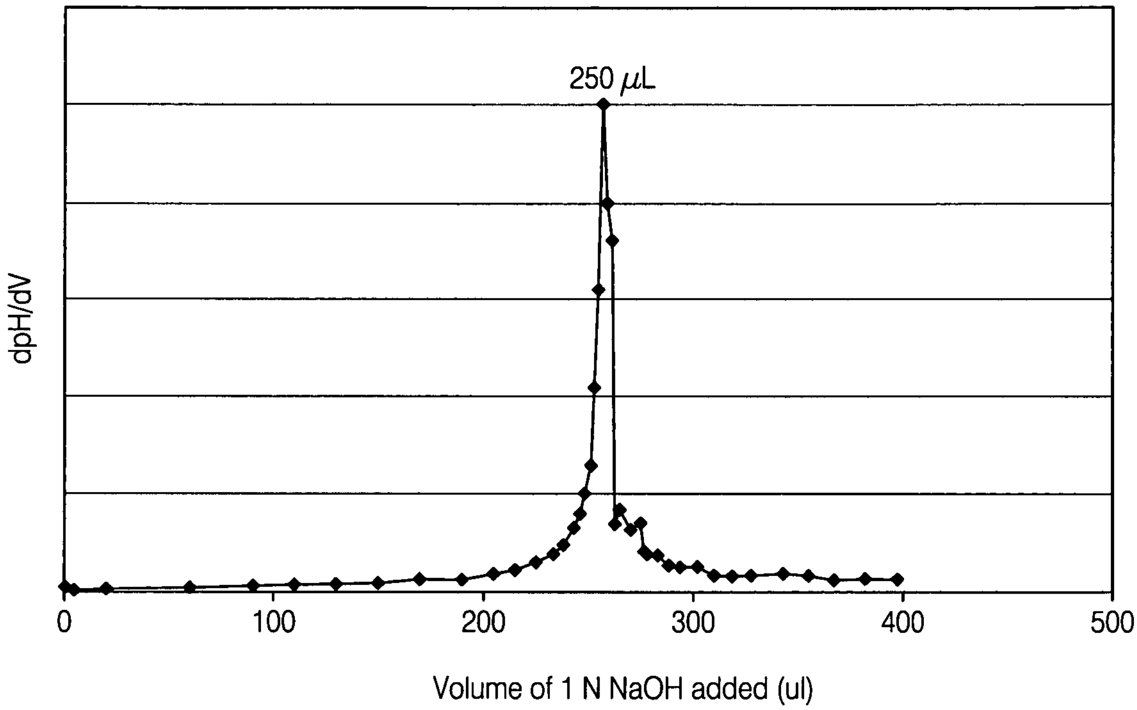
250 µL
dpH/dV
0
100
200
300
400
500
Volume of 1 N NaOH added (ul)

FIG.16

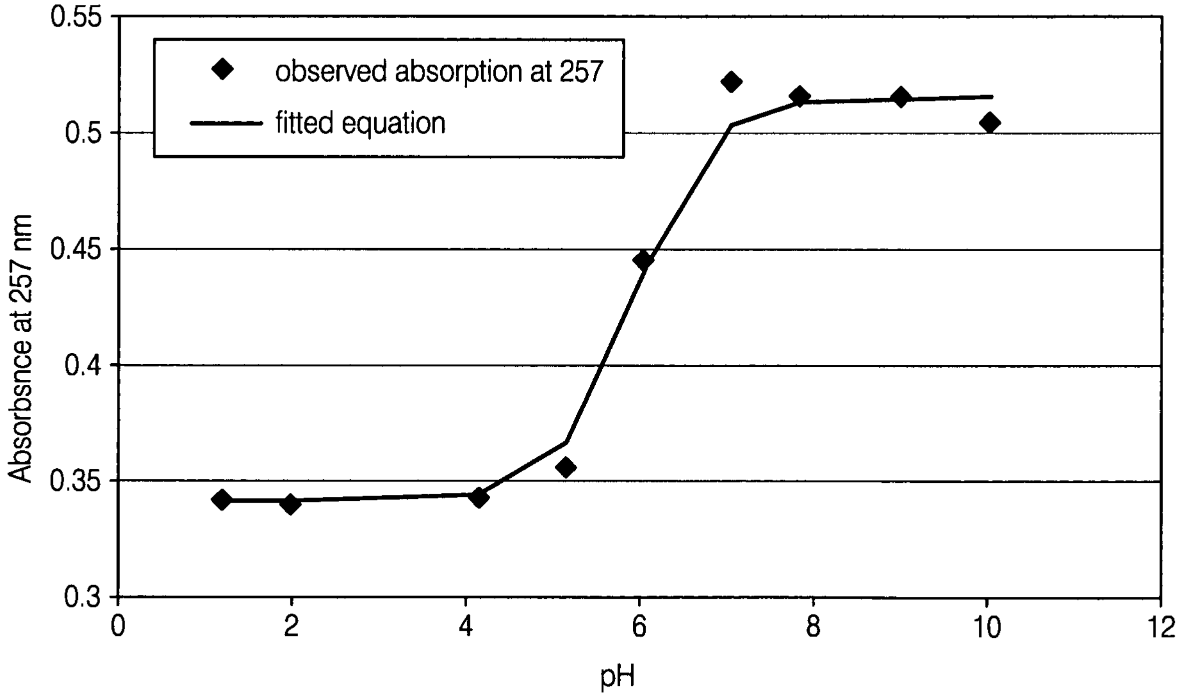
0.55
0.5
0.45
0.4
0.35
0.3
observed absorption at 257
fitted equation
Absorbsnce at 257 nm
0
2
4
6
8
10
12
pH

FIG.17

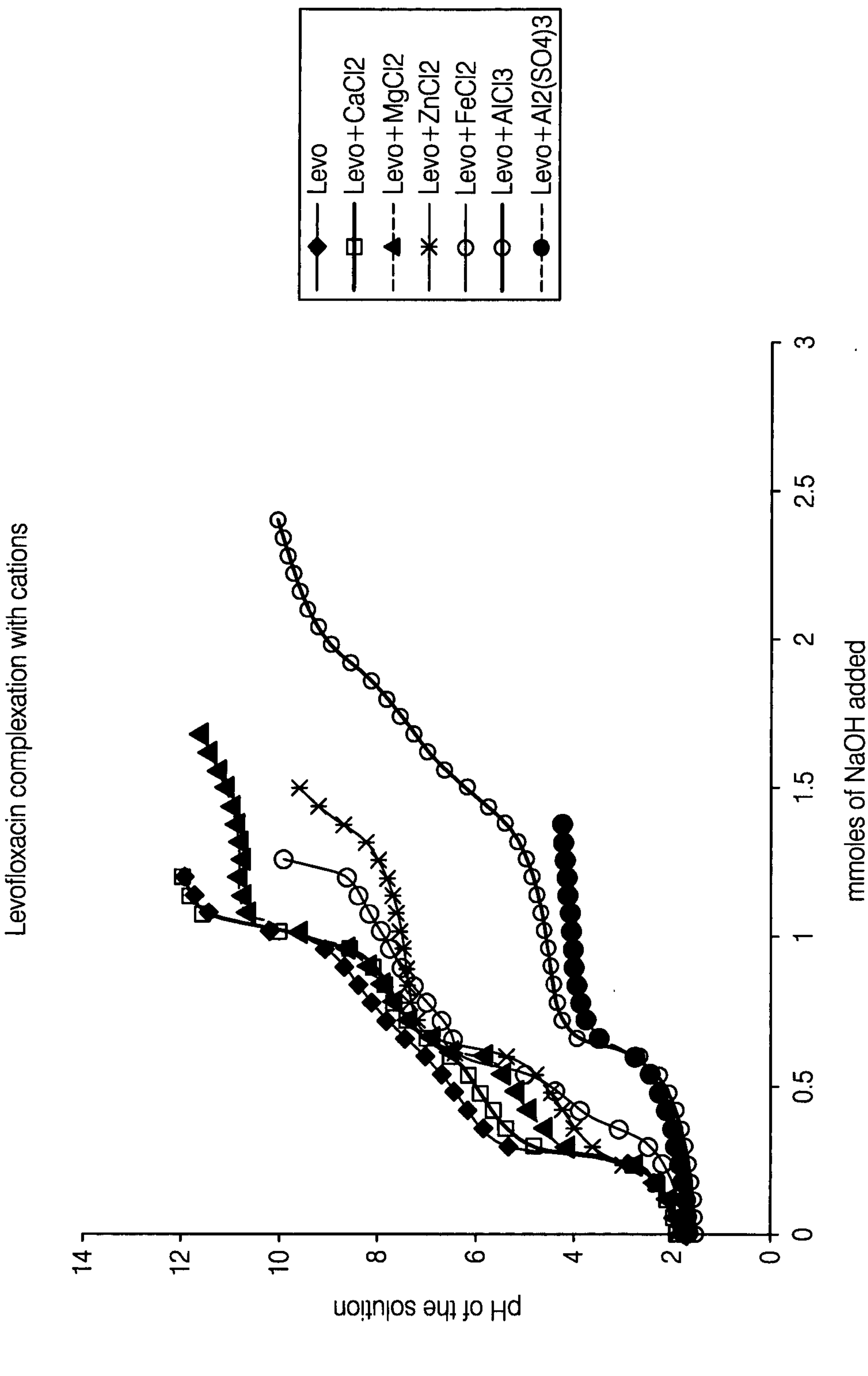
Levofloxacin complexation with cations
pH of the solution
mmoles of NaOH added
0
2
4
6
8
10
12
14
0
0.5
1
1.5
2
2.5
3
Levo
Levo+CaCl2
Levo+MgCl2
Levo+ZnCl2
Levo+FeCl2
Levo+AlCl3
Levo+Al2(SO4)3

FIG.18

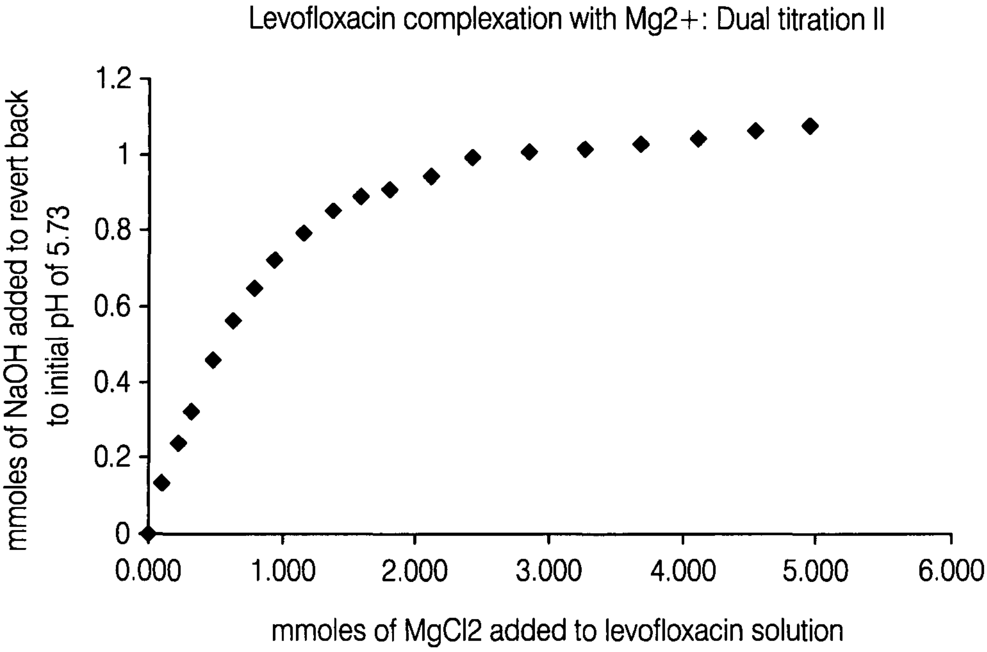
Levofloxacin complexation with Mg2+: Dual titration II
mmoles of NaOH added to revert back to initial pH of 5.73
1.2
1
0.8
0.6
0.4
0.2
0
0.000
1.000
2.000
3.000
4.000
5.000
6.000
mmoles of MgCl2 added to levofloxacin solution

FIG. 19

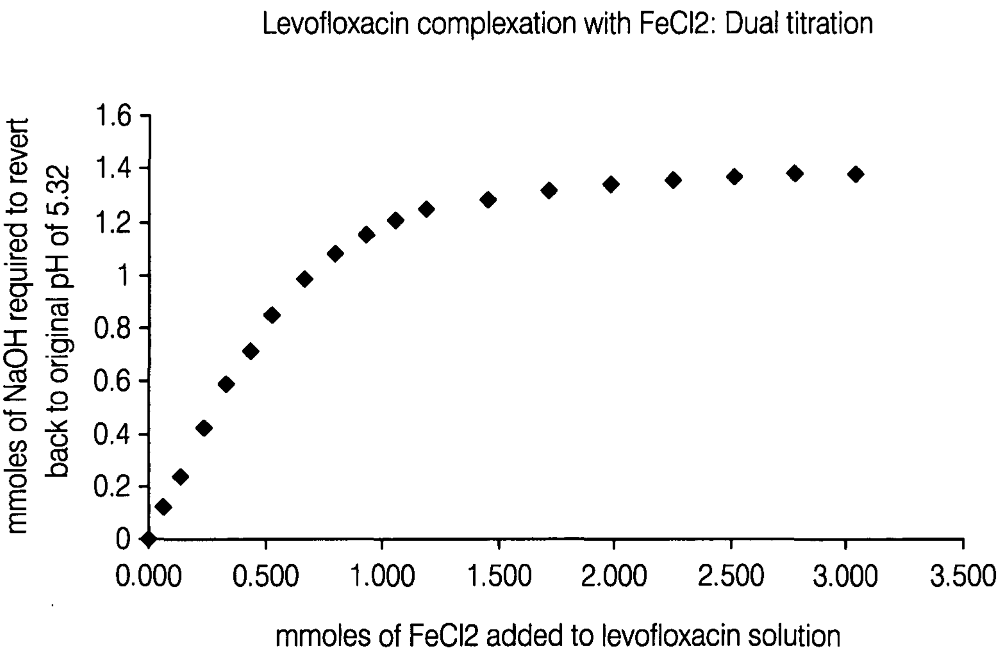
Levofloxacin complexation with FeCl2: Dual titration
mmoles of NaOH required to revert back to original pH of 5.32
1.6
1.4
1.2
1
0.8
0.6
0.4
0.2
0
0.000
0.500
1.000
1.500
2.000
2.500
3.000
3.500
mmoles of FeCl2 added to levofloxacin solution

FIG. 20

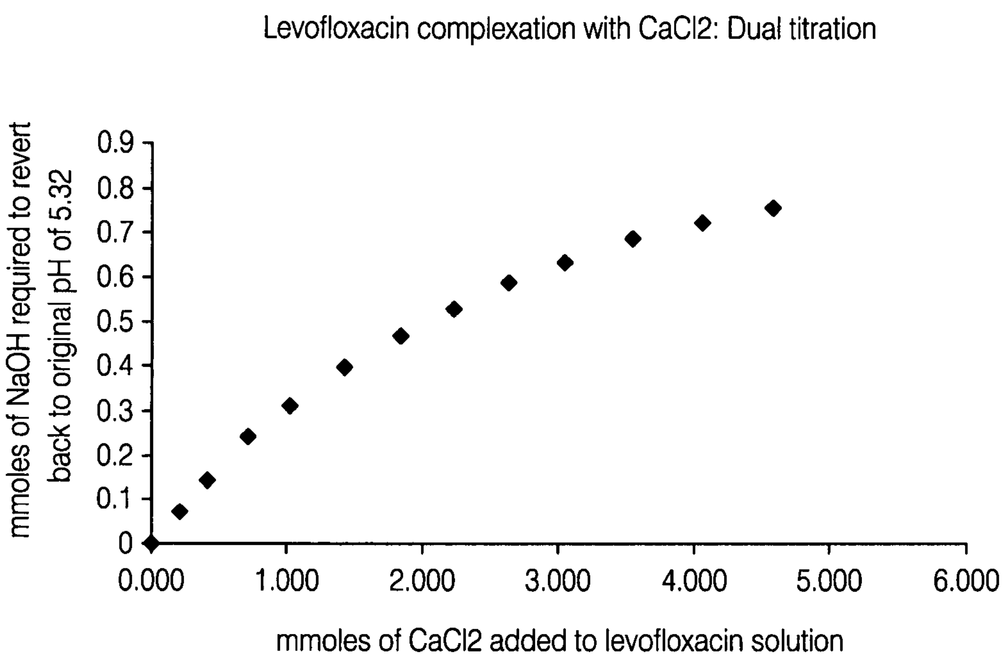
Levofloxacin complexation with CaCl2: Dual titration
mmoles of NaOH required to revert back to original pH of 5.32
0.9
0.8
0.7
0.6
0.5
0.4
0.3
0.2
0.1
0
0.000
1.000
2.000
3.000
4.000
5.000
6.000
mmoles of CaCl2 added to levofloxacin solution

FIG. 21

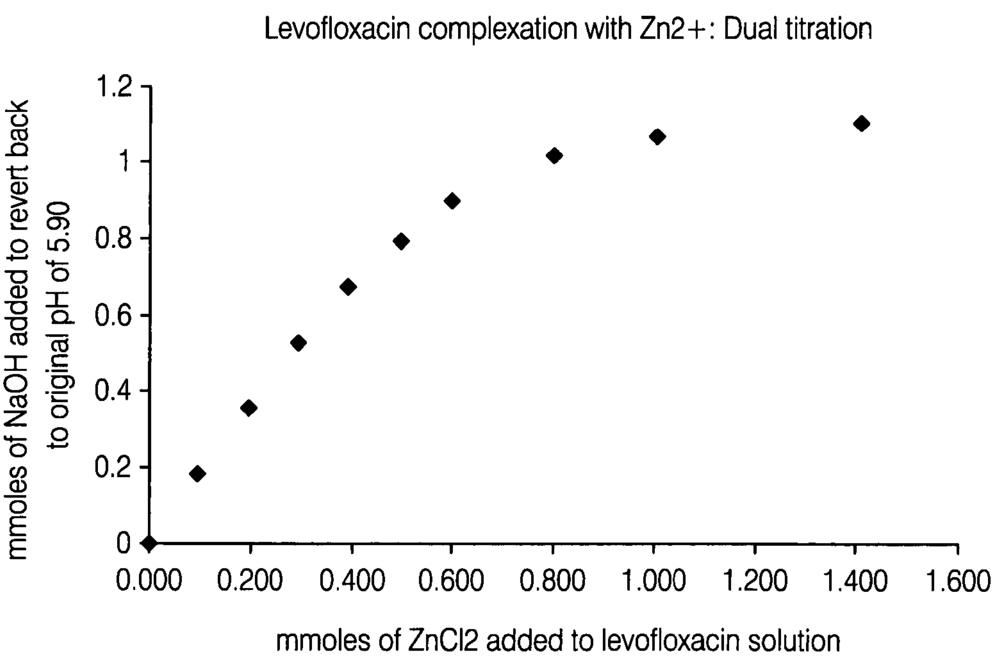
Levofloxacin complexation with Zn2+: Dual titration
mmoles of NaOH added to revert back to original pH of 5.90
1.2
1
0.8
0.6
0.4
0.2
0
0.000
0.200
0.400
0.600
0.800
1.000
1.200
1.400
1.600
mmoles of ZnCl2 added to levofloxacin solution

FIG. 22

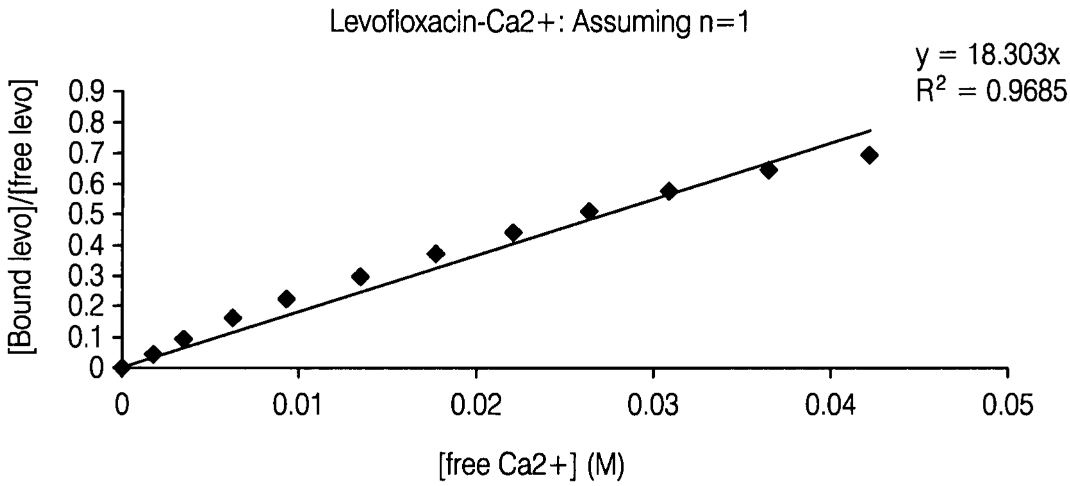


FIG. 23A

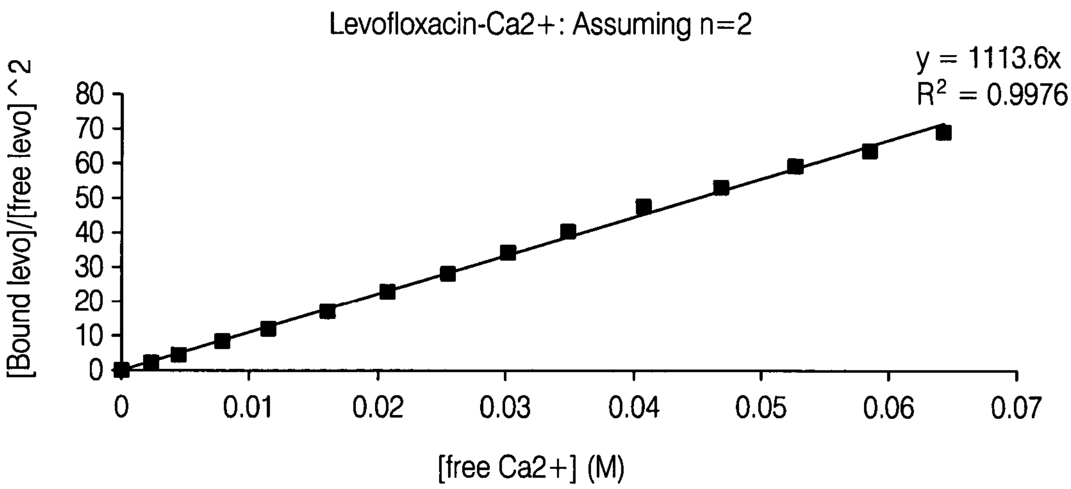


FIG. 23B

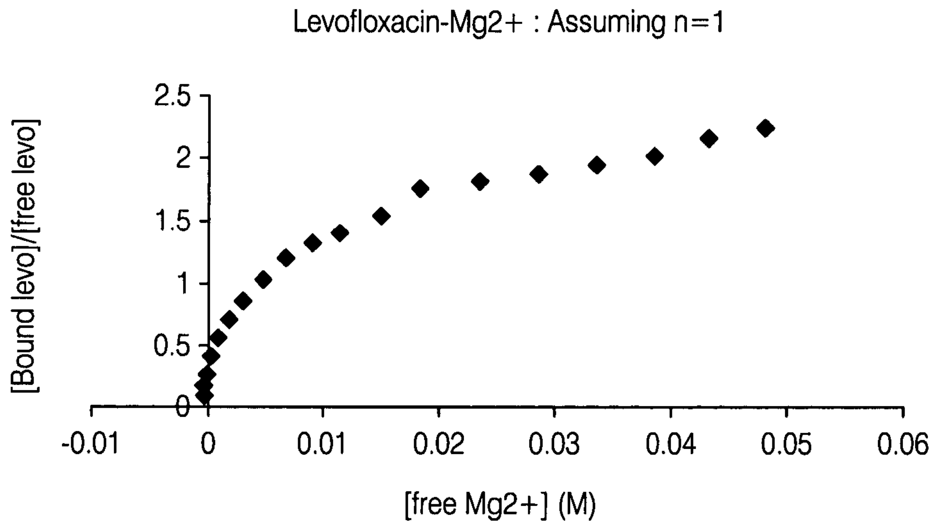


FIG. 24A

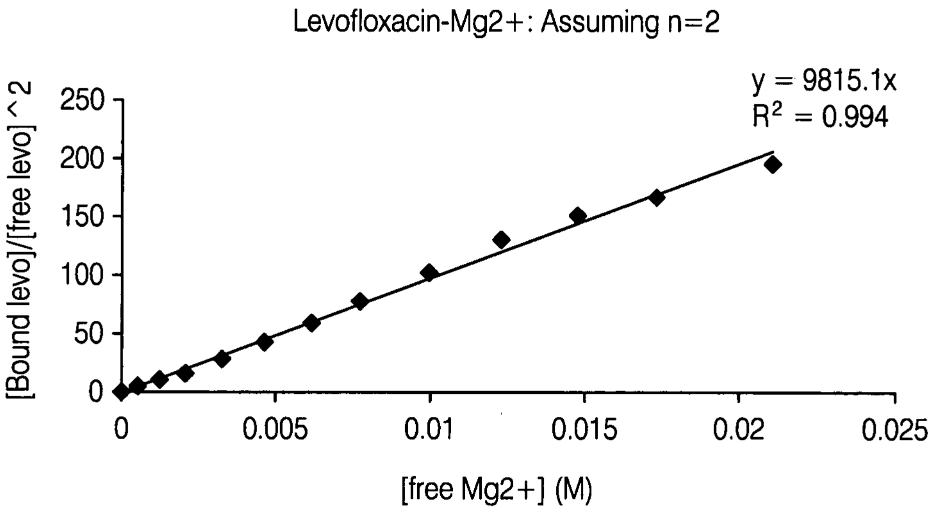


FIG. 24B

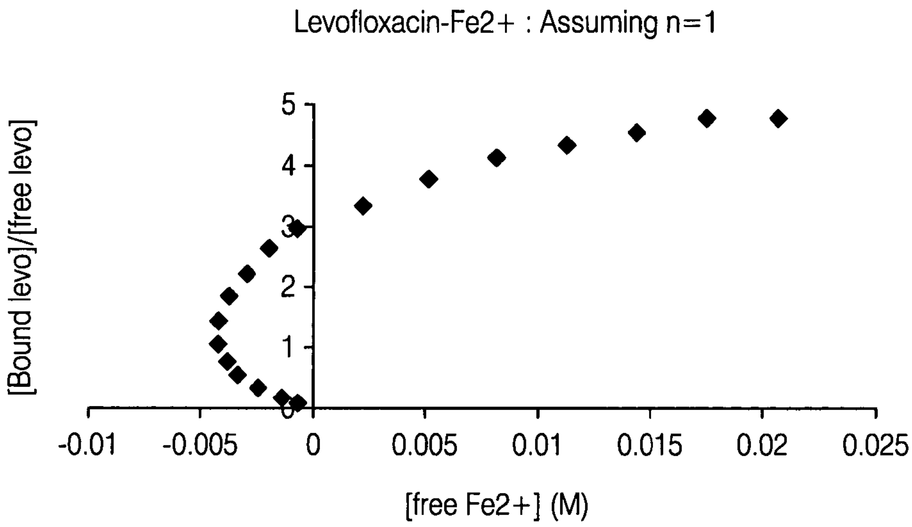


FIG. 25A

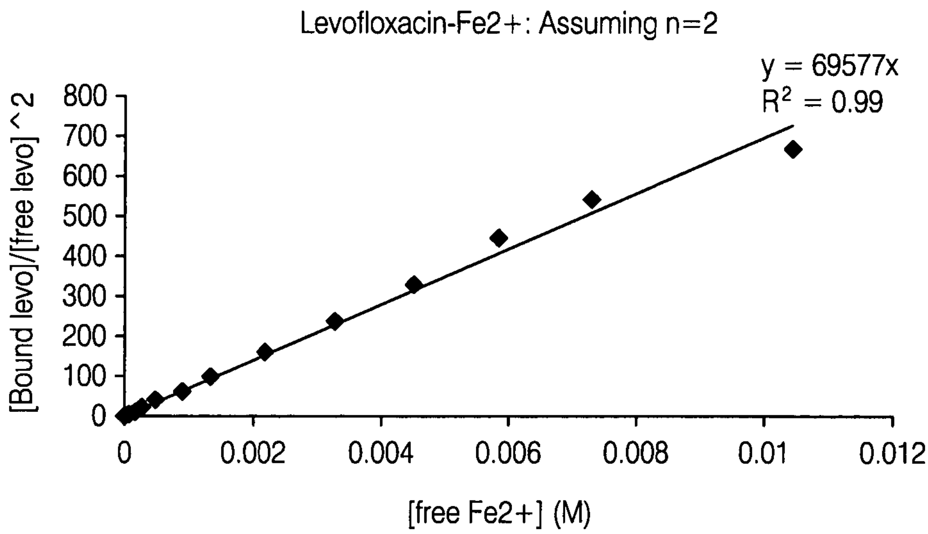


FIG. 25B

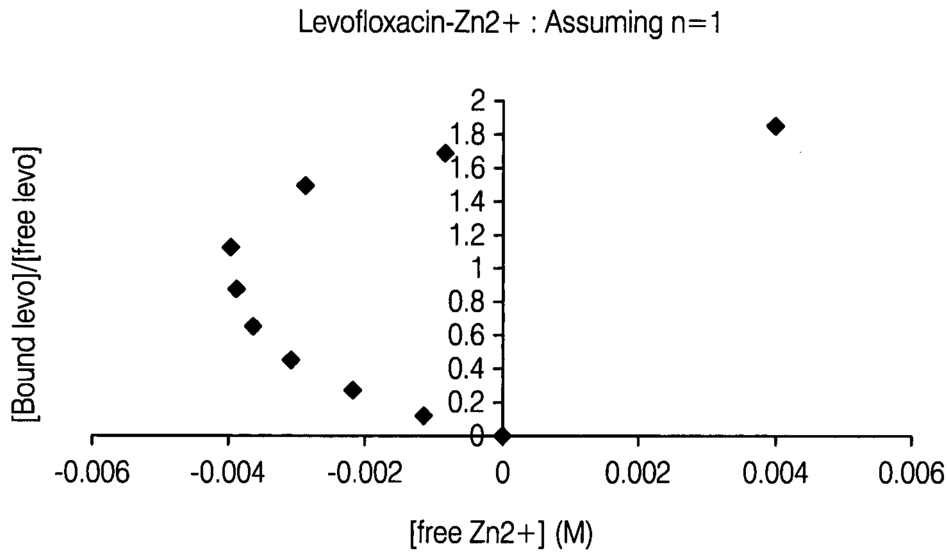


FIG. 26A

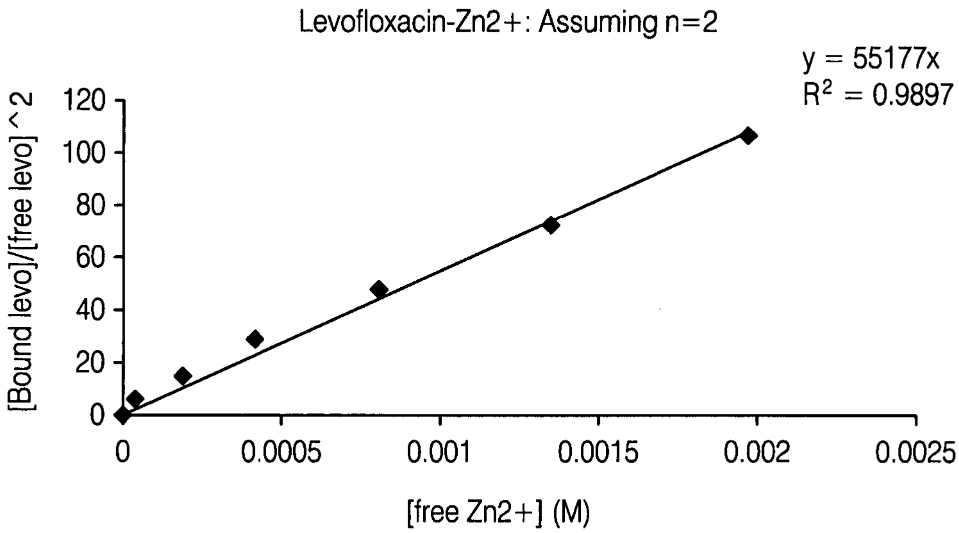


FIG. 26B

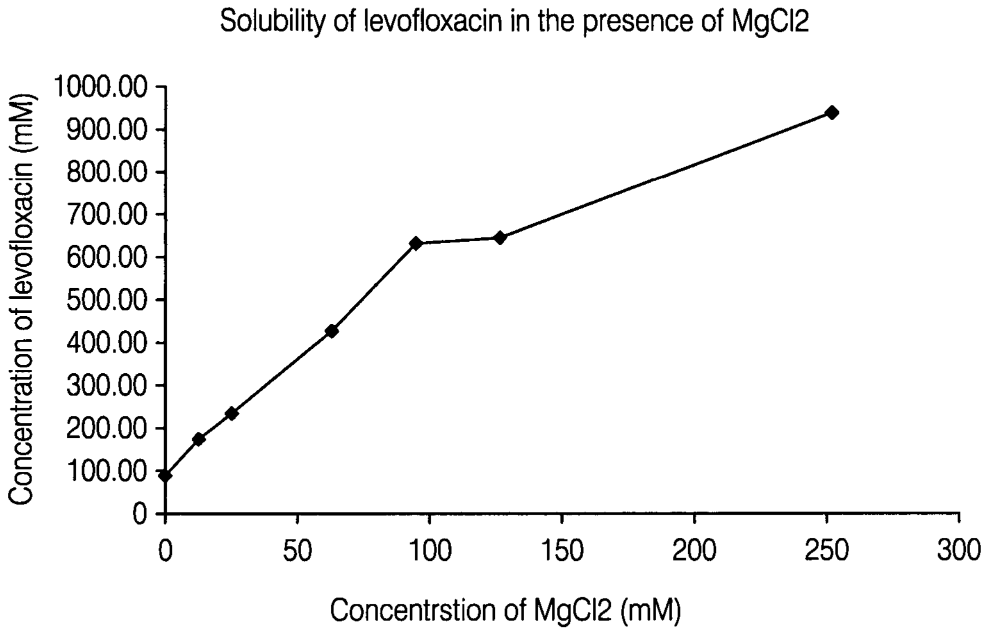
Solubility of levofloxacin in the presence of MgCl2
Concentration of levofloxacin (mM)
1000.00
900.00
800.00
700.00
600.00
500.00
400.00
300.00
200.00
100.00
0
0
50
100
150
200
250
300
Concentrstion of MgCl2 (mM)

FIG. 27

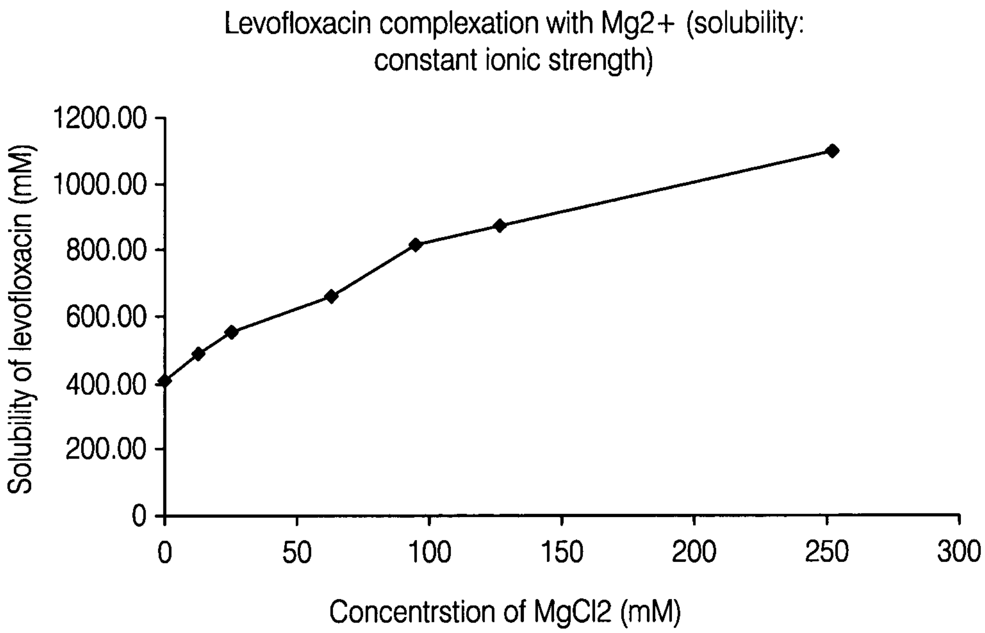
Levofloxacin complexation with Mg2+ (solubility: constant ionic strength)
Solubility of levofloxacin (mM)
1200.00
1000.00
800.00
600.00
400.00
200.00
0
0
50
100
150
200
250
300
Concentrstion of MgCl2 (mM)

FIG. 28

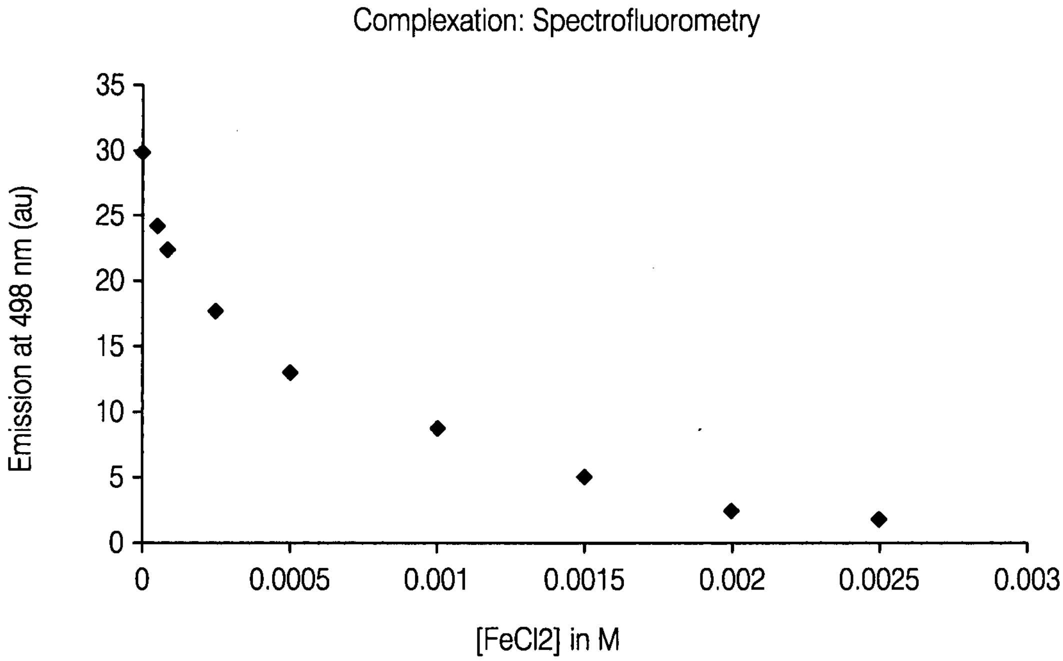
Complexation: Spectrofluorometry
Emission at 498 nm (au)
35
30
25
20
15
10
5
0
0
0.0005
0.001
0.0015
0.002
0.0025
0.003
[FeCl2] in M

FIG. 29

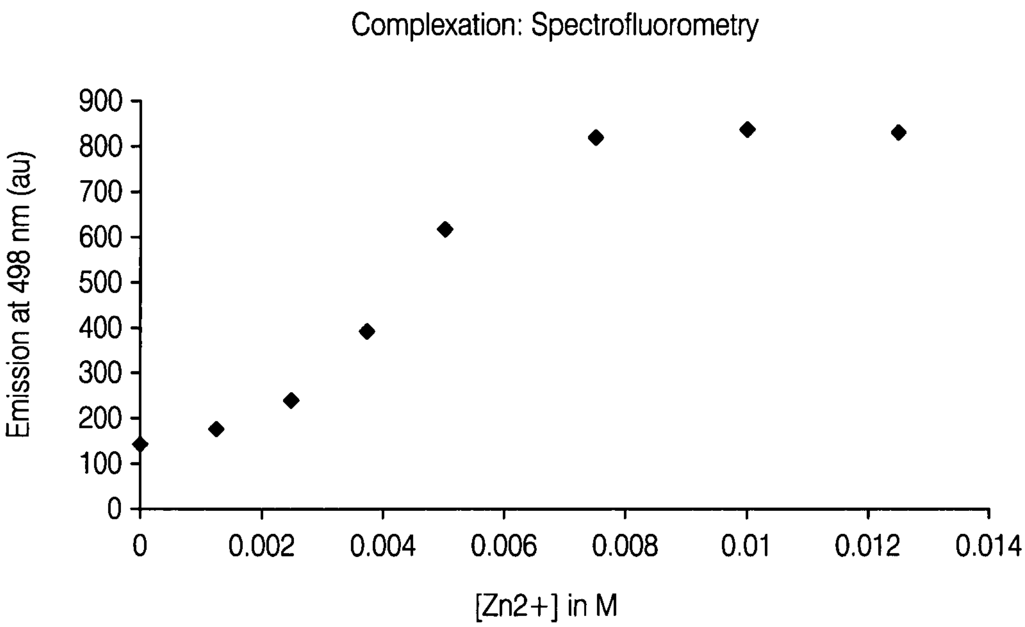
Complexation: Spectrofluorometry
Emission at 498 nm (au)
900
800
700
600
500
400
300
200
100
0
0
0.002
0.004
0.006
0.008
0.01
0.012
0.014
[Zn2+] in M

FIG. 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0218345 A
- US 4268460 A
- US 4253468 A
- US 4046146 A
- US 3826255 A
- US 4649911 A
- US 4510929 A
- US 4624251 A
- US 5164740 A
- US 5586550 A
- US 5758637 A
- US 6644304 B
- US 6338443 B
- US 5906202 A
- US 5934272 A
- US 5960792 A
- US 5971951 A
- US 6070575 A
- US 6192876 B
- US 6230706 B
- US 6349719 B
- US 6367470 B
- US 6543442 B
- US 6584971 B
- US 6601581 B
- US 4263907 A
- US 5709202 A
- US 5823179 A
- US 6196219 B
- US 5549102 A
- US 6083922 A
- US 6161536 A
- US 6264922 B
- US 6557549 B
- US 6612303 B


### Non-patent literature cited in the description


- Merck Index. Merck & Company
- Goodman and Gilman's: The Pharmacological Basis of Therapeutics. Pergamon Press, 1990
- Remington's Pharmaceutical Sciences. Mack Publishing Company
- Remington: The Science & Practice of Pharmacy. Williams & Williams, 1995
- Physician's Desk Reference. Medical Economics. 1998, vol. 52
- **ALFRED MARTIN.** Physical Pharmacy. 261-263
- **ALFRED MARTIN.** Physical Pharmacy. 265